# EUROPEAN PATENT SPECIFICATION

(11) **EP 4 545 519 B1**
(45) Date of publication and mention of the grant of the patent: **25.11.2026**
(21) Application number: 23846810.2
(22) Date of filing: 16.06.2023
(51) Int. Cl.: C07D 307/91, C07D 311/96, C07D 333/76, C07D 405/04, C07D 407/04, C07D 407/12, C07D 409/04, C07D 409/12, H10K 85/60

(54) **COMPOUND FOR ORGANIC ELECTRIC ELEMENT, ORGANIC ELECTRIC ELEMENT USING SAME, AND ELECTRONIC DEVICE THEREOF**
VERBINDUNG FÜR ORGANISCHES ELEKTRISCHES ELEMENT, ORGANISCHES ELEKTRISCHES ELEMENT DAMIT UND ELEKTRONISCHE VORRICHTUNG DAFÜR
COMPOSÉ POUR ÉLÉMENT ÉLECTRIQUE ORGANIQUE, ÉLÉMENT ÉLECTRIQUE ORGANIQUE L'UTILISANT, ET DISPOSITIF ÉLECTRONIQUE ASSOCIÉ

(30) Priority: 25.07.2022 KR 20220091984
(43) Date of publication of application: 30.04.2025
(60) Divisional application: 26199793.6 / 4 813 982
(73) Proprietor: Duk San Neolux Co., Ltd., Chungcheongnam-do 31027 (KR)
(72) Inventor: YU, Jeong Wan, Cheonan-si Chungcheongnam-do 31027 (KR); LEE, Je Woo, Cheonan-si Chungcheongnam-do 31027 (KR); OH, Hyun Ji, Cheonan-si Chungcheongnam-do 31027 (KR)
(74) Representative: Prüfer & Partner mbB Patentanwälte · Rechtsanwälte
(86) International application number: PCT/KR2023/008343
(87) International publication number: WO 2024/025151

(56) References cited:
- CN-A- 113 045 434
- CN-A- 113 045 434
- KR-A- 20190 060 914
- KR-A- 20210 015 616
- KR-A- 20210 015 616
- KR-A- 20210 053 209
- KR-A- 20210 053 209
- KR-B1- 102 374 470
- KR-B1- 102 374 470

## Description

### Technical Field

The present invention relates to a compound for an organic electric element, an organic electric element using the same, and an electronic device thereof.

### Background Art

Generally, organic luminescence refers to the phenomenon of converting electrical energy into light energy using organic materials. An organic electronic element that utilize the organic light-emitting phenomenon typically has a structure that includes an anode, a cathode, and an organic material layer between them. Here, the organic material layer is often composed of a multilayer structure composed of different materials to increase the efficiency and stability of the organic electric element, and may be composed of, for example, a hole injection layer, a hole transport layer, a light-emitting layer, an electron transport layer, and an electron injection layer.

The biggest issues in organic electroluminescent element are life and efficiency, and as the display becomes larger, these efficiency and life problems must be solved.

Efficiency, life span, driving voltage, and the like are correlated with each other. If efficiency is increased, then driving voltage is relatively lowered, and the crystallization of an organic material due to Joule heating generated during operation is reduced as driving voltage is lowered, as a result of which life span shows a tendency to increase.

However, efficiency cannot be maximized only by simply improving the organic material layer. This is because long life span and high efficiency can be simultaneously achieved when energy levels and T1 values among the respective layers included in the organic material layer, inherent material properties (mobility, interfacial properties, etc.) and the like are optimal combination.

Document KR 102 374 470 B1 and CN 113 045 434 A disclose compounds comprising a tertiary amine core for an organic electric element. Therefore, in order to fully demonstrate the excellent characteristics of organic electric element, it is necessary to develop materials that form the organic material layer of the element, especially a light-emitting auxiliary layer.

### Detailed Description of the Invention

### Technical Challenge

The object of the present invention is to provide a compound capable of lowering the driving voltage of an element and improving the luminous efficiency and life time of an element, an organic electric element using the same, and an electronic device thereof.

### Means of Solving Problems

The present invention provides a compound according to claim 1, an organic electric element including thereof in an organic material layer according to claim 14, and an electronic device comprising the organic electric element according to claim 16. Preferred embodiments are set out in the subclaims.

In an aspect of the present invention, the present invention provides compound represented by the following formula. 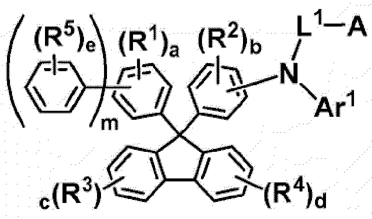
A is Formula A-1 or Formula A-2, 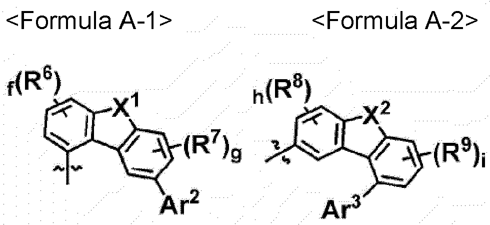
R¹ to R⁹ are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₆₀ aryloxy group, and -L'-N(Rₐ)(R_{b}), and adjacent groups may be bonded to each other to form a ring,
a is an integer of 0 to 5, b to d are each an integer of 0 to 4, e is an integer of 0 to 5, m is an integer of 0 to 2, f and i are each an integer of 0 to 3,
X¹ and X² are O or S,
L¹ and L' are each independently selected from the group consisting of a single bond, a C₆-C₆₀ arylene group, a fluorenylene group, a C₃-C₆₀ aliphatic ring group, and a C₂-C₆₀ heterocyclic group containing at least one heteroatom of O, N, S, Si and P,
Ar², Ar³, Rₐ and R_{b} are each independently selected from the group consisting of a C₆-C₆₀ aryl group, a fluorenyl group, a C₃-C₆₀ aliphatic ring group, and a C₂-C₆₀ heterocyclic group containing at least one heteroatom of O, N, S, Si and P, and
the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, and the ring formed by adjacent groups may be each optionally substituted with one or more substituents selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₃₀ aryloxy group, a C₁-C₂₀ alkylthio group, and a C₆-C₃₀ arylthio group, and
wherein Ar¹ is selected from the group consisting of Formula Ar-1 to Ar-6: 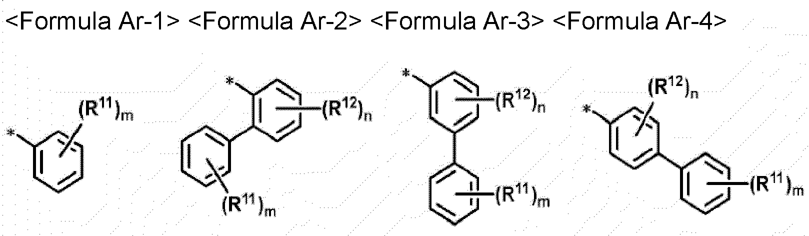 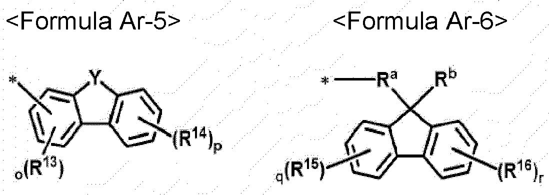
in Formula Ar-1 to Formula Ar-6,
R¹¹ to R¹⁶ are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and adjacent groups may be bonded to each other to form a C₆-C₁₄ aromatic ring group,
m' is an integer of 0 to 5, n, p, q and r are each an integer of 0 to 4, o is an integer of 0 to 3, and when each of these is an integer of 2 or more, each of R¹³s, each of R¹⁴s, each of R¹⁵s, each of R¹⁶s are the same as or different from each other,
Y is S, O, or N(R₃),
R^{a} is selected from the group consisting of a single bond, a C₁-C₂₀ alkylene group, a C₆-C₃₀ arylene group, a fluorenylene group, a C₂-C₃₀ heterocyclic group comprising at least one heteroatom selected from the group consisting of O, N, S, Si and P, and a C₃-C₃₀ aliphatic ring group, and

R₃ and R^{b} are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group.

In another aspect of the present invention, the present invention provides an organic electric element comprising the compound represented by formula above and an electronic device thereof.

### Effect of Invention

By using the compound according to embodiment of the present invention, a driving voltage of an organic electric element can be lowered and the luminous efficiency and lifetime of the element can be improved.

### BRIEF DESCRIPTION OF THE DRAWINGS

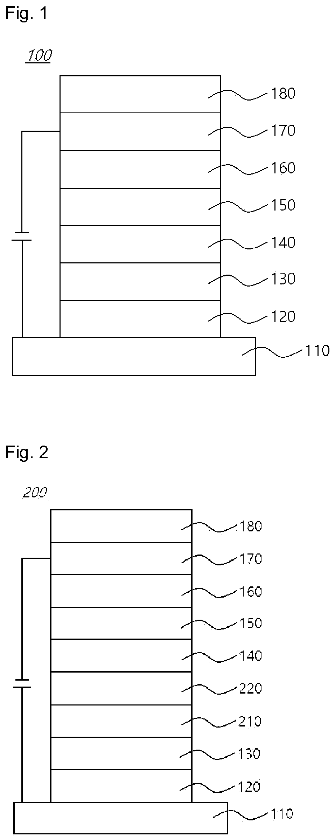

Figures 1 to 3 illustrate examples of organic electroluminescent element according to the present invention.

**[Description of Reference Numerals]**

| | | | |
|---|---|---|---|
| 100, 200, 300: | organic electric element | 110: | first electrode |
| 120: | hole injection layer | 130: | hole transport layer |
| 140: | light-emitting layer | 150: | electron transport layer |
| 160: | electron injection layer | 170: | second electrode |
| 180: | layer for improving light efficiency | 210: | buffer layer |
| 220: | light-emitting auxiliary layer | 320: | first hole injection layer |
| 330: | first hole transport layer | 340: | first light-emitting layer |
| 350: | first electron transport layer | 360: | first charge generation layer |
| 361: | second charge generation layer | 420: | second hole injection layer |
| 430: | second hole transport layer | 440: | second light-emitting layer |
| 450: | second electron transport layer | CGL: | charge generation layer |
| ST1: | first stack | | |

### DETAILED DESCRIPTION

Unless otherwise stated, the term "aryl group" or "arylene group" as used herein has, but not limited to, 6 to 60 carbon atoms. The aryl group or arylene group in the present invention may comprise a monocyclic ring, ring assemblies, a fused polycyclic system, spiro compound and the like.

As used herein, the term "fluorenyl group" refers to a substituted or unsubstituted fluorenyl group, and "fluorenylene group" refers to a substituted or unsubstituted fluorenylene group. The fluorenyl group or fluorenylene group used in the present invention comprises a spiro compound formed by combining R and R' with each other in the following structure, and also comprises compound formed by linking adjacent R"s to each other. "Substituted fluorenyl group", "substituted fluorenylene group" means that at least one of R, R', R" in the following structure is a substituent other than hydrogen, and R" may be 1 to 8 in the following formula. In this specification, a fluorenyl group, a fluorenylene group, and the like may be referred to as a fluorene group or fluorene regardless of the valence.

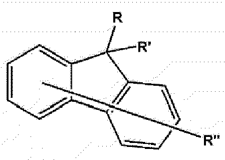

The term "spiro compound" as used herein has a spiro union which means union having one atom as the only common member of two rings. Here, the atom shared between the two rings is called as a 'spiro atom' and the compounds are called as 'monospiro-', 'dispiro-' or 'trispiro-' depending on the number of spiro atoms in one compound.

The term "heterocyclic group" used in the specification comprises a non-aromatic ring as well as an aromatic ring like "heteroaryl group" or "heteroarylene group". Unless otherwise stated, the term "heterocyclic group" means, but not limited to, a ring containing one or more heteroatoms and having 2 to 60 carbon atoms. Unless otherwise stated, the term "heteroatom" as used herein represents, for example, N, O, S, P or Si and may comprise a heteroatom group such as SO₂, P=O etc. instead of carbon forming a ring such as the following compound. In the specification, "heterocyclic group" comprises a monocyclic, ring assemblies, fused polycyclic system, a spiro-compound and the like.

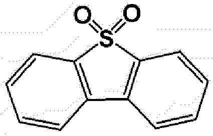

The term "aliphatic ring group" as used herein refers to a cyclic hydrocarbon except for aromatic hydrocarbons, and comprises a monocyclic ring, ring assemblies, a fused polycyclic system, a spiro-compound and the like, and unless otherwise specified, it means, but not limited thereto, a ring of 3 to 60 carbon atoms. For example, a fused ring of benzene being an aromatic ring and cyclohexane being a non-aromatic ring corresponds to aliphatic ring group.

In this specification, a 'group name' corresponding to an aryl group, an arylene group, a heterocyclic group, and the like exemplified for each symbol and its substituent may be written in the name of functional group reflecting the valence, and may also be described as the name of a parent compound. For example, in the case of phenanthrene which is a kind of aryl group, it may be described by distinguishing valence such as 'phenanthryl (group)' when it is 'monovalent group', and as 'phenanthrylene (group)' when it is 'divalent group', and it may also be described as a parent compound name, 'phenanthrene', regardless of its valence. Similarly, in the case of pyrimidine, it may be described as 'pyrimidine' regardless of its valence, and it may also be described as the name of corresponding functional group such as pyrimidinyl (group) when it is 'monovalent group', and as 'pyrimidylene (group)' when it is 'divalent group'.

In addition, in the present specification, the numbers and alphabets indicating a position may be omitted when describing a compound name or a substituent name, For example, pyrido[4,3-d]pyrimidine, benzopuro[2,3-d] pyrimidine and 9,9-dimethyl-9H-fluorene can be described as pyridopyrimidine, benzofurropyrimidine and dimethylfluorene, respectively. Therefore, both benzo[g]quinoxaline and benzo[f] quinoxaline can be described as benzoquinoxaline.

In addition, unless otherwise expressed, where any formula of the present invention is represented by the following formula, the substituent according to the index may be defined as follows.

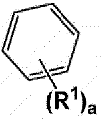

In the above formula, where a is an integer of zero, the substituent R¹ is absent, that is, hydrogen atoms are bonded to all the carbon constituting the benzene ring. Here, chemical formulas or compounds may be written without indicating the hydrogen bonded to carbon. In addition, when a is an integer of 1, one substituent R¹ is bonded to one of the carbons forming the benzene ring, and when a is an integer of 2 or 3, for example, it can be bonded as follows, it is bonded to the carbon of the benzene ring in a similar manner when a is an integer from 4 to 6, and when a is an integer greater than or equal to 2, R¹s may be the same or different.

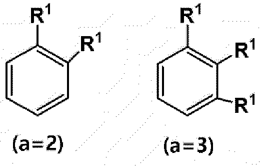

In addition, unless otherwise specified in the specification, the term 'ring' refers to an aryl ring, heteroaryl ring, fluorene ring, aliphatic ring, etc., and a number-membered(atom) ring may refer to the shape of a ring. For example, naphthalene corresponds to a two-fused(condensed) ring, anthracene to a three-fused(condensed) ring, thiophene or furan corresponds to a five-membered ring, and benzene or pyridine corresponds to a six-membered ring.

In addition, unless otherwise specified in the present specification, when adjacent groups are linked to each other to form a ring, the ring may be selected from the group consisting of a C₆-C₆₀ aromatic ring group, a fluorenyl group, a C₂-C₆₀ heterocyclic group containing at least one heteroatom selected from the group consisting of O, N, S, Si and P, and a C₃-C₆₀ aliphatic ring. Here, the aromatic ring group may include an aryl ring, and the heterocyclic group may include a heteroaryl ring.

Unless otherwise stated, the term "between adjacent groups", for example, in case of the following Formulas, comprises not only "between R₁ and R₂", "between R₂ and R₃", "between R₃ and R₄", "between R₅ and R₆", but also "between R₇ and R₈" sharing one carbon, and may comprise "between substituents" attached to atom(carbon or nitrogen) consisting different ring, such as "between R₁ and R₇", "between R₁ and R₈", or "between R₄ and R₅" and the like. That is, when the substituents substituted on the elements forming the ring are not directly adjacent to each other, any one substituent may be be adjacent groups to the substituent bonded to the next element forming the ring, and substituents bonded to the same ring constituent carbons may also correspond to neighboring groups. In the following Formula, when the substituents bonded to the same carbon, such as R₇ and R₈, are linked to each other to form a ring, a compound containing a spiro-moiety may be formed.

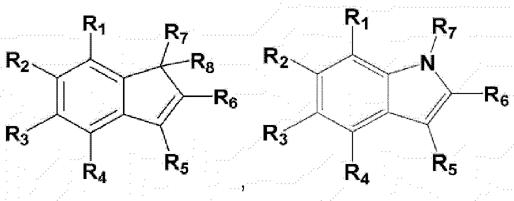

In addition, in the present specification, the expression 'adjacent groups may be linked to each other to form a ring' is used in the same sense as 'adjacent groups are linked selectively to each other to form a ring', and a case where at least one pair of adjacent groups may be bonded to each other to form a ring.

In addition, unless otherwise specified in the present specification, substituents such as an aryl group, an arylene group, a fluorenyl group, a fluorenylene group, a heterocyclic group, an aliphatic ring group, an alkyl group, an alkenyl group, an alkynyl group, an alkoxyl group, an aryloxyl group, alkylthio group, arylthio group, etc., and a ring formed by adjacent groups may be each optionally substituted with one or more substituents selected from the group consisting of deuterium, halogen, a cyano group, a nitro group, siloxane group, a C₆-C₃₀ aryl group, a fluorenyl group, a C₂-C₃₀ heterocyclic group containing at least one heteroatom of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, a C₆-C₂₀ arylthio group, a silane group unsubstituted or substituted with a C₁-C₂₀ alkyl group or a C₆-C₂₀ aryl group, and a phosphine oxide group unsubstituted or substituted with a C₁-C₂₀ alkyl group or a C₆-C₂₀ aryl group.

Hereinafter, referring to Figures 1 to 3, a lamination structure of an organic electric element including the compound of the present invention will be described.

In designation of reference numerals to components in respective drawings, it should be noted that the same elements will be designated by the same reference numerals although they are shown in different drawings. In addition, in the following description of the present invention, a detailed description of known functions and configurations incorporated herein will be omitted when it may make the subject matter of the present invention rather unclear.

Terms, such as first, second, A, B, (a), (b) or the like may be used herein when describing components of the present invention. Each of these terminologies is not used for defining an essence, order or sequence of a corresponding component but used merely to distinguish the corresponding component from other component(s). It will be understood that the expression 'one component is "connected," "coupled" or "joined" to another component' comprises the case where a third component may be "connected," "coupled" or "joined" between a first component and a second component as well as the case where the first component may be directly connected, coupled or joined to the second component.

In addition, it will be understood that when an element such as a layer, film, region or substrate is referred to as being "on" or "over" another element, it can be directly on the other element or intervening elements may also be present. In contrast, when an element is referred to as being "directly on" another element, there are no intervening elements present.

The Figures 1 to 3 show an example of an organic electric element according to an embodiment of the present invention, respectively.

Referring to the FIG. 1, an organic electric element 100 according to an embodiment of the present invention includes a first electrode 110 formed, a second electrode 170, and an organic material layer between the first electrode 110 and the second electrode 170 on a substrate (not shown), and an inorganic material layer may be included between the first electrode 110 and the second electrode 120.

For example, the first electrode 110 may be an anode (positive electrode), and the second electrode 170 may be a cathode (negative electrode). In the case of an inverted organic electric element, the first electrode may be a cathode, and the second electrode may be an anode.

The organic material layer refers to a layer containing at least one organic substance. For example, the organic material layer may include a hole injection layer 120, a hole transport layer 130, a light-emitting layer 140, an electron transport layer 150, and an electron injection layer 160. Further, the electron injection layer 160 may be an inorganic material layer that does not contain organic material.

Specifically, a hole injection layer 120, a hole transport layer 130, a light-emitting layer 140, an electron transport layer 150, and an electron injection layer 160 may be formed on the first electrode 110 in sequence.

Preferably, a layer for improving the luminous efficiency 180 may be formed one side of sides of the first electrode 110 and the second electrode 170, wherein the one side is not facing an organic material layer or an inorganic material layer. When a layer for improving the luminous efficiency 180 is formed, the luminous efficiency of an organic electric element can be improved.

For example, the light efficiency improving layer 180 may be formed on the second electrode 170, as a result, in the case of a top emission organic light emitting element, the optical energy loss due to Surface Plasmon Polaritons (SPPs) at the second electrode 170 may be reduced and in the case of a bottom emission organic light emitting element, the light efficiency improving layer 180 may serve as a buffer for the second electrode 170.

A buffer layer 210 or a light-emitting auxiliary layer 220 may be further formed between the hole transport layer 130 and the light-emitting layer 140, which will be described with reference to FIG. 2.

Referring to FIG. 2, the organic electric element 200 according to another embodiment of the present invention may include a hole injection layer 120, a hole transport layer 130, a buffer layer 210, a light-emitting auxiliary layer 220, a light-emitting layer 140, the electron transport layer 150, the electron injection layer 160, and a second electrode 170 formed on a first electrode 110 in sequence, and a layer for improving light efficiency 180 may be formed on the second electrode.

Although not shown in FIG. 2, an electron transport auxiliary layer may be further formed between the light-emitting layer 140 and the electron transport layer 150.

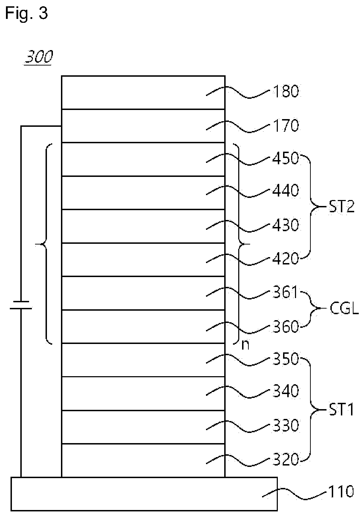

In addition, according to another embodiment of the present invention, the organic material layer may be a form consisting of multiple stacks, wherein the stacks comprise a hole transport layer, a light-emitting layer, and an electron transport layer, respectively. This will be described with reference to FIG. 3.

Referring to FIG. 3, two or more sets of stacks of the organic material layers ST1 and ST2 may be formed between the first electrode 110 and the second electrode 170 in the organic electric element 300 according to another embodiment of the present invention, wherein the organic material layers may be consisted of multiple layers, respectively, and the charge generation layer CGL may be formed between the stacks of the organic material layer.

Specifically, the organic electric element according to the embodiment of the present invention may comprise a first electrode 110, a first stack ST1, a charge generation layer CGL, a second stack ST2, and a second electrode 170 and a layer for improving light efficiency 180.

The first stack ST1 is an organic material layer formed on the first electrode 110, and the first stack ST1 may comprise the first hole injection layer 320, the first hole transport layer 330, the first light-emitting layer 340 and the first electron transport layer 350 and the second stack ST2 may comprise a second hole injection layer 420, a second hole transport layer 430, a second light-emitting layer 440 and a second electron transport layer 450. As such, the first stack and the second stack may be the organic material layers having the same or different stacked structures.

The charge generation layer CGL may be formed between the first stack ST1 and the second stack ST2. The charge generation layer CGL may comprise a first charge generation layer 360 and a second charge generation layer 361. The charge generating layer CGL is formed between the first light-emitting layer 340 and the second light-emitting layer 440 to increase the current efficiency generated in each light-emitting layer and to smoothly distribute charges.

The first light-emitting layer 340 may comprise a light emitting material comprising a blue host doped with a blue fluorescent dopant and the second light-emitting layer 440 may comprise a light emitting material comprising a green host doped with a greenish yellow dopant and a red dopant together, but the material of the first light-emitting layer 340 and the second light-emitting layer 440 according to an embodiment of the present invention is not limited thereto.

In FIG. 3, n may be an integer of 1 to 5 and the charge generation layer CGL and the third stack may be further stacked on the second stack ST2 when n is 2.

When a plurity of light-emitting layers are formed in a multi-layer stack structure as shown in FIG. 3, it is possible to manufacture an organic electroluminescent element that emits not only white light but also various colors, wherein the white light is emitted by the mixing effect of light emitted from each light-emitting layer.

Compound(s) represented by Formula 1 of the present invention may be included in an organic material layer. For example, the compounds represented by Formulas 1 of the present invention can be used as a material for a hole injection layer 120, 320, 420, a hole transport layer 130, 330, 430, a buffer layer 210, a light-emitting auxiliary layer 220, and an electron transport layer 150, 350, 450, an electron injection layer 160, a light-emitting layer 140, 340, 440, or a layer for improving light efficiency 180, preferably, a light-emitting auxiliary layer 220 or/and a layer for improving light efficiency 180, more preferably, a light-emitting auxiliary layer 220.

Even if the cores of compounds are identical or similar to each other, the band gap, the electrical characteristics, the interface characteristics and the like may be different depending on which substituent is bonded at which position. Therefore, it is necessary to study the selection of the core and the combination with sub-substituent bonded to the core. In particular, long life span and high efficiency can be simultaneously achieved when the optimal combination of energy levels and T₁ values, inherent material properties (mobility, interfacial properties, etc.), and the like among the respective layers of an organic material layer is achieved.

Therefore, the energy level and T₁ value between the respective layers of the organic material layer, inherent material properties (mobility, interfacial properties, etc.) and the like can be optimized by using the compound represented by Formula 1 as a material for a light-emitting auxiliary layer 220.

The organic electric element according to an embodiment of the present invention may be manufactured using various deposition methods. The organic electric element according to an embodiment of the present invention may be manufactured using a PVD (physical vapor deposition) method or CVD (chemical vapor deposition) method. For example, the organic electric element may be manufactured by depositing a metal, a conductive metal oxide, or a mixture thereof on the substrate to form the anode 110, forming the organic material layer comprising the hole injection layer 120, the hole transport layer 130, the light-emitting layer 140, the electron transport layer 150, and the electron injection layer 160 thereon, and then depositing a material, which can be used as the cathode 170, thereon. In addition, a light-emitting auxiliary layer 220 may be formed between a hole transport layer 130 and a light-emitting layer 140, and an electron transport auxiliary layer(not shown) may be further formed between a light-emitting layer 140 and an electron transport layer 150 and, as described above, a stack structure may be formed.

In addition, the organic material layer may be manufactured in such a manner that the fewer layers are formed using various polymer materials by a soluble process or solvent process, for example, spin coating, nozzle printing, inkjet printing, slot coating, dip coating, roll-to-roll, doctor blading, screen printing, or thermal transfer, instead of deposition. Since the organic material layer according to the present invention may be formed in various ways, the scope of protection of the present invention is not limited by a method of forming the organic material layer.

The organic electric element according to an embodiment of the present invention may be of a top emission type, a bottom emission type, or a dual emission type depending on the material used.

In addition, the organic electric element according to an embodiment of the present invention may be selected from the group consisting of an organic electroluminescent element, an organic solar cell, an organic photo conductor, an organic transistor, an element for monochromatic illumination and an element for a quantum dot display.

Another embodiment of the present invention provides an electronic device including a display device which includes the above described organic electric element, and a control unit for controlling the display device. Here, the electronic device may be a wired/wireless communication terminal which is currently used or will be used in the future, and covers all kinds of electronic devices including a mobile communication terminal such as a cellular phone, a navigation unit, a game player, various kinds of TVs, and various kinds of computers.

Hereinafter, compound according to one aspect of the present invention will be described.

Compound according to an aspect of the present invention is represented by Formula 1 below.

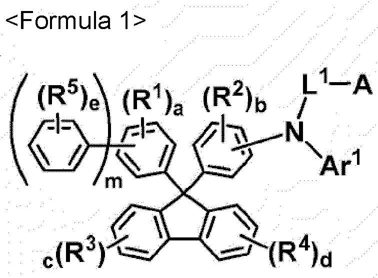

In Formula 1, each of symbols may be defined as follows.

A is the following Formula A-1 or Formula A-2.

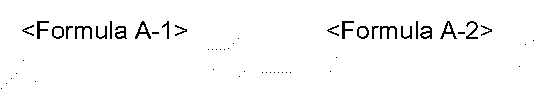

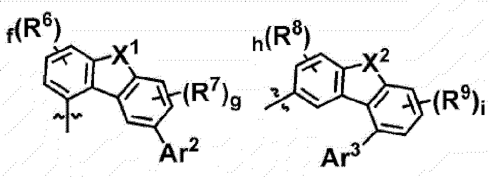

R¹ to R⁹ are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₆₀ aryloxy group and -L'-N(Rₐ)(R_{b}), and adjacent groups may be bonded to each other to form a ring.

a is an integer of 0 to 5, b to d are each an integer of 0 to 4, e is an integer of 0 to 5, m is an integer of 0 to 2, f to i are each an integer of 0 to 3, and when each of these is an integer of 2 or more, a plurality of R¹ each, R² each, R³ each, R⁴ each, R⁵ each, R⁶ each, R⁷ each, R⁸ each, and R⁹ each are equal to or different from each other.

A ring formed by at least one pair of adjacent groups, such as adjacent R¹s, adjacent R²s, adjacent R³s, adjacent R⁴s, adjacent R⁵s, adjacent R⁶s, adjacent R⁷s, adjacent R⁸s, and adjacent R⁹s, may be selected from the group consisting of a C₆-C₆₀ aromatic ring group, a fluorenylene group, a C₃-C₆₀ heterocyclic group containing at least one heteroatom of O, N, S, Si and P, and a C₆-C₆₀ aliphatic ring.

When an aromatic ring is formed by adjacent groups, the aromatic ring may be, for example, a C₆-C₂₀, a C₆-C₁₈, a C₆-C₁₆, a C₆-C₁₄, a C₆-C₁₃, a C₆-C₁₂, a C₆-C₁₀, a C₆, a C₁₀, a C₁₂, a C₁₄, a C₁₅, a C₁₆, or a C₁₈ aromatic ring, specifically, an aryl ring such as benzene, naphthalene, anthracene, phenanthrene, pyrene, etc.

X¹ and X² are independently O or S.

L¹ and L' are each independently selected from the group consisting of a single bond, C₆-C₆₀ arylene group, a fluorenylene group, a C₃-C₆₀ aliphatic ring group, and a C₂-C₆₀ heterocyclic group containing at least one heteroatom of O, N, S, Si and P.

Ar², Ar³, Rₐ and R_{b} are each independently selected from the group consisting of a C₆-C₆₀ aryl group, a fluorenyl group, a C₃-C₆₀ aliphatic ring group, and a C₂-C₆₀ heterocyclic group containing at least one heteroatom of O, N, S, Si and P.

When at least one of R¹ to R⁹, Ar², Ar³, Rₐ and R_{b} is an aryl group, the aryl group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₉, a C₆-C₁₈, a C₆-C₁₇, a C₆-C₁₆, a C₆-C₁₅, a C6-C14, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, or a C₁₈ aryl group, specifically, phenyl, biphenyl, naphthyl, terphenyl, phenanthrene, triphenylene, or the like.

When at least one of L¹ and L' is an arylene group, the arylene group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C6-C21, a C₆-C₂₀, a C₆-C₁₉, a C₆-C₁₈, a C₆-C₁₇, a C₆-C₁₆, a C₆-C₁₅, a C₆-C₁₄, a C6-C13, a C₆-C₁₂, a C₆-C₁₁, a C₆-C₁₀, a C₆, a C₁₀, a C₁₂, a C₁₃, a C₁₄, a C₁₅, a C₁₆, a C₁₇, a C₁₈ arylene group, specifically, phenylene, biphenyl, naphthylene, terphenyl, phenanthrene, triphenylene, or the like. Preferably, L³ may be phenyl, biphenyl or terphenyl.

When at least one of R¹ to R⁹, Ar², Ar³, Rₐ, R_{b}, L¹ and L' is a heterocyclic group, the heterocyclic group may be, for example, a C₂-C₃₀, a C₂-C₂₉, a C₂-C₂₈, a C₂-C₂₇, a C₂-C₂₆, a C₂-C₂₅, a C₂-C₂₄, a C₂-C₂₃, a C₂-C₂₂, a C₂-C₂₁, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, or a C₂₉ heterocyclic group, specifically, pyridine, pyrimidine, pyrazine, pyridazine, triazine, furan, pyrrole, silole, indene, indole, phenyl-indole, benzoindole, phenyl-benzoindole, pyrazinoindol, quinoline, isoquinoline, benzoquinoline, pyridoquinoline, quinazoline, benzoquinazoline, dibenzoquinazoline, phenanthroquinazoline, quinoxaline, benzoquinoxaline, dibenzoquinoxaline, benzofuran, naphthobenzofuran, dibenzofuran, dinaphthofuran, thiophene, benzothiophene, dibenzothiophene, naphthobenzothiophene, dinaphthothiophene, carbazole, phenyl-carbazole, benzocarbazole, phenyl-benzocarbazole, naphthyl-benzocarbazole, dibenzocarbazole, indolocarbazole, benzofuropyridine, benzothienopyridine, benzofuropyridine, benzothienopyrimidine, benzofuropyrimidine, benzothienopyrazine, benzofuropyrazine, benzoimidazole, benzothiazole, benzooxazole, benzosiloe, phenanthroline, dihydro-phenylphenazine, 10-phenyl-10H-phenoxazine, phenoxazine, phenothiazine, dibenzodioxin, benzodibenzodioxin, thianthrene, 9,9-dimethyl-9H-xanthene, 9,9-dimethyl-9H-thioxanthene, dihydrodimethylphenylacridine, spiro[fluorene-9,9'-xanthene] and the like.

When at least one of R¹ to R⁹, Ar², Ar³, Rₐ and R_{b} is a fluorenyl group or at least one of L¹ and L' are a fluorenylene group, the fluorenyl group or the fluorenylene group may be, for example, 9,9-dimethyl-9H-fluorene, 9,9-diphenyl-9H-fluorene, 9,9'-spirobifluorene, spiro[benzo[b]fluorene-11,9'-fluorene], benzo[b]fluorene, 11,11-diphenyl-11H-benzo[b]fluorene, 9-(naphthalen-2-yl)9-phenyl-9H-fluorene, and the like.

When at least one of R¹ to R⁹, Ar², Ar³, Rₐ and R_{b} is an aliphatic ring group, the aliphatic ring group, may be, for example, a C₃-C₂₀, a C₃-C₁₉, a C₃-C₁₈, a C₃-C₁₇, a C₃-C₁₆, a C₃-C₁₅, a C₃-C₁₄, a C₃-C₁₃, a C₃-C₁₂, a C₃-C₁₁, a C₃-C₁₀, a C₃-C₈, a C₃-C₆, a C₆, a C₁₀, a C₁₁, a C₁₂, a C₁₃, a C₁₄, a C₁₅, a C₁₆, a C₁₇ or a C₁₈ aliphatic ring group.

When at least one of R¹ to R⁹ is an alkyl group, the alkyl group may be, for example, a C₁-C₂₀, a C₁-C₁₀, a C₁-C₄, a C₁, a C₂, a C₃ and a C₄ alkyl group, for example, methyl, ethyl, t-butyl, etc.

The aryl group, the arylene group, the fluorenyl group, the fluorenylene group, the heterocyclic group, the aliphatic ring group, the alkyl group, the alkenyl group, the alkynyl group, the alkoxyl group, the aryloxyl group, and the ring formed by adjacent groups may be each optionally substituted with one or more substituents selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₃₀ aryloxy group, a C₁-C₂₀ alkylthio group, and a C₆-C₃₀ arylthio group.

When at least one of the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, the ring formed by adjacent groups is substituted with an aryl group, the aryl group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₉, a C₆-C₁₈, a C₆-C₁₇, a C₆-C₁₆, a C₆-C₁₅, a C₆-C₁₄, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, a C₂₉, or a C₃₀ aryl group.

When at least one of the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, the ring formed by adjacent groups is substituted with a heterocyclic group, the heterocyclic group may be, for example, a C₂-C₃₀, a C₂-C₂₉, a C₂-C₂₈, a C₂-C₂₇, a C₂-C₂₆, a C₂-C₂₅, a C₂-C₂₄, a C₂-C₂₃, a C₂-C₂₂, a C₂-C₂₁, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, a C₂₉, or a C₃₀ heterocyclic group.

When at least one of the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, the ring formed by adjacent groups is substituted with a fluorenyl group, the fluorenyl group may be 9,9-dimethyl-9H-fluorene, 9,9-diphenyl-9H-fluorene, 9,9'-spirobifluorene, spiro[benzo[b]fluorene-11,9'-fluorene], benzo[b]fluorene, 11,11-diphenyl-11H-benzo[b]fluorene, 9-(naphthalen-2-yl)9-phenyl-9H-fluorene, and the like.

When at least one of the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, the ring formed by adjacent groups is substituted with an aliphatic ring group, the aliphatic ring group may be, for example, a C₃-C₂₀, a C₃-C₁₉, a C₃-C₁₈, a C₃-C₁₇, a C₃-C₁₆, a C₃-C₁₅, a C₃-C₁₄, a C₃-C₁₃, a C₃-C₁₂, a C₃-C₁₁, a C₃-C₁₀, a C₃-C₈, a C₃-C₆, a C₆, a C₁₀, a C₁₁, a C₁₂, a C₁₃, a C₁₄, a C₁₅, a C₁₆, a C₁₇ or a C₁₈ aliphatic ring group.

When at least one of the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, the ring formed by adjacent groups is substituted with an alkyl group, the alkyl group may be, for example, a C₁-C₂₀, a C₁-C₁₀, a C₁-C₄, a C₁, a C₂, a C₃, or a C₄ alkyl group.

Ar¹ is selected from the group consisting of Formula Ar-1 to Ar-6: 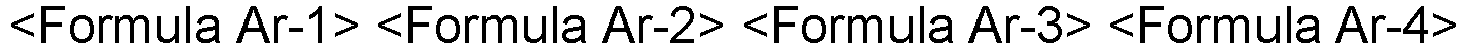 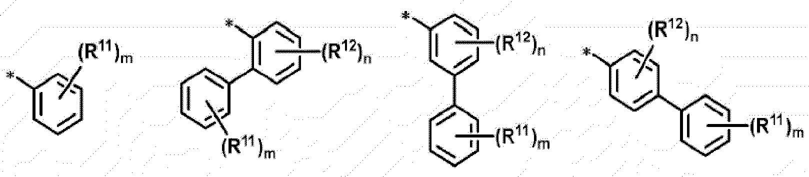 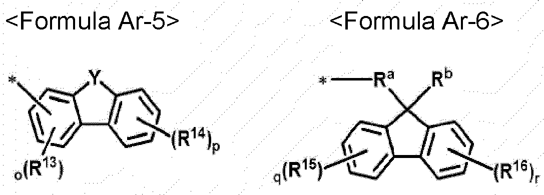
in Formula Ar-1 to Formula Ar-6,
R¹¹ to R¹⁶ are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and adjacent groups may be bonded to each other to form a C₆-C₁₄ aromatic ring group,
m' is an integer of 0 to 5, n, p, q and r are each an integer of 0 to 4, o is an integer of 0 to 3, and when each of these is an integer of 2 or more, each of R¹³s, each of R¹⁴s, each of R¹⁵s, each of R¹⁶s are the same as or different from each other,
Y is S, O, or N(R₃),
R^{a} is selected from the group consisting of a single bond, a C₁-C₂₀ alkylene group, a C₆-C₃₀ arylene group, a fluorenylene group, a C₂-C₃₀ heterocyclic group comprising at least one heteroatom selected from the group consisting of O, N, S, Si and P, and a C₃-C₃₀ aliphatic ring group, and R₃ and R^{b} are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group.

Formula 1 may be represented by one of Formula 2-1 to Formula 2-6.

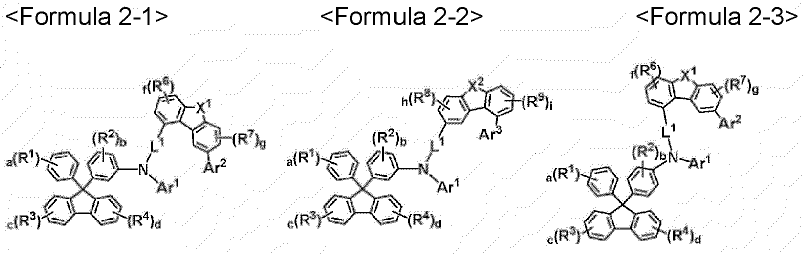

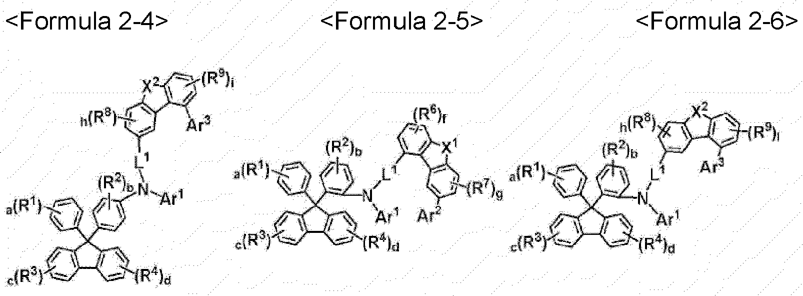

In Formula 2-1 to Formula 2-6, R¹ to R⁴, R⁶ to R⁹, X¹, X², L¹, Ar¹ to Ar³, a to d, f to i are the same as defined for Formula 1.

In addition, Formula 1 may be represented by one of Formula 3-1 to Formula 3-8.

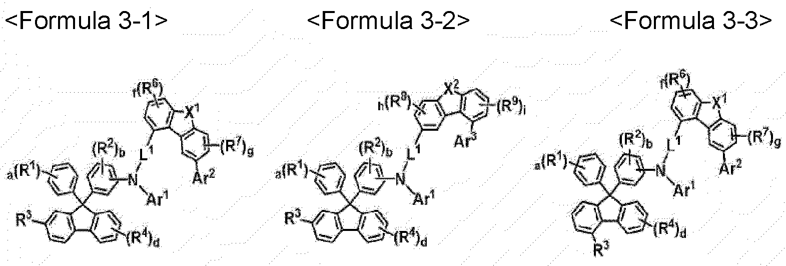

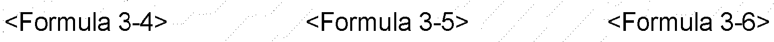

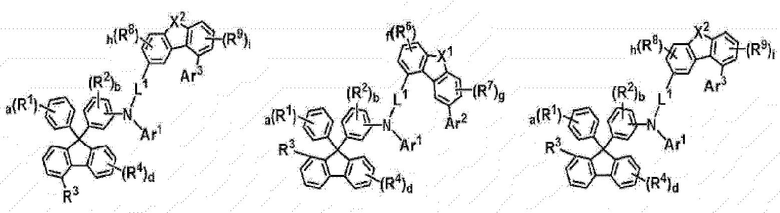

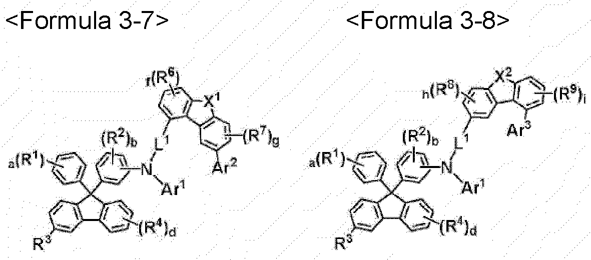

In Formula 3-1 to Formula 3-8, R¹, R², R⁴, R⁶ to R⁹, X¹, X², L¹, Ar¹ to Ar³, a, b, d, f to i are the same as defined for Formula 1.

R³ is selected from the group consisting of deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, and -L'-N(Rₐ)(R_{b}).

In addition, Formula 1 may be represented by one of Formula 4-1 to Formula 4-8.

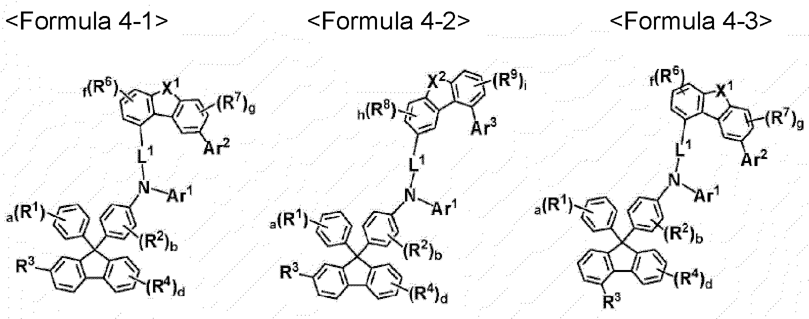

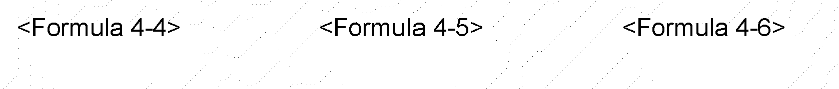

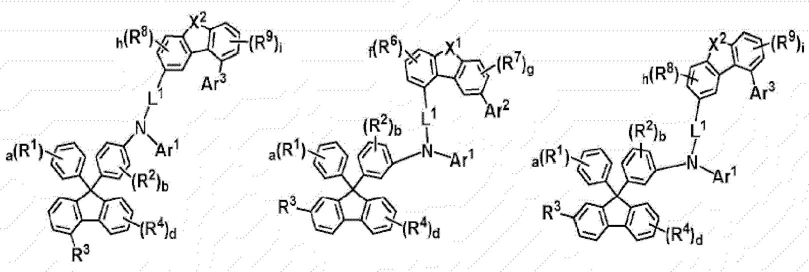

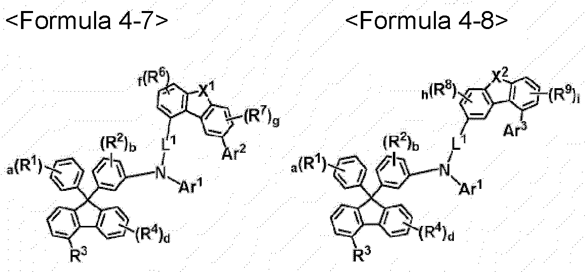

In Formula 4-1 to Formula 4-8, R¹, R², R⁴, R⁶ to R⁹, X¹, X², L¹, Ar¹ to Ar³, a, b, d, f to i are the same as defined for Formula 1.

R³ is selected from the group consisting of deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, and -L'-N(Rₐ)(R_{b}).

In addition, Formula 1 may be represented by Formula 5-1 or Formula 5-2.

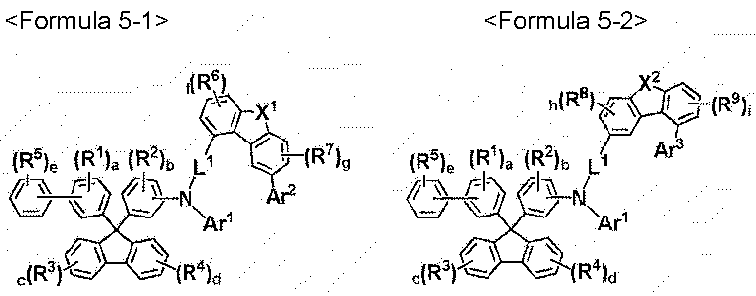

In Formula 5-1 and Formula 5-2, R¹ to R⁹, X¹, X², L¹, Ar¹ to Ar³, a to i are the same as defined for Formula 1.

In addition, Formula 1 may be represented by one of Formula 6-1 to Formula 6-6.

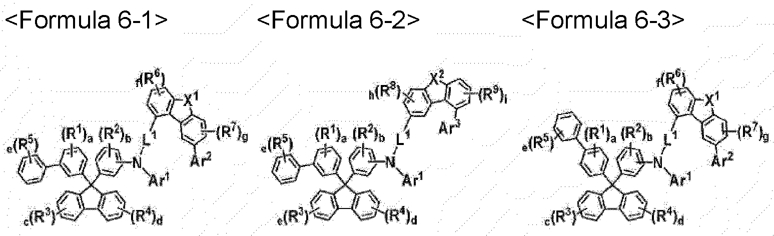

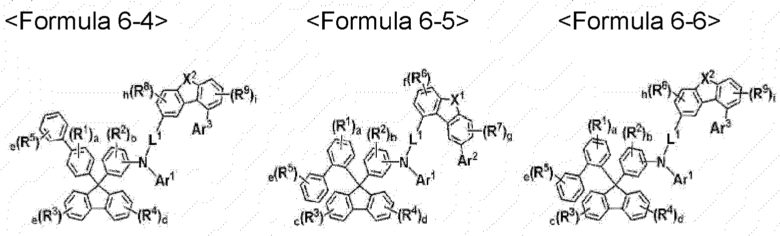

In Formula 6-1 to Formula 6-8, R¹ to R⁹, X¹, X², L¹, Ar¹ to Ar³, a to i are the same as defined for Formula 1.

In addition, Formula 1 may be represented by one of Formula 7-1 to Formula 7-6.

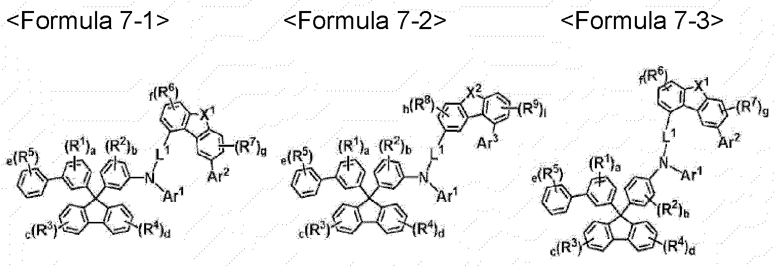

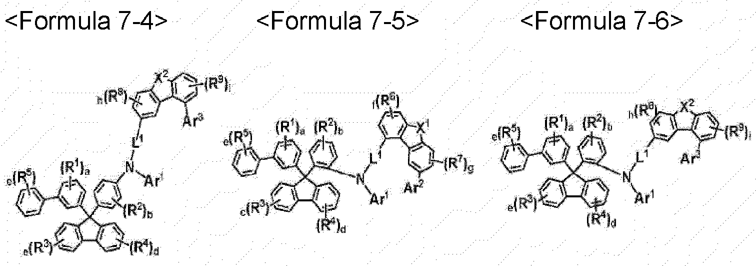

In Formula 7-1 to Formula 7-6, R¹ to R⁹, X¹, X², L¹, Ar¹ to Ar³, a to i are the same as defined for Formula 1.

The above Formulae, at least one of Ar² and Ar³ may be selected from the group consisting of Formula Ar-1 to Ar-6.

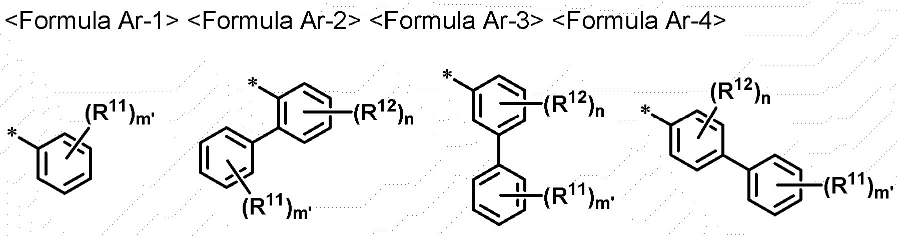

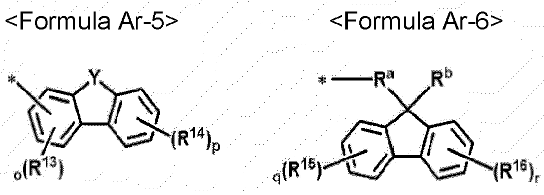

In Formula Ar-1 to Formula Ar-6, each of symbols may be defined as follows.

R¹¹ to R¹⁶ are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and adjacent groups may be bonded to each other to form a ring,

m' is an integer of 0 to 5, n, p, q and r are each an integer of 0 to 4, o is an integer of 0 to 3, and when each of these is an integer of 2 or more, each of R¹³s, each of R¹⁴s, each of R¹⁵s, each of R¹⁶s are the same as or different from each other,

A ring formed by adjacent groups may be selected from the group consisting of a C₆-C₆₀ aromatic ring group, a fluorenyl group, a C₃-C₆₀ heterocyclic group containing at least one heteroatom of O, N, S, Si and P, and a C₆-C₆₀ aliphatic ring.

When an aromatic ring is formed by adjacent groups, the aromatic ring may be, for example, a C₆-C₂₀, a C₆-C₁₈, a C₆-C₁₆, a C₆-C₁₄, a C₆-C₁₃, a C₆-C₁₂, a C₆-C₁₀, a C₆, a C₁₀, a C₁₂, a C₁₄, a C₁₅, a C₁₆, or a C₁₈ aromatic ring, specifically, an aryl ring such as benzene, naphthalene, anthracene, phenanthrene, pyrene, etc.

Y is S, O, C(R₁)(R₂) or N(R₃).

R^{a} is selected from the group consisting of a single bond, a C₁-C₂₀ alkylene group, a C₆-C₃₀ arylene group, a fluorenylene group, a C₂-C₃₀ heterocyclic group comprising at least one heteroatom selected from the group consisting of O, N, S, Si and P, and a C₃-C₃₀ aliphatic ring group.

R₁ to R₃, R^{b} are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and R₁ and R₂ may be bonded to each other to form a ring. When R₁ and R₂ are bonded to each other to form a ring, a spiro compound, for example, spirobifluorene, may be formed.

Both Ar² and Ar³ in <Formula 1> may be a C₆-C₆₀ aryl group.

Specifically, the compound represented by Formula 1 may be one of the following compounds, but there is no limitation thereto. However, P1-57 to 64, P1-66 to 68, P1 76-80, P1-90 to 92, P1-96, P1-100, P1-105 to 106, P2-57 to 64, P2-66 to 68, P2 76-80, P2-90 to 92, P2-96, P2-100, P2-105 to 106, P3-57 to 64, P3-66 to 68, P3 76-80, P3-90 to 92, P3-96, P3-100, and P3-105 to 106 are reference compounds.

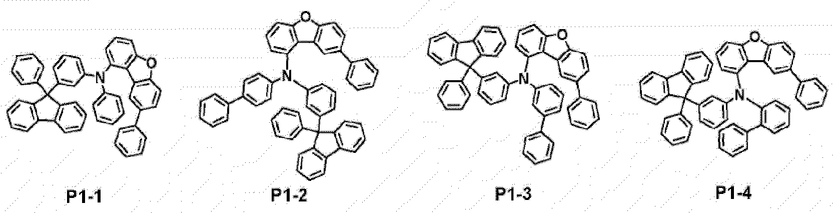

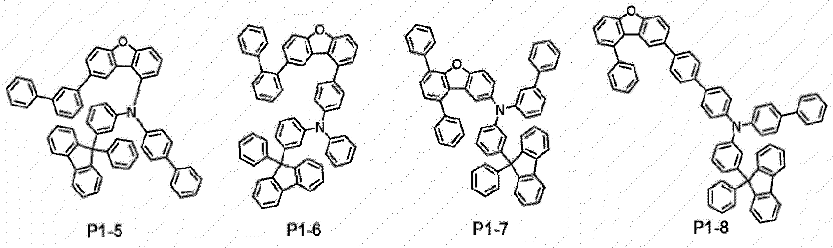

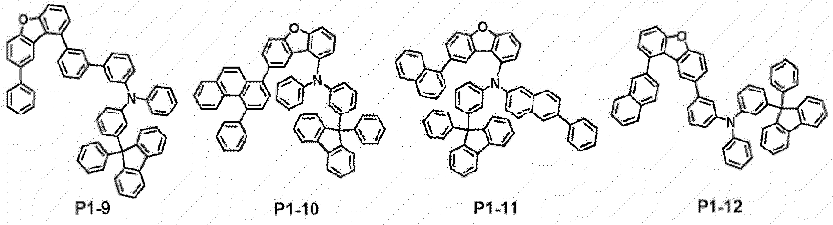

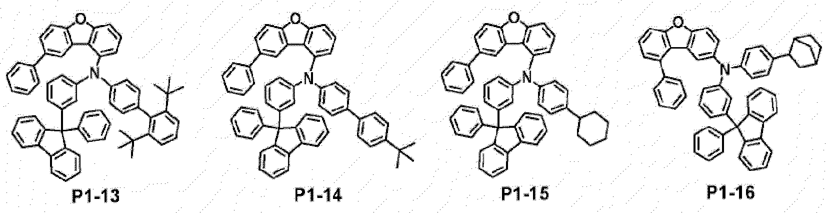

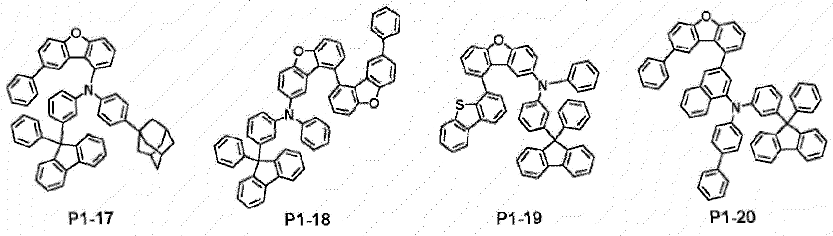

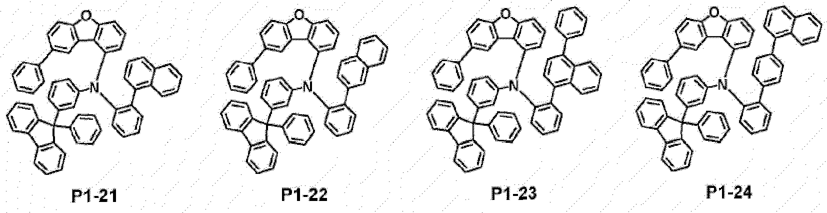

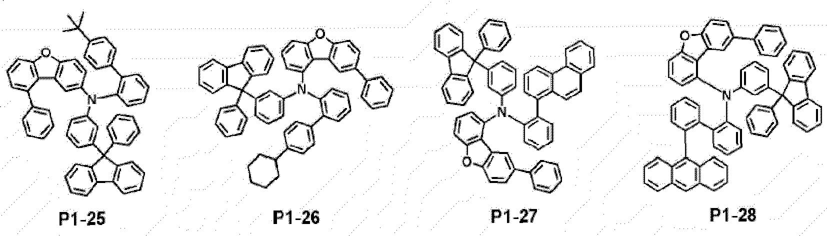

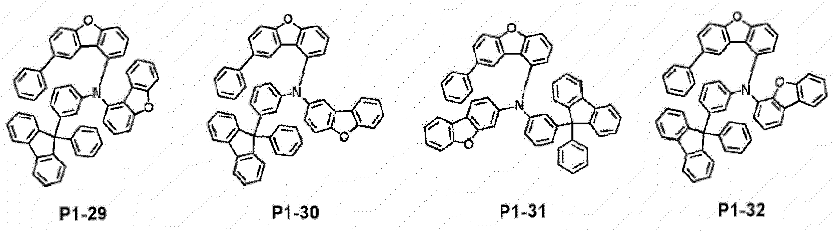

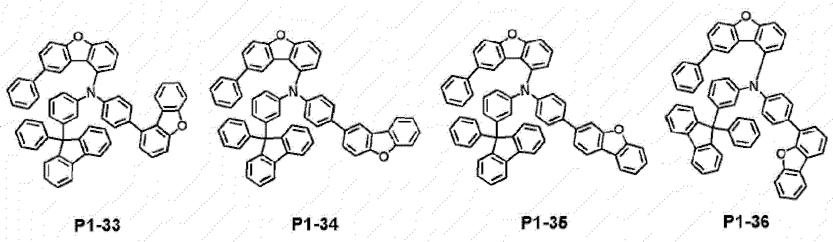

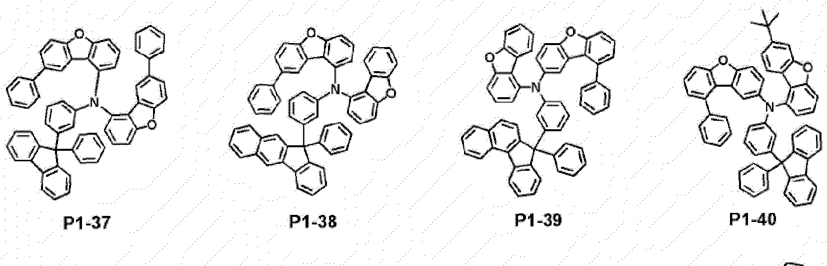

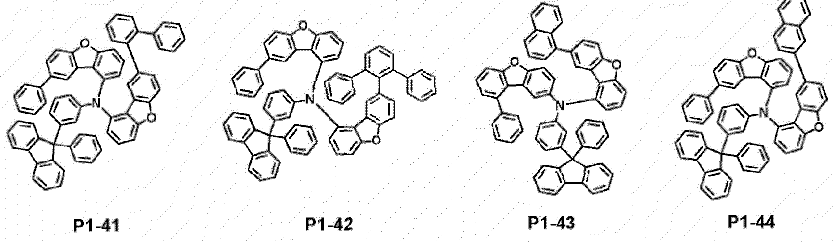

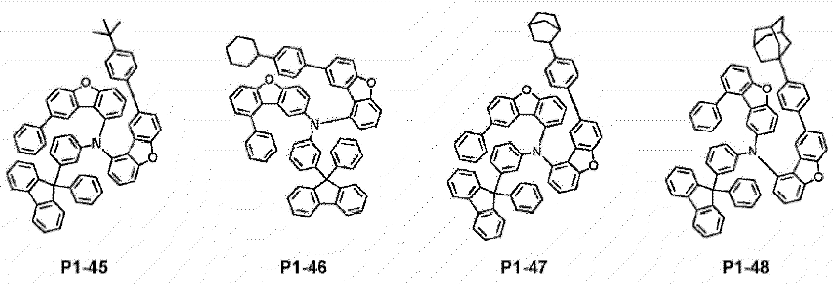

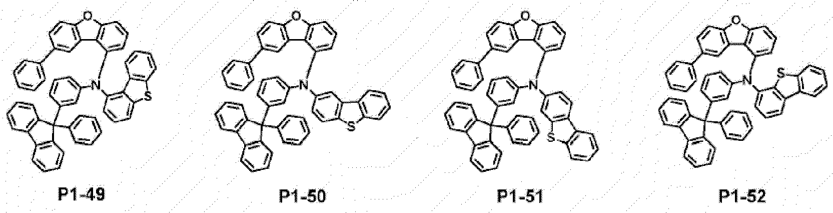

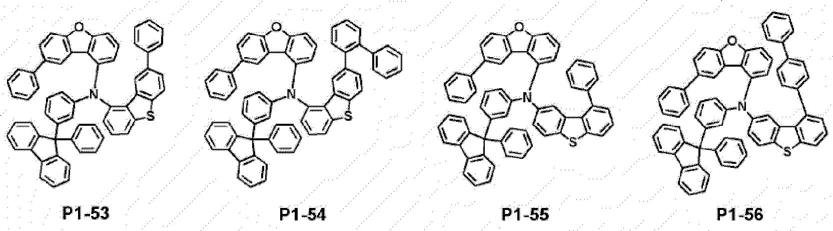

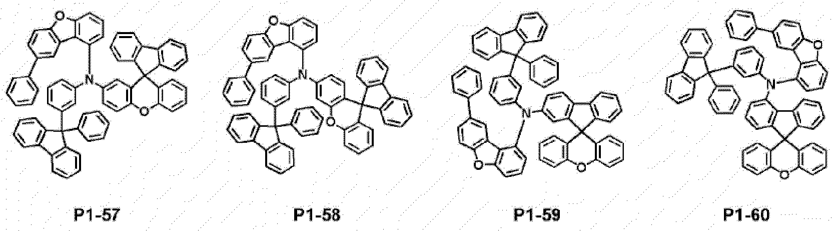

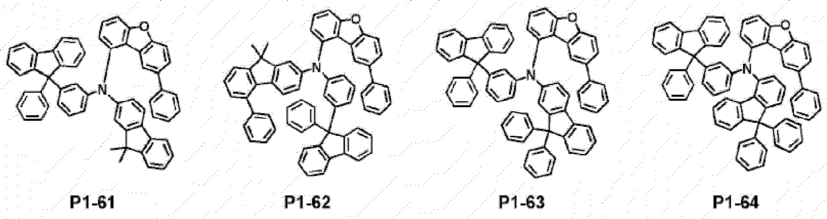

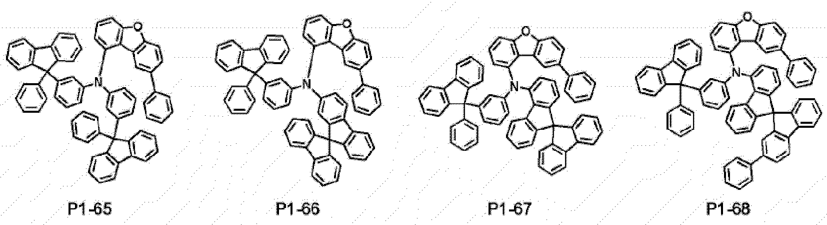

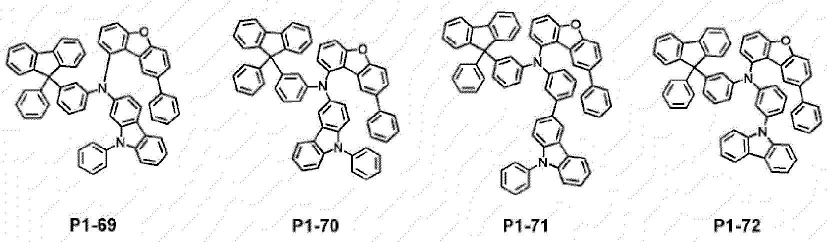

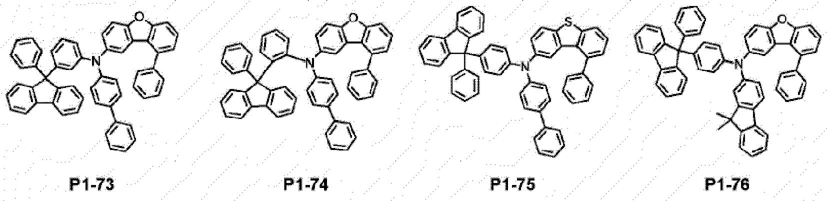

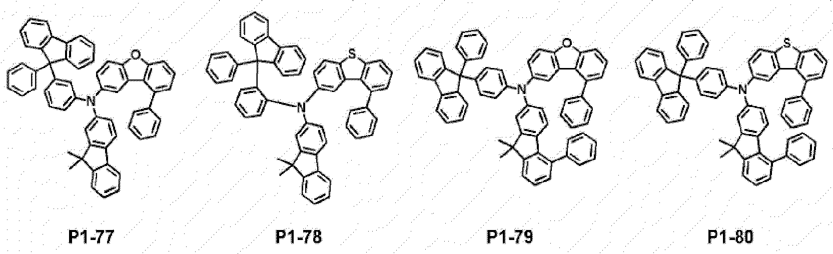

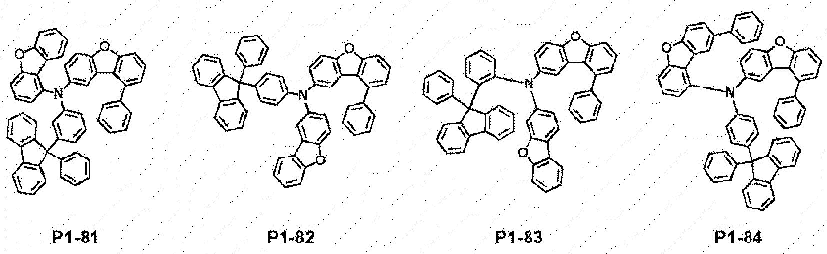

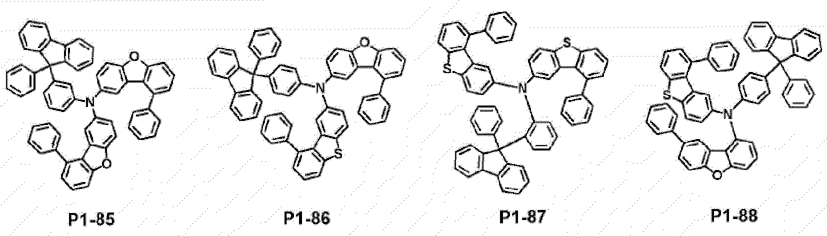

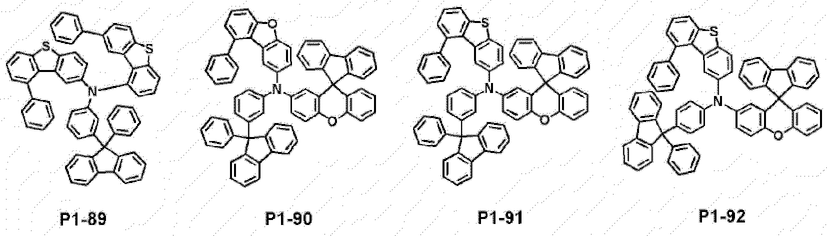

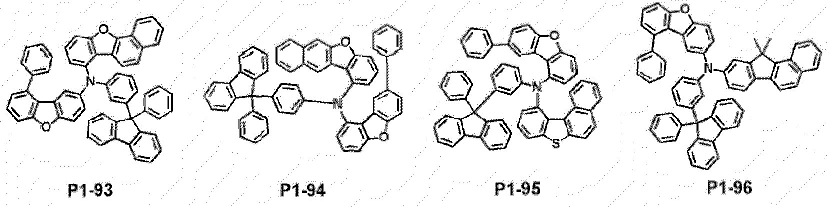

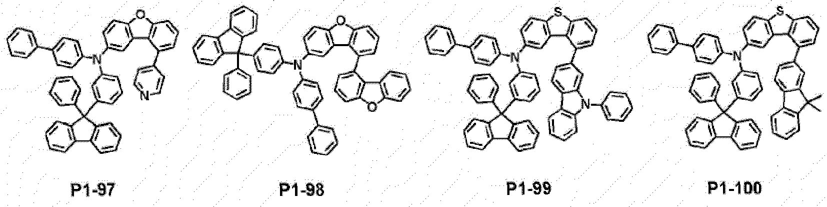

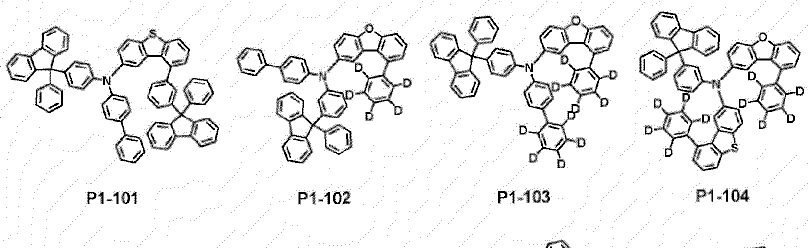

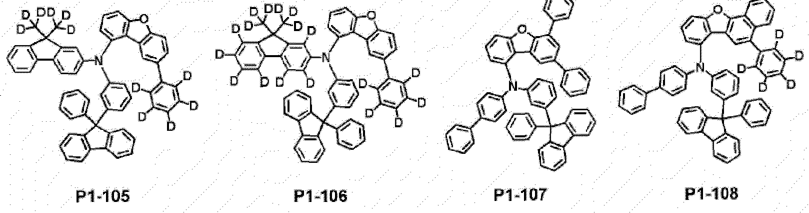

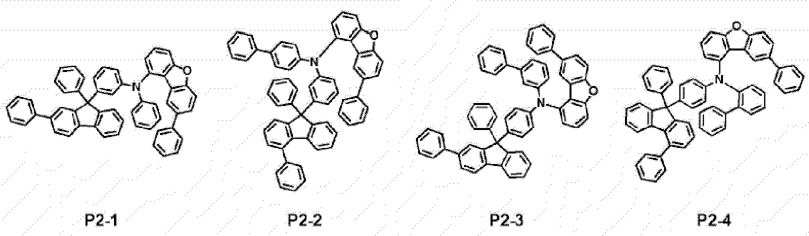

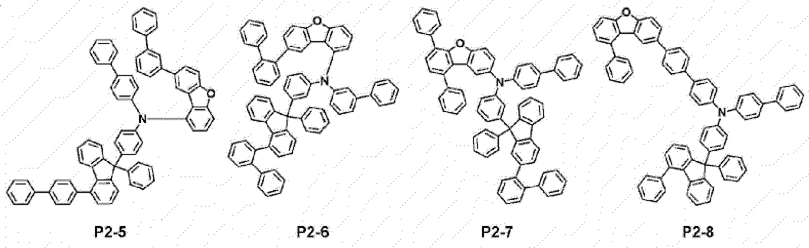

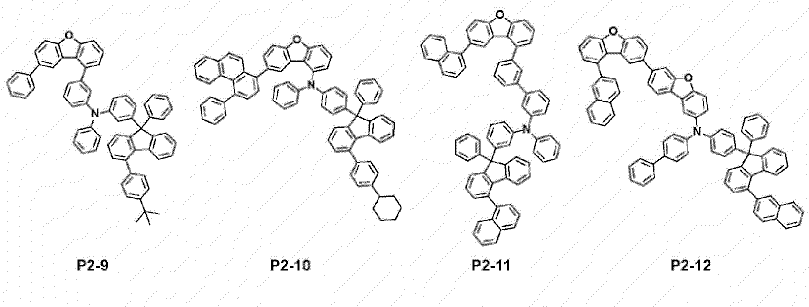

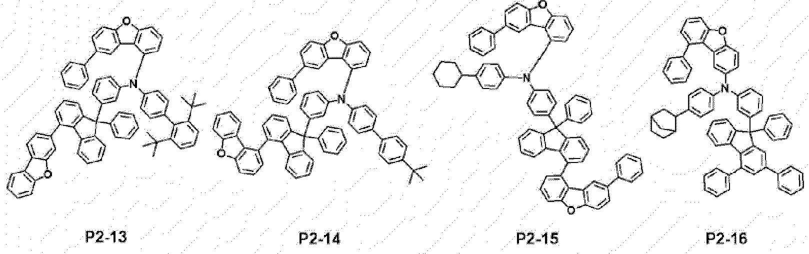

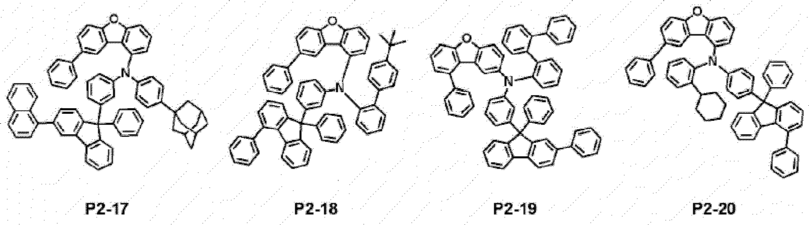

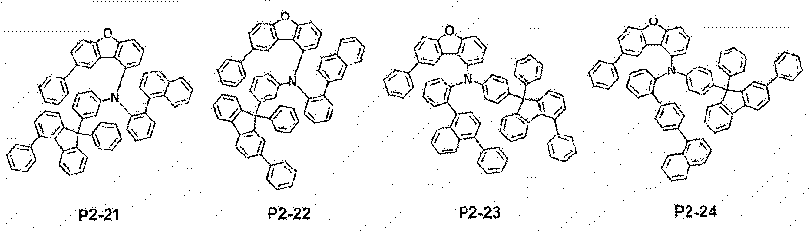

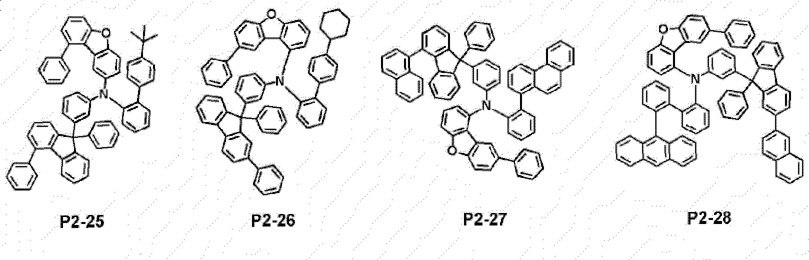

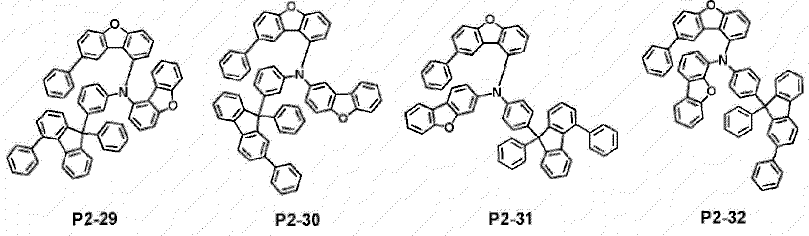

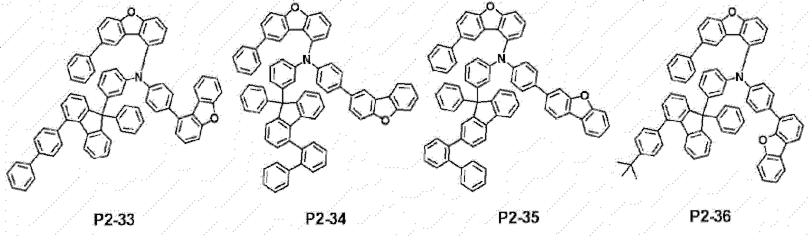

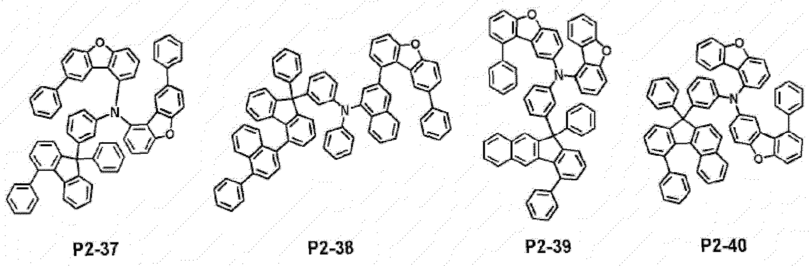

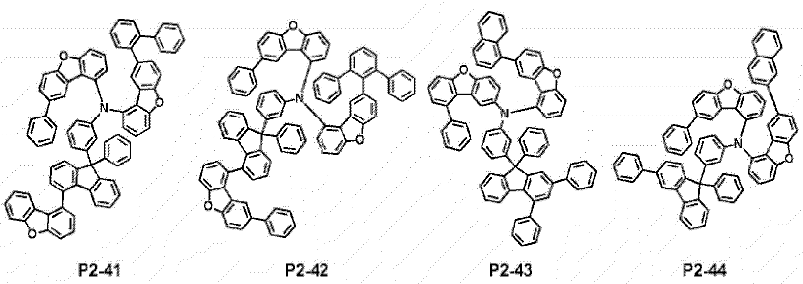

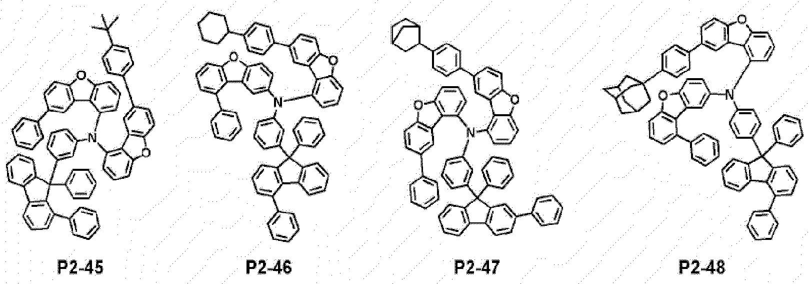

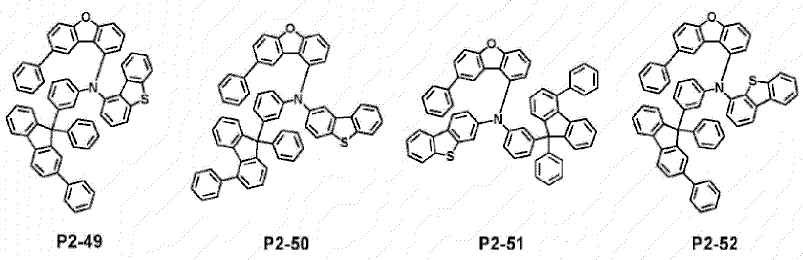

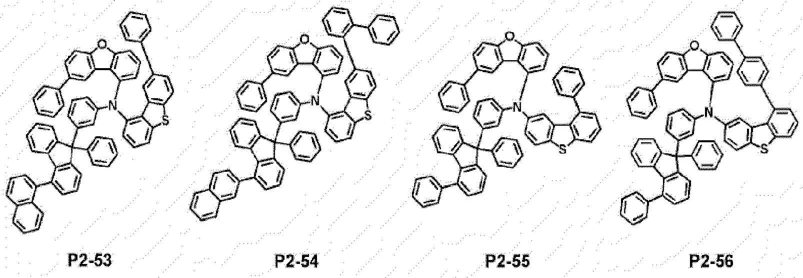

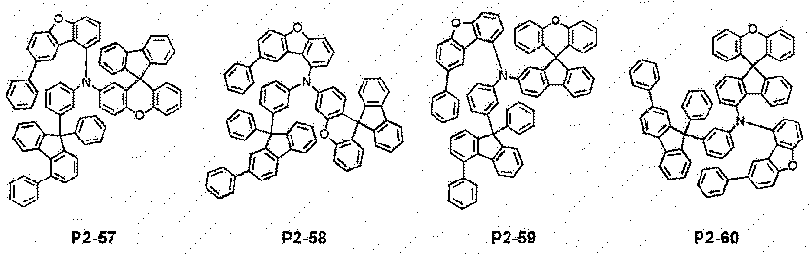

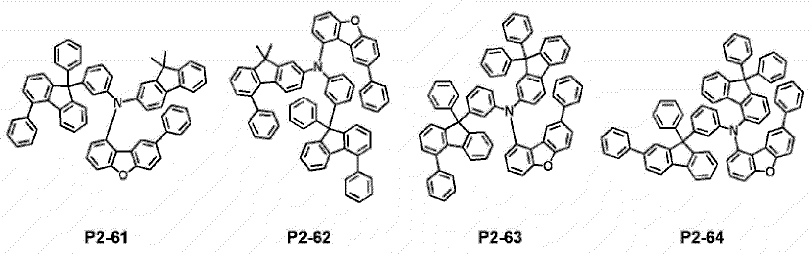

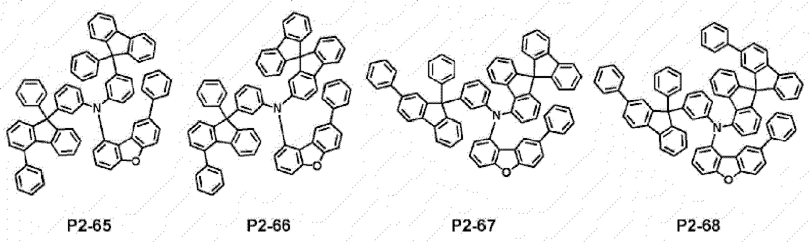

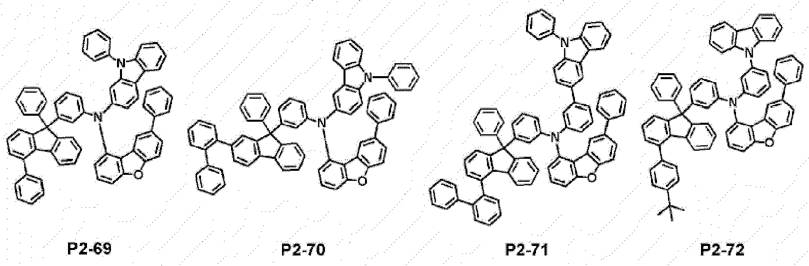

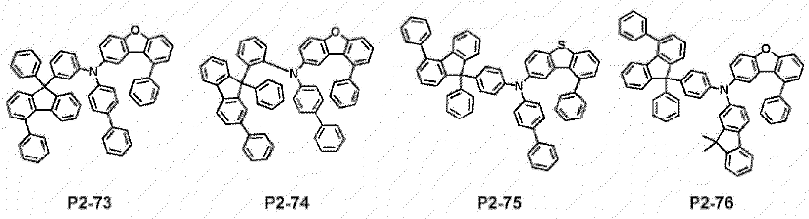

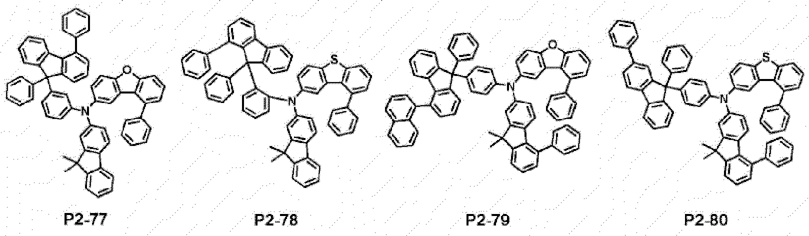

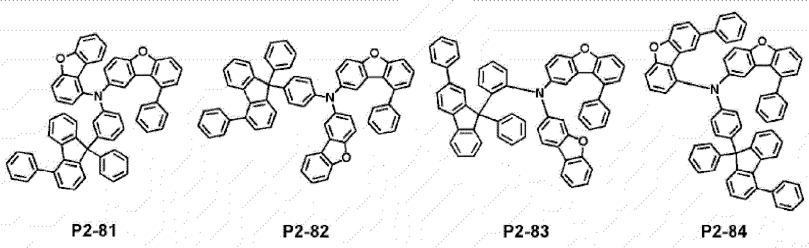

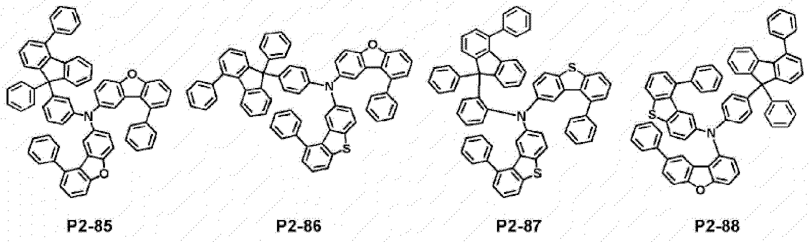

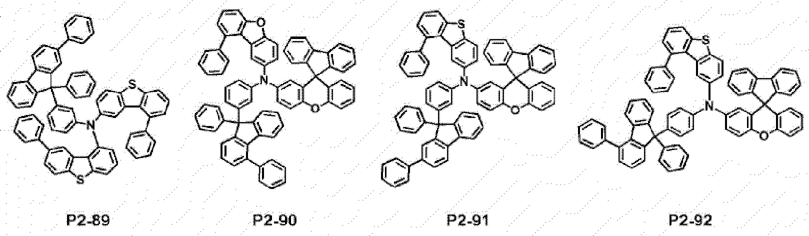

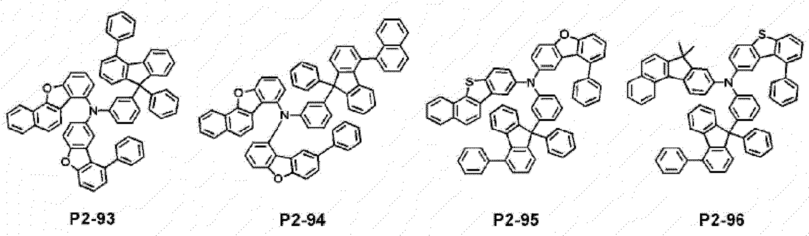

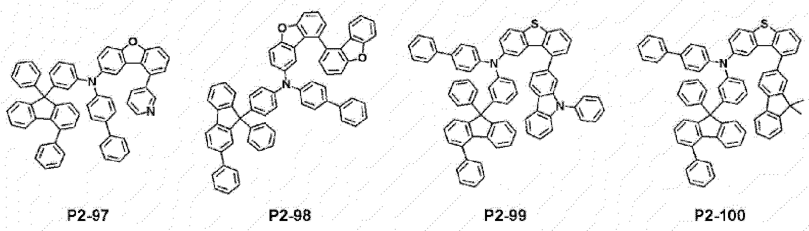

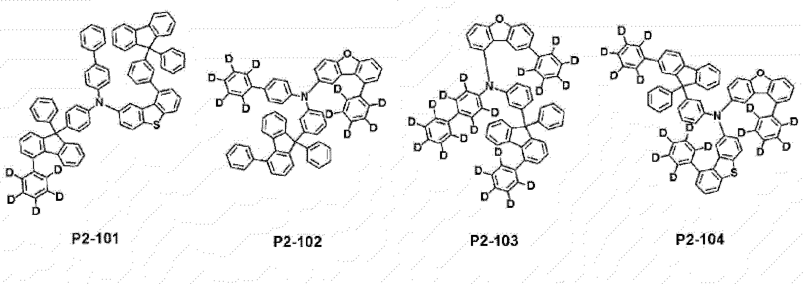

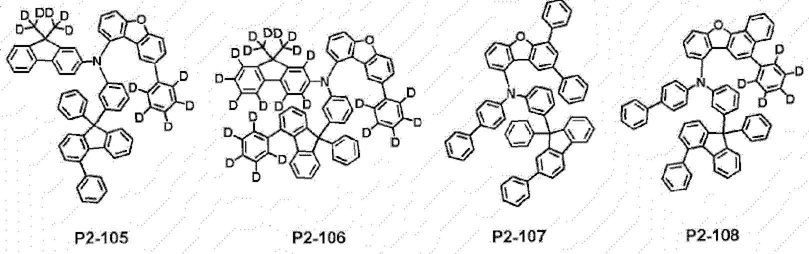

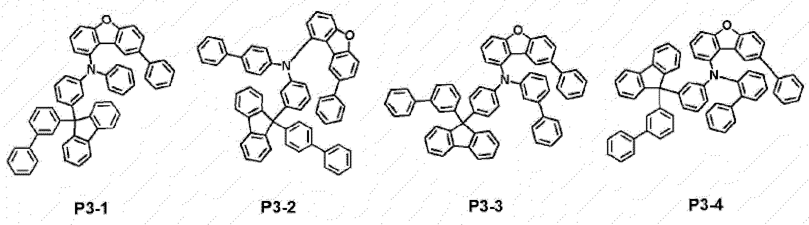

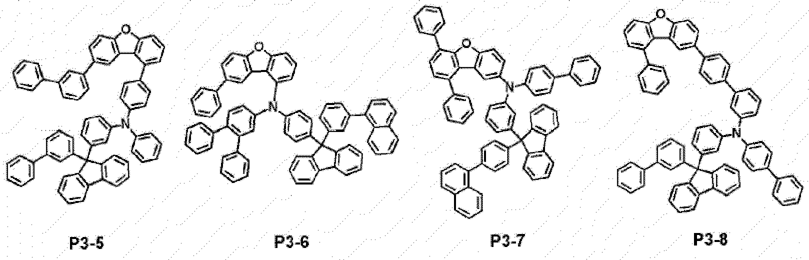

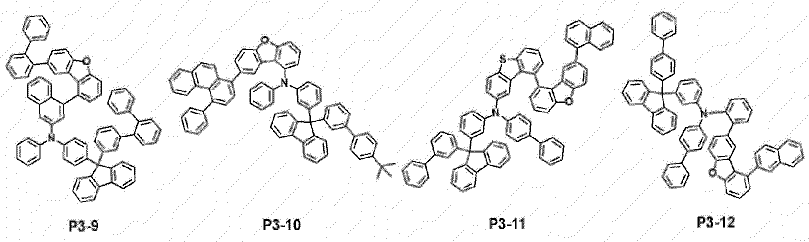

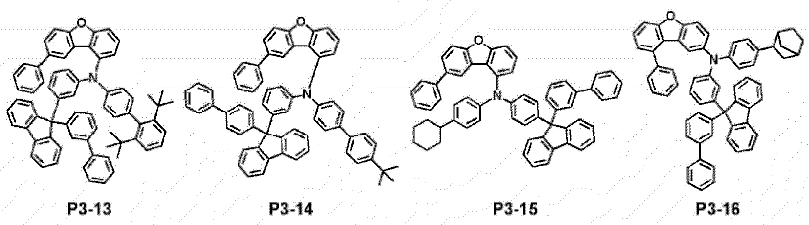

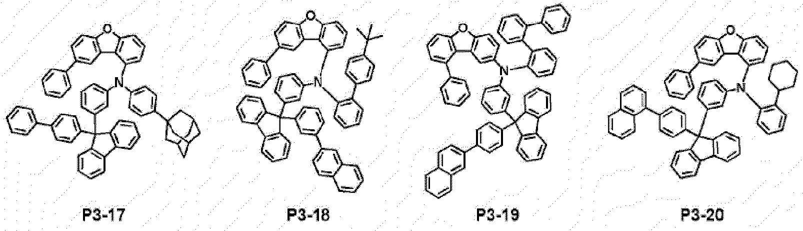

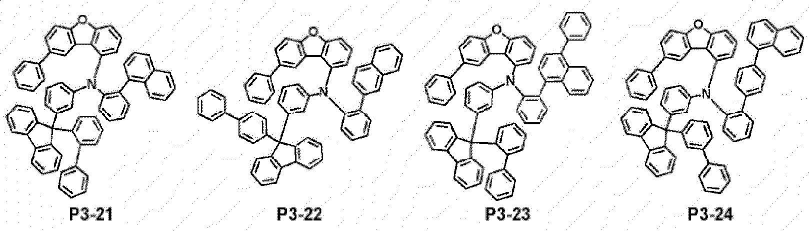

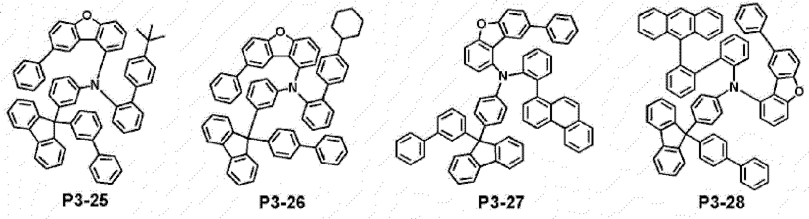

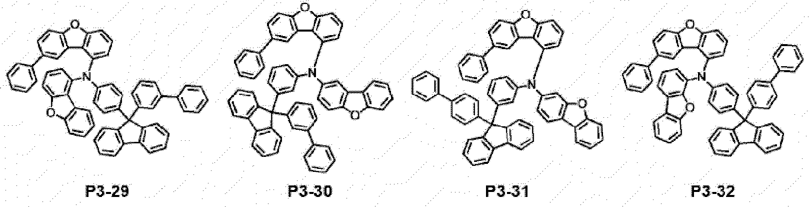

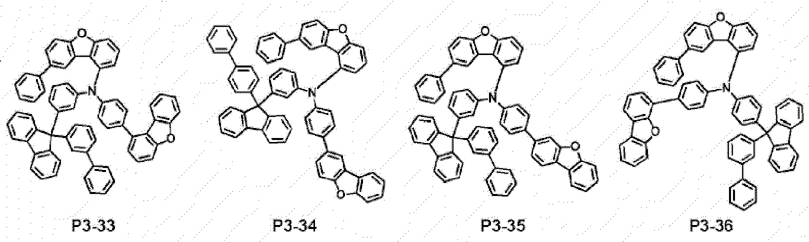

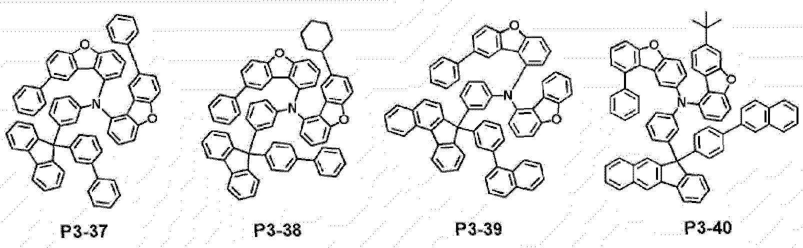

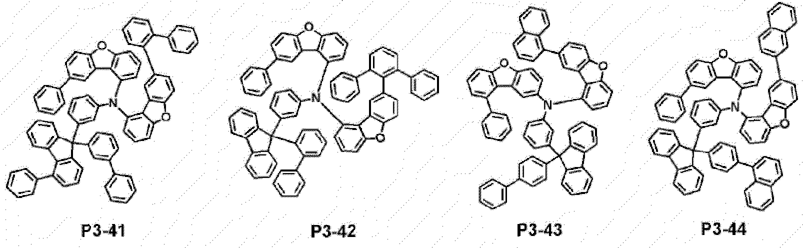

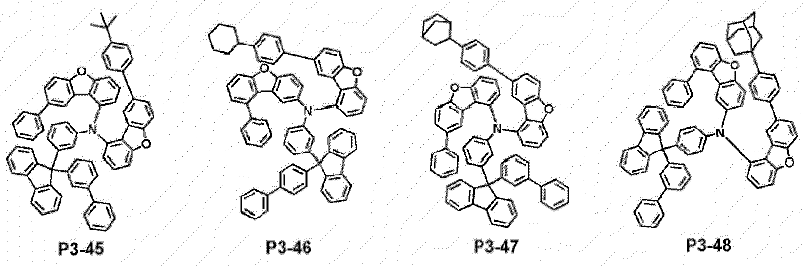

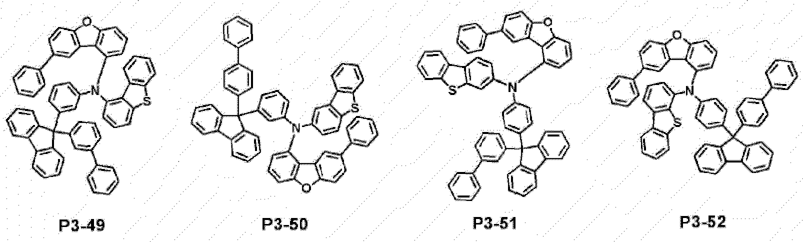

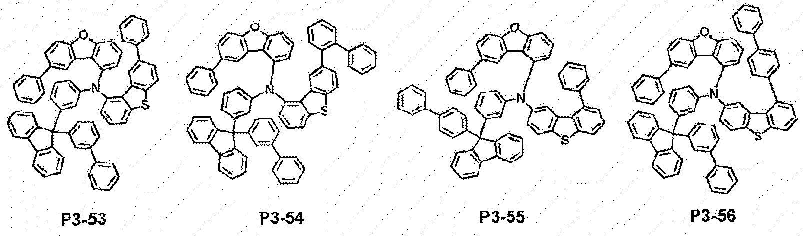

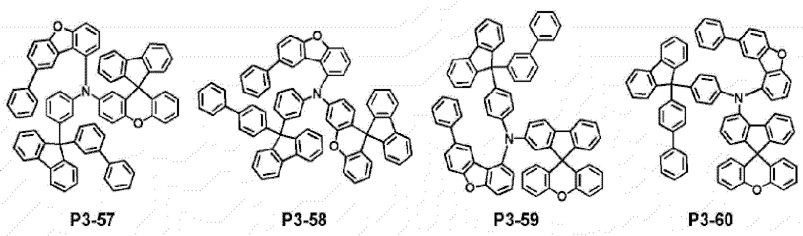

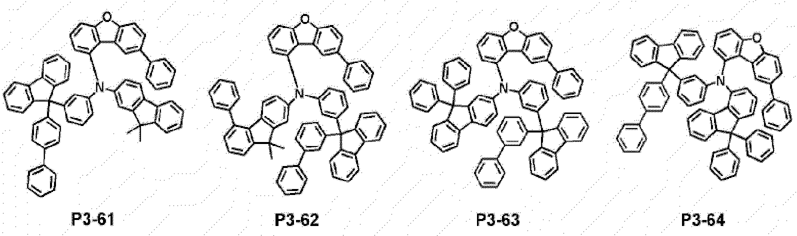

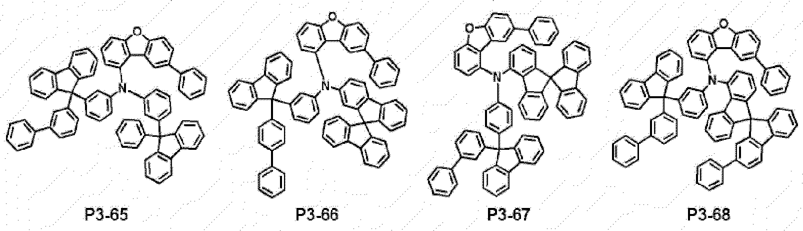

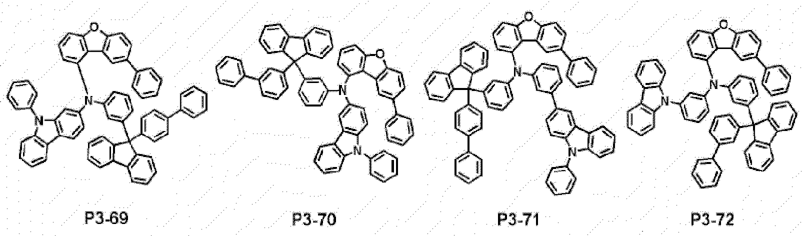

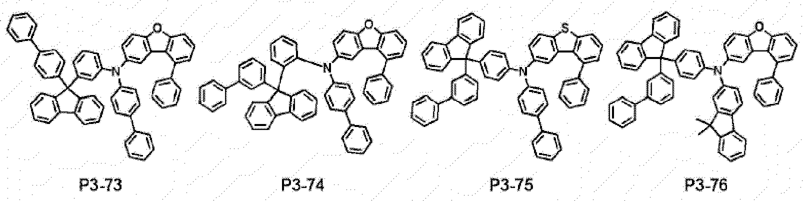

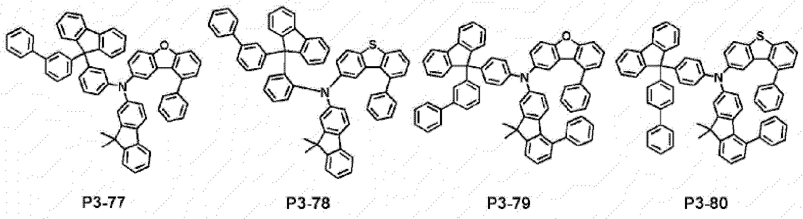

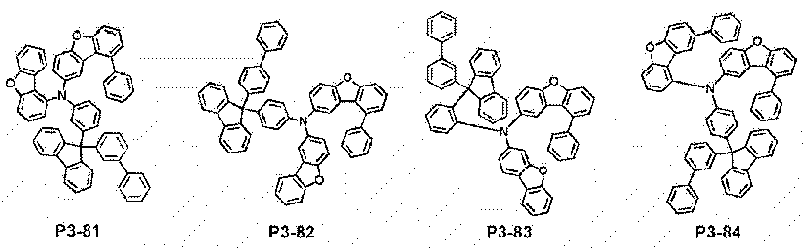

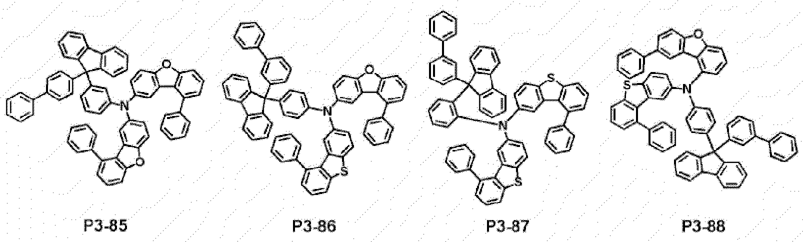

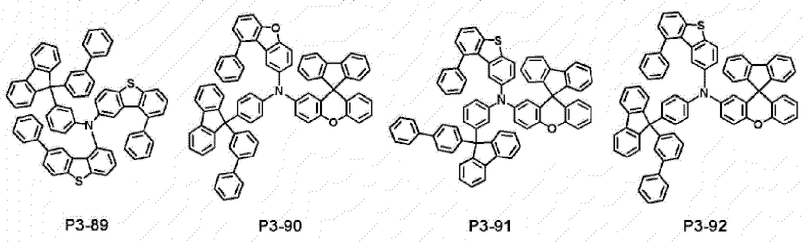

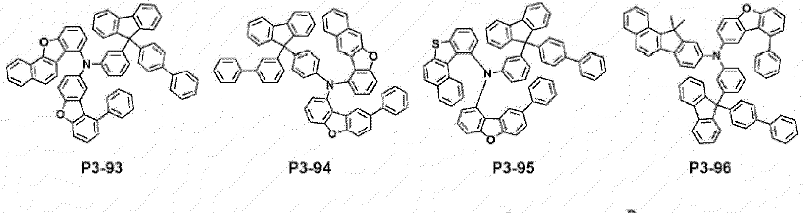

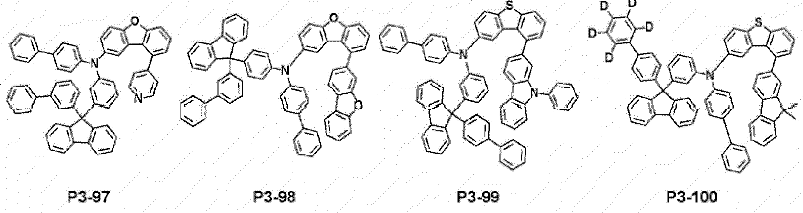

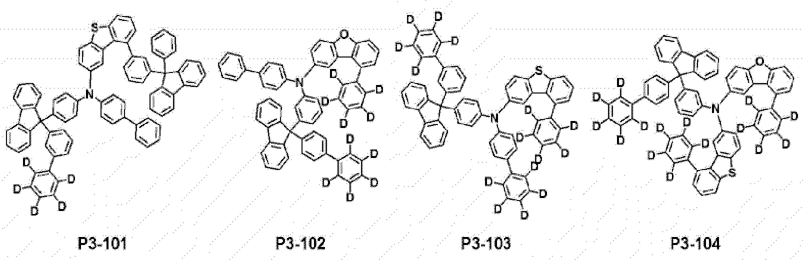

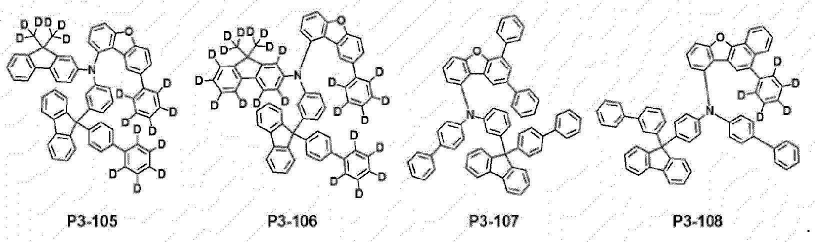

In another aspect of the present invention, the present invention provides an organic electric element comprising a first electrode, a second electrode, and an organic material layer between the first electrode and the second electrode, wherein the organic material layer includes compound represented by the Formula 1.

The organic material layer further includes a light-emitting layer and a light-emitting auxiliary layer between the light-emitting layer and the first electrode, and the light-emitting auxiliary layer includes the compound.

The organic material layer comprises two or more stacks, and the two or more stacks each comprise a hole transport layer, a light-emitting layer and an electron transport layer formed sequentially on the first electrode. Here, the organic material layer may comprise a charge generation layer formed between the two or more stacks.

The organic electric element may further include a layer for improving luminous efficiency. Here, the layer for improving luminous efficiency is formed on one side of two sides of a first electrode or a second electrode, the one side is not facing the organic material layer

In another aspect of the present invention, the present invention provides an electronic device comprising a display device and a control unit for controlling the display device, wherein the display device comprises the compound represented by Formula 1.

Hereinafter, a synthesis example of the compound represented by Formula 1 and a preparation method of an organic electroluminescent element will be described in detail by way of examples. However, the present invention is not limited to the following examples.

### [Synthesis example]

The compound (final product) represented by Formula 1 according to the present invention can be synthesized by reacting Sub 1 and Sub 2 as in the following reaction scheme 1, but is not limited thereto.

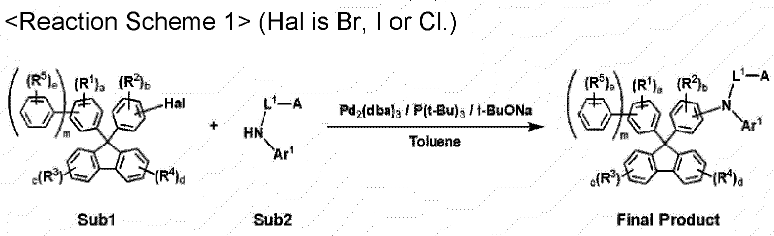

Sub 2 is the following Sub2-A or Sub2-B.

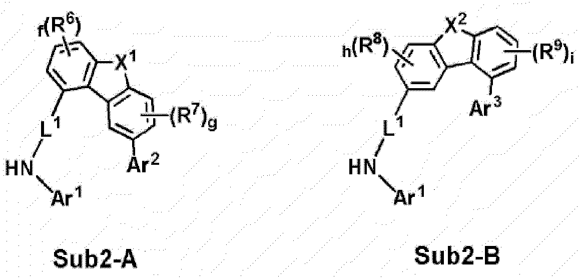

### Synthesis example of Sub 1

Sub 1 of the above reaction scheme 1 can be synthesized by the reaction path of the following reaction scheme 2-1 or reaction scheme 2-2, but is not limited thereto.

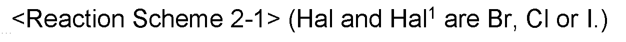

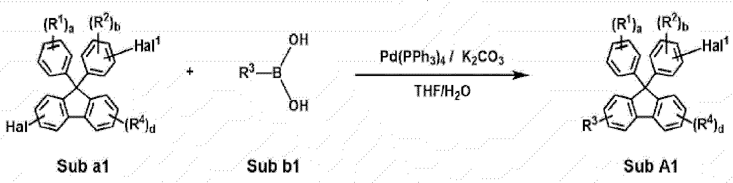

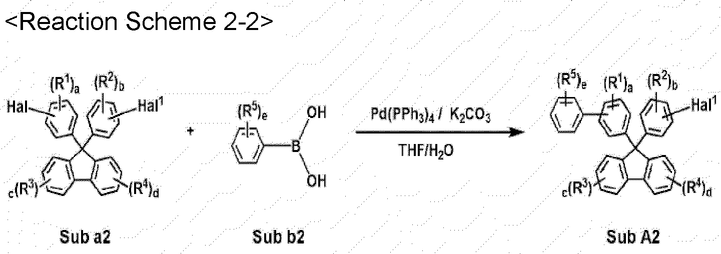

### 1. Synthesis example of Sub1-3

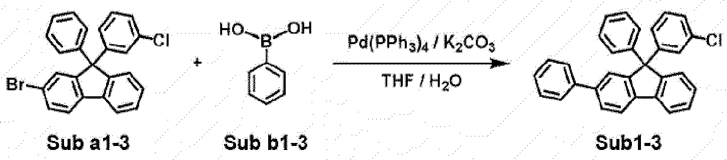

Sub a1-3 (50 g, 115.80 mmol) and Sub b1-3 (15.5g, 127.38 mmol) were placed in a round-bottom flask and dissolved in THF (290mL) and water (100mL). Then Pd(PPh₃)₄ (4.01 g, 3.47 mmol) and K₂CO₃ (32.00 g, 231.61 mmol) were added to the solution and the mixture was stirred at 60 °C. When the reaction was completed, the reaction product was extracted with toluene and water, dried with MgSO₄, and concentrated. Then, the concentrate was separated using a silica column and recrystallized to obtain 43.8 g (yield: 88.2%) of the product.

### 2. Synthesis example of Sub 1-5

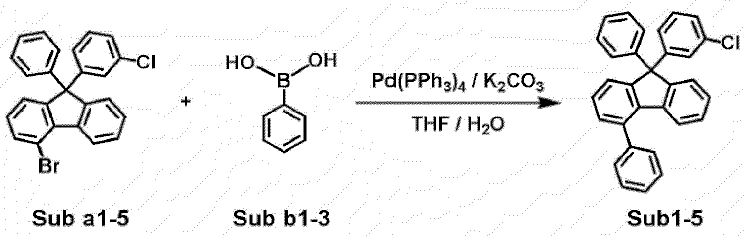

The same method as the synthesis of Sub1-3 was carried out using Sub a1-5 instead of Sub a1-3, to obtain 42.4 g of the product (yield: 85.4%).

### 3. Synthesis example of Sub1-18

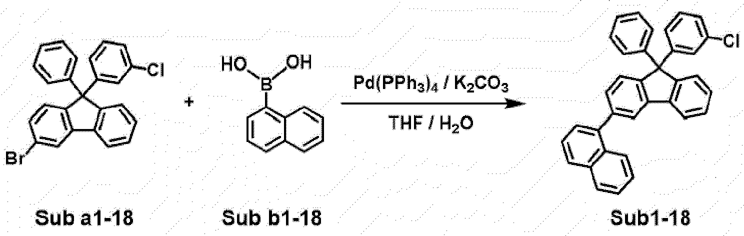

The same method as the synthesis of Sub1-3 was carried out using Sub a1-18 instead of Sub a1-3 and Sub b1-18 (21.9 g, 127.38 mmol) instead of Sub b1-3, to obtain 48.7 g of the product (yield: 87.8%).

### 4. Synthesis example of Sub1-20

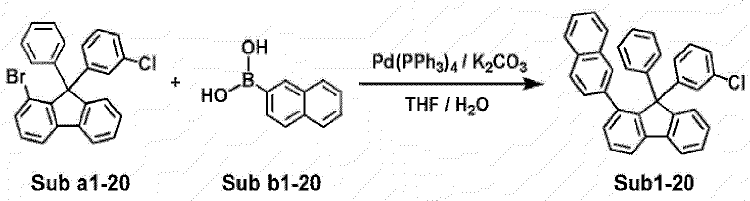

The same method as the synthesis of Sub1-3 was carried out using Sub a1-20 instead of Sub a1-3 and Sub b1-20 (21.9 g, 127.38 mmol) instead of Sub b1-3, to obtain 49.2 g of the product (yield: 88.7%).

### 5. Synthesis example of Sub1-24

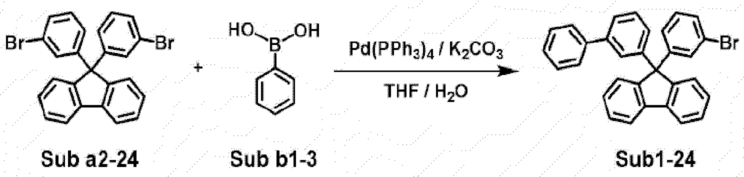

Sub a2-24 (50 g, 104.99 mmol) and Sub b1-3 (14.1g, 115.49 mmol) were placed in a round-bottom flask and dissolved in THF (270mL) and water (90mL), Pd(PPh₃)₄ (3.63 g, 3.15 mmol), K₂CO₃ (29.02 g, 209.99 mmol) were added thereto. Then the reaction was carried out in the same manner as the synthesis method of Sub 1-3 to obtain 12.9 g (yield: 25.9 %) of product.

### 6. Synthesis example of Sub 1-25

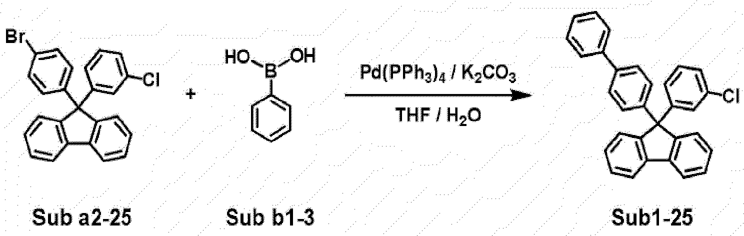

The same synthesis method as Sub1-3 was carried out using Sub a2-25 instead of Sub a1-3, to obtain 42.1 g of the product (yield: 84.7%).

Compounds belonging to Sub 1 may be, but not limited to, the following compounds, and Table 1 shows FD-MS(Field Desorption-Mass Spectrometry) values of the following compounds.

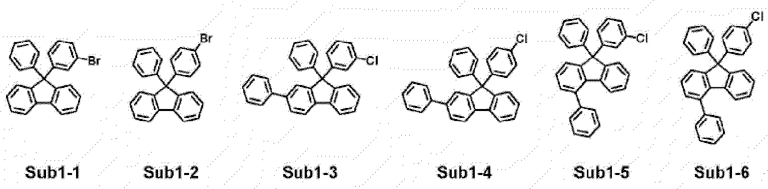

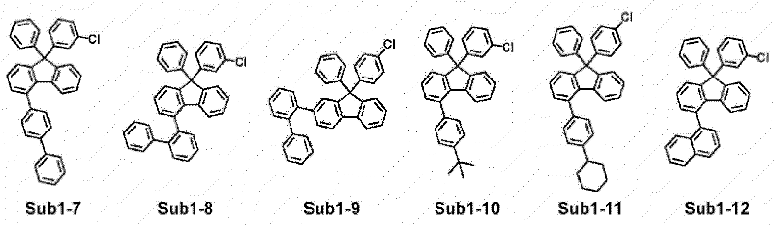

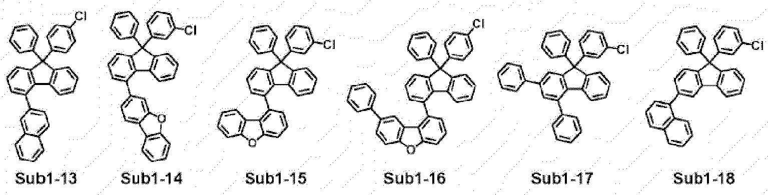

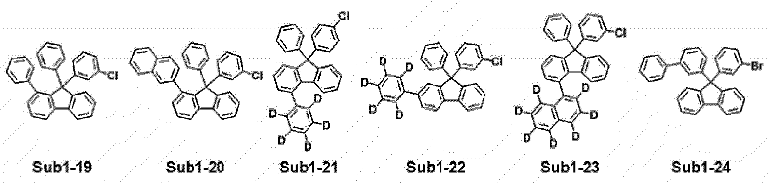

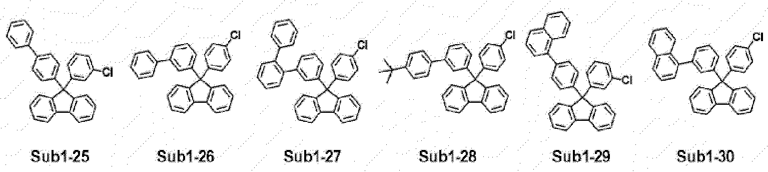

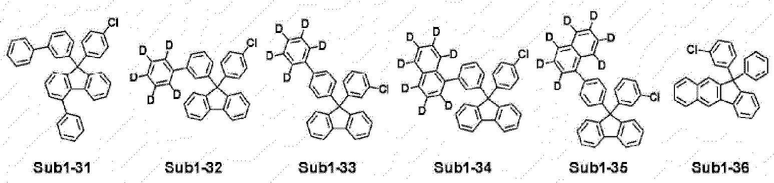

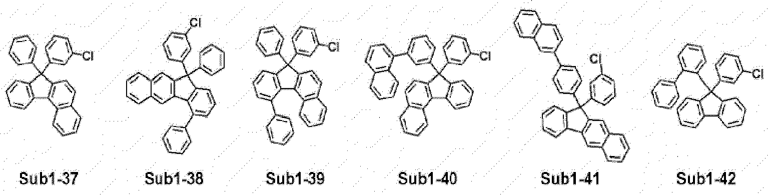

**[Table 1]**

| **Compound** | **FD-MS** | **Compound** | **FD-MS** |
|---|---|---|---|
| **Sub 1-1** | **m/z=396.05 (C₂₆H₁₇Br=397.32)** | **Sub 1-2** | **m/z=396.05 (C₂₆H₁₇Br=397.32)** |
| **Sub 1-3** | **m/z=428.13 (C₃₁H₂₁Cl=428.96)** | **Sub 1-4** | **m/z=428.13 (C₃₁H₂₁Cl=428.96)** |
| **Sub 1-5** | **m/z=428.13 (C₃₁H₂₁Cl=428.96)** | **Sub 1-6** | **m/z=428.13 (C₃₁H₂₁Cl=428.96)** |
| **Sub 1-7** | **m/z=504.16 (C₃₇H₂₅Cl=505.06)** | **Sub 1-8** | **m/z=504.16 (C₃₇H₂₅Cl=505.06)** |
| **Sub 1-9** | **m/z=504.16 (C₃₇H₂₅Cl=505.06)** | **Sub 1-10** | **m/z=484.20 (C₃₅H₂₉Cl=485.07)** |
| **Sub 1-11** | **m/z=510.21 (C₃₇H₃₁Cl=511.11)** | **Sub 1-12** | **m/z=478.15 (C₃₅H₂₃Cl=479.02)** |
| **Sub 1-13** | **m/z=478.15 (C₃₅H₂₃Cl=479.02)** | **Sub 1-14** | **m/z=518.14 (C₃₇H₂₃ClO=519.04)** |
| **Sub 1-15** | **m/z=518.14 (C₃₇H₂₃ClO=519.04)** | **Sub 1-16** | **m/z=594.18 (C₄₃H₂₇ClO=595.14)** |
| **Sub 1-17** | **m/z=504.16 (C₃₇H₂₅Cl=505.06)** | **Sub 1-18** | **m/z=478.15 (C₃₅H₂₃Cl=479.02)** |
| **Sub 1-19** | **m/z=428.13 (C₃₁H₂₁Cl=428.96)** | **Sub 1-20** | **m/z=478.15 (C₃₅H₂₃Cl=479.02)** |
| **Sub 1-21** | **m/z=433.16 (C₃₁H₁₆D₅Cl=433.99)** | **Sub 1-22** | **m/z=433.16 (C₃₁H₁₆D₅Cl=433.99)** |
| **Sub 1-23** | **m/z=485.19 (C₃₅H₁₆D₇Cl=486.06)** | **Sub 1-24** | **m/z=472.08 (C₃₁H₂₁Br=473.41)** |
| **Sub 1-25** | **m/z=428.13 (C₃₁H₂₁Cl=428.96)** | **Sub 1-26** | **m/z=428.13 (C₃₁H₂₁Cl=428.96)** |
| **Sub 1-27** | **m/z=504.16 (C₃₇H₂₅Cl=505.06)** | **Sub 1-28** | **m/z=484.20 (C₃₅H₂₉Cl=485.07)** |
| **Sub 1-29** | **m/z=478.15 (C₃₅H₂₃Cl=479.02)** | **Sub 1-30** | **m/z=478.15 (C₃₅H₂₃Cl=479.02)** |
| **Sub 1-31** | **m/z=504.16 (C₃₇H₂₅Cl=505.06)** | **Sub 1-32** | **m/z=433.16 (C₃₁H₁₆D₅Cl=433.99)** |
| **Sub 1-33** | **m/z=433.16 (C₃₁H₁₆D₅Cl=433.99)** | **Sub 1-34** | **m/z=485.19 (C₃₅H₁₆D₇Cl=486.06)** |
| **Sub 1-35** | **m/z=485.19 (C₃₅H₁₆D₇Cl=486.06)** | **Sub 1-36** | **m/z=402.12 (C₂₉H₁₉Cl=402.92)** |
| **Sub 1-37** | **m/z=402.12 (C₂₉H₁₉Cl=402.92)** | **Sub 1-38** | **m/z=478.15 (C₃₅H₂₃Cl=479.02)** |
| **Sub 1-39** | **m/z=478.15 (C₃₅H₂₃Cl=479.02)** | **Sub 1-40** | **m/z=528.16 (C₃₉H₂₅Cl=529.08)** |
| **Sub 1-41** | **m/z=528.16 (C₃₉H₂₅Cl=529.08)** | **Sub 1-42** | **m/z=428.13 (C₃₁H₂₁Cl=428.96)** |

### Synthesis example of Sub 2

Sub 2 of the above reaction scheme 1 can be synthesized by the reaction route of the following reaction scheme 3, but is not limited thereto.

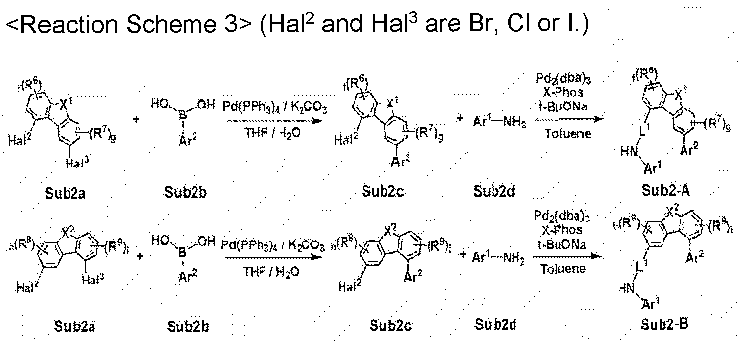

### 1. Synthesis example of Sub2-2

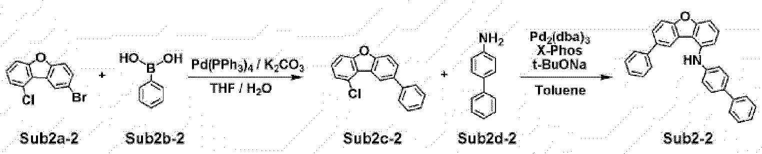

### (1) Synthesis example of Sub2c-2

Sub2a-2 (50 g, 177.60 mmol) and Sub2b-2 (23.82 g, 195.36 mmol) were placed in a round-bottom flask and dissolved in THF (450mL) and water (150mL). Then, Pd(PPh₃)₄ (6.15 g, 5.32 mmol) and K₂CO₃ (49.08 g, 355.20 mmol) were added to the solution and the mixture was stirred at 70 °C. When the reaction was completed, the reaction product was extracted with toluene and water, dried with MgSO₄, and concentrated. Then, the concentrate was separated using a silica column and recrystallized to obtain 42.4 g (yield: 85.6 %) of the product.

### (2) Synthesis example of Sub2-2

Sub2c-2 (20 g, 71.75 mmol) and Sub2d-2(12.14 g, 71.75 mmol) were placed in a round-bottom flask and dissolved in toluene (240 mL). Then, Pd₂(dba)₃ (1.97 g, 2.15mmol), X-Phos (2.05 g, 4.30 mmol) and t-BuONa (13.79 g, 143.50mmol) were added to the solution and the mixture was stirred at 60 °C. When the reaction was completed, the reaction product was extracted with toluene and water, dried with MgSO₄, and concentrated. Then, the concentrate was separated using a silica column and recrystallized to obtain 23.5 g (yield: 79.6%) of the product.

### 2. Synthesis example of Sub2-75

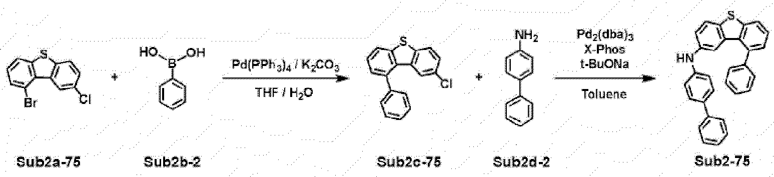

### (1) Synthesis example of Sub2c-75

Sub2a-75 (50 g, 168.01 mmol) and Sub2b-2 (22.53 g, 184.81 mmol) were placed in a round-bottom flask and dissolved in THF (450 mL) and water (150 mL). Then, Pd(PPh₃)₄ (5.82 g, 5.04 mmol) and K₂CO₃ (46.43 g, 336.03 mmol) were added to the solution and the same method as the synthesis of Sub2c-2 was carried out to obtain 34.6 g (yield: 69.8 %) of product.

### (2) Synthesis example of Sub2-75

Sub2c-75 (20 g, 67.84 mmol) and Sub2d-2 (11.48 g, 67.84 mmol) were placed in a round-bottom flask and dissolved in toluene (240 mL), and Pd₂(dba)₃ (1.86 g, 2.03mmol), X-Phos (4.70 g, 4.07 mmol) and t-BuONa (13.04 g, 135.68 mmol) were added to the solution and the same method as the synthesis of Sub2-2 was carried out to obtain 23.4 (yield: 80.7%) of product.

### 3. Synthesis example of Sub2-76

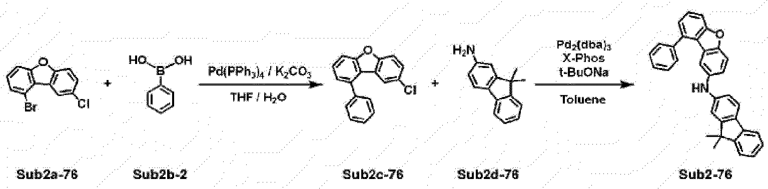

### (1) Synthesis example of Sub2c-76

Sub2a-76 (50 g, 177.60 mmol) and Sub2b-2 (23.82 g, 195.36 mmol) were placed in a round-bottom flask and dissolved in in THF (450mL) and water (150mL). Then, Pd(PPh₃)₄ (6.15 g, 5.32 mmol) and K₂CO₃ (49.08 g, 355.20 mmol) were added to the solution and the same method as the synthesis of Sub2c-2 was carried out to obtain 40.8 g (yield: 82.4 %) of product.

### (2) Synthesis example of Sub2-76

Sub2c-76 (20 g, 71.75 mmol) and Sub2d-76 (12.14 g, 71.75 mmol) were placed in a round-bottom flask and dissolved in toluene (240 mL), and Pd₂(dba)₃ (1.97 g, 2.15mmol), X-Phos (2.05 g, 4.30 mmol) and t-BuONa (13.79 g, 143.50mmol) were added to the solution and the same method as the synthesis of Sub2-2 was carried out to obtain 26.3 g (yield: 81.2%) of product.

### 4. Synthesis example of Sub 2-82

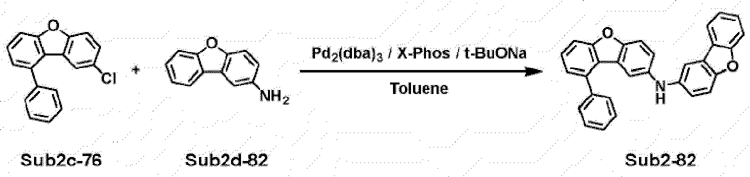

Sub2c-76 (20 g, 71.75 mmol) and Sub2d-82 (13.14 g, 71.75 mmol) were placed in a round-bottom flask and dissolved in toluene (240 mL), and Pd₂(dba)₃ (1.97 g, 2.15mmol), X-Phos (2.05 g, 4.30 mmol) and t-BuONa (13.79 g, 143.50mmol) were added to the solution and the same method as the synthesis of Sub2-2 was carried out to obtain 25.8 g (yield: 84.5%) of product.

Compounds belonging to Sub 2 may be, but not limited to, the following compounds, and Table 2 shows FD-MS values of the following compounds. Here, Sub 2-56 to 63, Sub 2-65 to 68, Sub 2-76 to 80, Sub 2-90 to 91, Sub 2-96, Sub 2-105 to 106 are reference intermediate compounds.

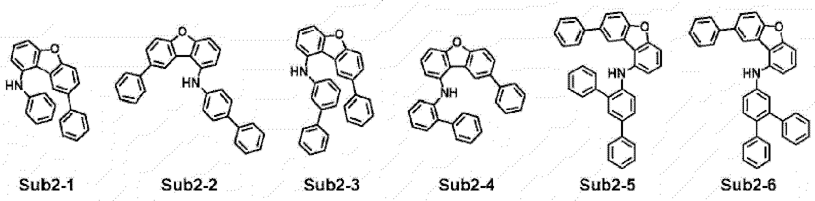

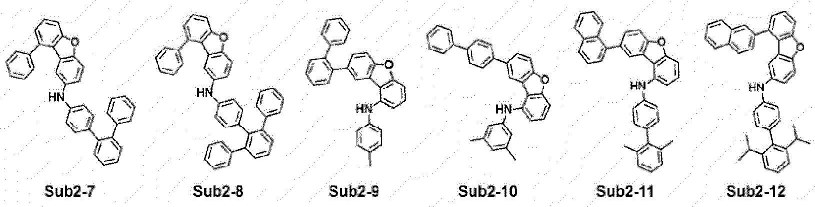

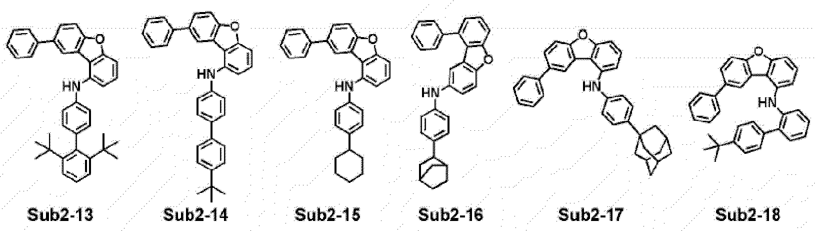

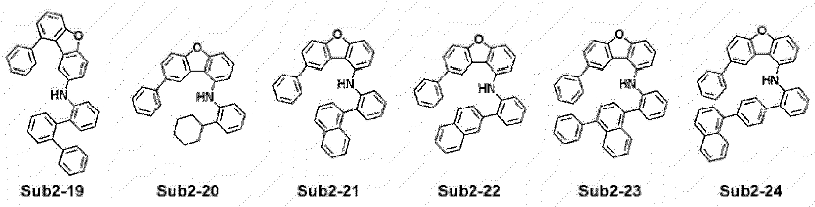

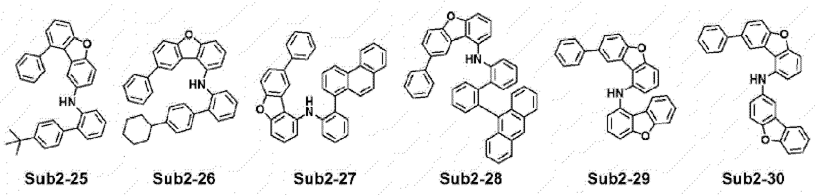

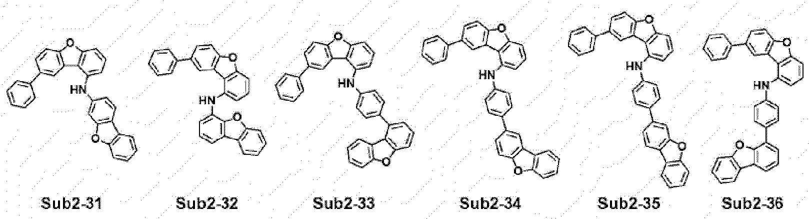

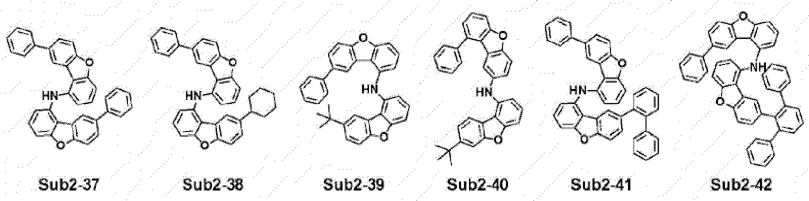

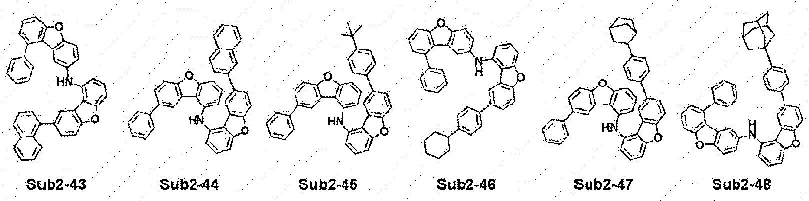

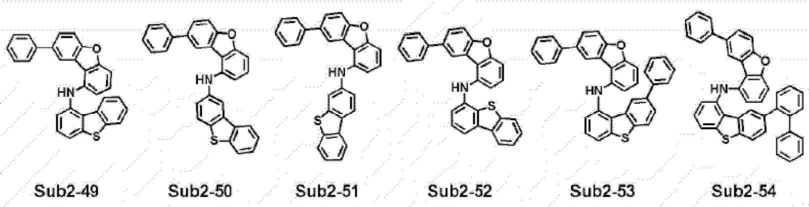

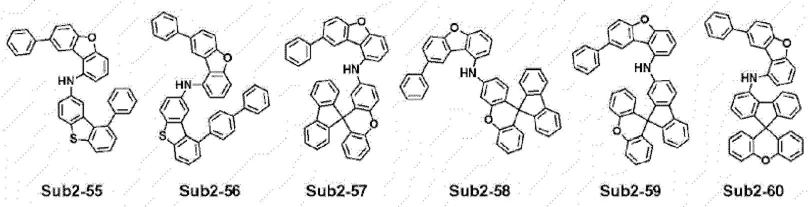

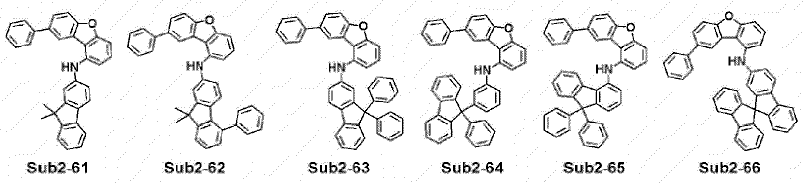

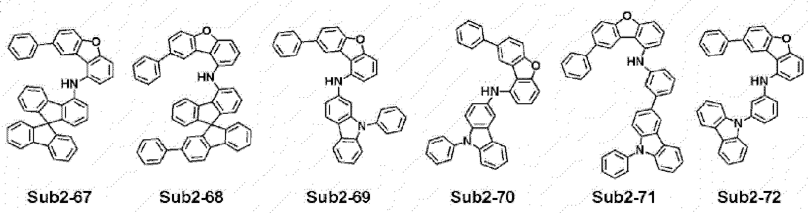

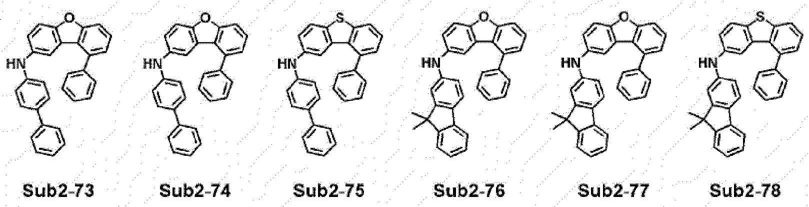

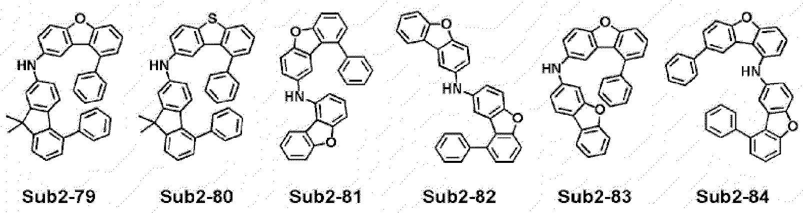

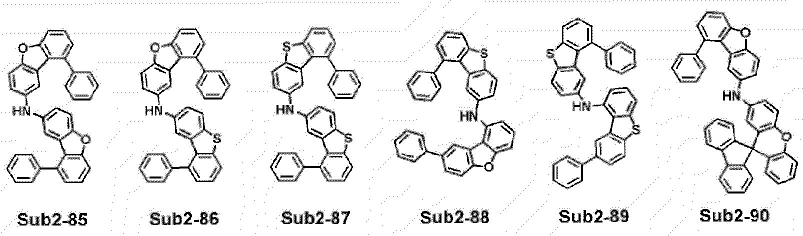

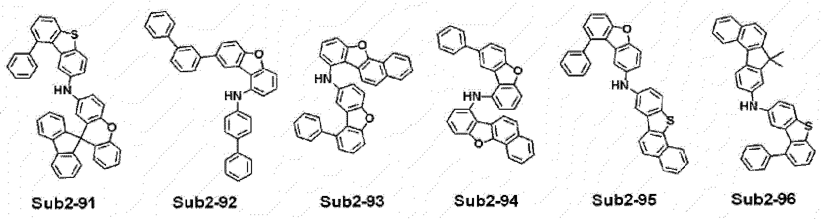

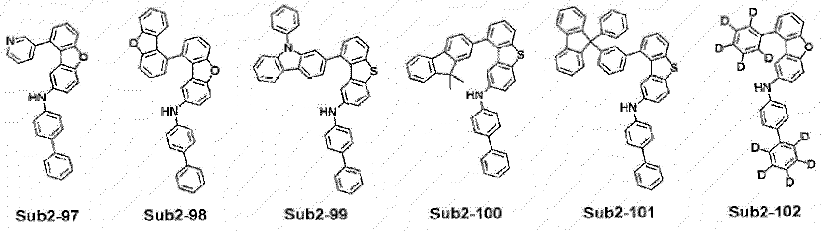

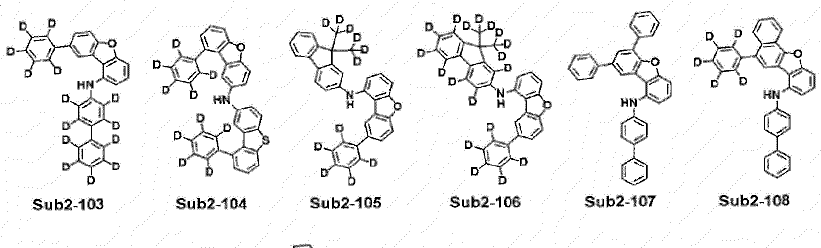

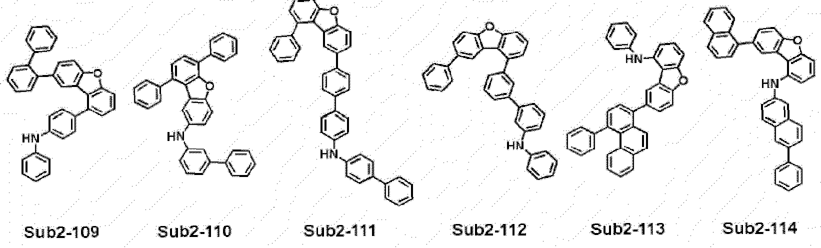

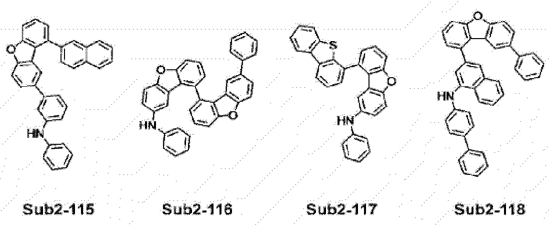

**[Table 2]**

| **Compound** | **FD-MS** | **Compound** | **FD-MS** |
|---|---|---|---|
| **Sub2-1** | **m/z=335.13 (C₂₄H₁₇NO=335.41)** | **Sub2-2** | **m/z=411.16 (C₃₀H₂₁NO=411.50)** |
| **Sub2-3** | **m/z=411.16 (C₃₀H₂₁NO=411.50)** | **Sub2-4** | **m/z=411.16 (C₃₀H₂₁NO=411.50)** |
| **Sub2-5** | **m/z=487.19 (C₃₆H₂₅NO=487.60)** | **Sub2-6** | **m/z=487.19 (C₃₆H₂₅NO=487.60)** |
| **Sub2-7** | **m/z=487.19 (C₃₆H₂₅NO=487.60)** | **Sub2-8** | **m/z=563.22 (C₄₂H₂₉NO=563.70)** |
| **Sub2-9** | **m/z=425.18 (C₃₁H₂₃NO=425.53)** | **Sub2-10** | **m/z=439.19 (C₃₂H₂₅NO=439.56)** |
| **Sub2-11** | **m/z=489.21 (C₃₆H₂₇NO=489.62)** | **Sub2-12** | **m/z=545.27 (C₄₀H₃₅NO=545.73)** |
| **Sub2-13** | **m/z=523.29 (C₃₈H₃₇NO=523.72)** | **Sub2-14** | **m/z=467.22 (C₃₄H₂₉NO=467.61)** |
| **Sub2-15** | **m/z=417.21 (C₃₀H₂₇NO=417.55)** | **Sub2-16** | **m/z=429.21 (C₃₁H₂₇NO=429.56)** |
| **Sub2-17** | **m/z=469.24 (C₃₄H₃₁NO=469.63)** | **Sub2-18** | **m/z=467.22 (C₃₄H₂₉NO=467.61)** |
| **Sub2-19** | **m/z=487.19 (C₃₆H₂₅NO=487.60)** | **Sub2-20** | **m/z=417.21 (C₃₀H₂₇NO=417.55)** |
| **Sub2-21** | **m/z=461.18 (C₃₄H₂₃NO=461.56)** | **Sub2-22** | **m/z=461.18 (C₃₄H₂₃NO=461.56)** |
| **Sub2-23** | **m/z=537.21 (C₄ₒH₂₇NO=537.66)** | **Sub2-24** | **m/z=537.21 (C₄ₒH₂₇NO=537.66)** |
| **Sub2-25** | **m/z=467.22 (C₃₄H₂₉NO=467.61)** | **Sub2-26** | **m/z=493.24 (C₃₆H₃₁NO=493.65)** |
| **Sub2-27** | **m/z=511.19 (C₃₈H₂₅NO=511.62)** | **Sub2-28** | **m/z=587.22 (C₄₄H₂₉NO=587.72)** |
| **Sub2-29** | **m/z=425.14 (C₃₀H₁₉NO₂=425.49)** | **Sub2-30** | **m/z=425.14 (C₃₀H₁₉NO₂=425.49)** |
| **Sub2-31** | **m/z=425.14 (C₃₀H₁₉NO₂=425.49)** | **Sub2-32** | **m/z=425.14 (C₃₀H₁₉NO₂=425.49)** |
| **Sub2-33** | **m/z=501.17 (C₃₆H₂₃NO₂=501.59)** | **Sub2-34** | **m/z=501.17 (C₃₆H₂₃NO₂=501.59)** |
| **Sub2-35** | **m/z=501.17 (C₃₆H₂₃NO₂=501.59)** | **Sub2-36** | **m/z=501.17 (C₃₆H₂₃NO₂=501.59)** |
| **Sub2-37** | **m/z=501.17 (C₃₆H₂₃NO₂=501.59)** | **Sub2-38** | **m/z=507.22 (C₃₆H₂₉NO₂=507.63)** |
| **Sub2-39** | **m/z=481.20 (C₃₄H₂₇NO₂=481.60)** | **Sub2-40** | **m/z=481.20 (C₃₄H₂₇NO₂=481.60)** |
| **Sub2-41** | **m/z=577.20 (C₄₂H₂₇NO₂=577.68)** | **Sub2-42** | **m/z=653.24 (C₄₈H₃₁NO₂=653.78)** |
| **Sub2-43** | **m/z=551.19 (C₄₀H₂₅NO₂=551.65)** | **Sub2-44** | **m/z=551.19 (C₄₀H₂₅NO₂=551.65)** |
| **Sub2-45** | **m/z=557.24 (C₄₀H₃₁NO₂=557.69)** | **Sub2-46** | **m/z=583.25 (C₄₂H₃₃NO₂=583.73)** |
| **Sub2-47** | **m/z=595.25 (C₄₃H₃₃NO₂=595.74)** | **Sub2-48** | **m/z=635.28 (C₄₆H₃₇NO₂=635.81)** |
| **Sub2-49** | **m/z=441.12 (C₃₀H₁₉NOS=441.55)** | **Sub2-50** | **m/z=441.12 (C₃₀H₁₉NOS=441.55)** |
| **Sub2-51** | **m/z=441.12 (C₃₀H₁₉NOS=441.55)** | **Sub2-52** | **m/z=441.12 (C₃₀H₁₉NOS=441.55)** |
| **Sub2-53** | **m/z=517.15 (C₃₆H₂₃NOS=517.65)** | **Sub2-54** | **m/z=593.18 (C₄₂H₂₇NOS=593.74)** |
| **Sub2-55** | **m/z=517.15 (C₃₆H₂₃NOS=517.65)** | **Sub2-56** | **m/z=593.18 (C₄₂H₂₇NOS=593.74)** |
| **Sub2-57** | **m/z=589.20 (C₄₃H₂₇NO₂=589.69)** | **Sub2-58** | **m/z=589.20 (C₄₃H₂₇NO₂=589.69)** |
| **Sub2-59** | **m/z=589.20 (C₄₃H₂₇NO₂=589.69)** | **Sub2-60** | **m/z=589.20 (C₄₃H₂₇NO₂=589.69)** |
| **Sub2-61** | **m/z=451.19 (C₃₃H₂₅NO=451.57)** | **Sub2-62** | **m/z=527.22 (C₃₉H₂₉NO=527.67)** |
| **Sub2-63** | **m/z=575.22 (C₄₃H₂₉NO=575.71)** | **Sub2-64** | **m/z=575.22 (C₄₃H₂₉NO=575.71)** |
| **Sub2-65** | **m/z=575.22 (C₄₃H₂₉NO=575.71)** | **Sub2-66** | **m/z=573.21 (C₄₃H₂₇NO=573.70)** |
| **Sub2-67** | **m/z=573.21 (C₄₃H₂₇NO=573.70)** | **Sub2-68** | **m/z=649.27 (C₄₉H₃₁NO=649.79)** |
| **Sub2-69** | **m/z=500.19 (C₃₆H₂₄N₂O=500.60)** | **Sub2-70** | **m/z=500.19 (C₃₆H₂₄N₂O=500.60)** |
| **Sub2-71** | **m/z=576.22 (C₄₂H₂₈N₂O=576.70)** | **Sub2-72** | **m/z=500.19 (C₃₆H₂₄N₂O=500.60)** |
| **Sub2-73** | **m/z=411.16 (C₃₀H₂₁NO=411.50)** | **Sub2-74** | **m/z=411.16 (C₃₀H₂₁NO=411.50)** |
| **Sub2-75** | **m/z=427.14(C₃₀H₂₁NS=427.57)** | **Sub2-76** | **m/z=451.19 (C₃₃H₂₅NO=451.57)** |
| **Sub2-77** | **m/z=451.19 (C₃₃H₂₅NO=451.57)** | **Sub2-78** | **m/z=467.17 (C₃₃H₂₅NS=467.63)** |
| **Sub2-79** | **m/z=527.22 (C₃₉H₂₉NO=527.67)** | **Sub2-80** | **m/z=543.20 (C₃₉H₂₉NS=543.73)** |
| **Sub2-81** | **m/z=425.14 (C₃₀H₁₉NO₂=425.49)** | **Sub2-82** | **m/z=425.14 (C₃₀H₁₉NO₂=425.49)** |
| **Sub2-83** | **m/z=425.14 (C₃₀H₁₉NO₂=425.49)** | **Sub2-84** | **m/z=501.17 (C₃₆H₂₃NO₂=501.59)** |
| **Sub2-85** | **m/z=501.17 (C₃₆H₂₃NO₂=501.59)** | **Sub2-86** | **m/z=517.15 (C₃₆H₂₃NOS=517.65)** |
| **Sub2-87** | **m/z=533.13 (C₃₆H₂₃NS₂=533.71)** | **Sub2-88** | **m/z=517.15 (C₃₆H₂₃NOS=517.65)** |
| **Sub2-89** | **m/z=533.13 (C₃₆H₂₃NS₂=533.71)** | **Sub2-90** | **m/z=589.20 (C₄₃H₂₇NO₂=589.69)** |
| **Sub2-91** | **m/z=605.18 (C₄₃H₂₇NOS=605.76)** | **Sub2-92** | **m/z=487.19 (C₃₆H₂₅NO=487.60)** |
| **Sub2-93** | **m/z=475.16 (C₃₄H₂₁NO₂=475.55)** | **Sub2-94** | **m/z=475.16 (C₃₄H₂₁NO₂=475.55)** |
| **Sub2-95** | **m/z=491.13 (C₃₄H₂₁NOS=491.61)** | **Sub2-96** | **m/z=517.19 (C₃₇H₂₃NS=517.69)** |
| **Sub2-97** | **m/z=412.16 (C₂₉H₂₀N₂O=412.49)** | **Sub2-98** | **m/z=501.17 (C₃₆H₂₃NO₂=501.59)** |
| **Sub2-99** | **m/z=592.20 (C₄₂H₂₈N₂S=592.76)** | **Sub2-100** | **m/z=543.20 (C₃₉H₂₉NS=543.73)** |
| **Sub2-101** | **m/z=667.23 (C₄₉H₃₃NS=667.87)** | **Sub2-102** | **m/z=421.23 (C₃₀H₁₁D₁₀NO=421.57)** |
| **Sub2-103** | **m/z=425.25 (C₃₀H₇D₁₄NO=425.59)** | **Sub2-104** | **m/z=527.21 (C₃₆H₁₃D₁₀NOS=527.71)** |
| **Sub2-105** | **m/z=462.26 (C₃₃H₁₄D₁₁NO=462.64)** | **Sub2-106** | **m/z=469.31 (C₃₃H₇D₁₈NO=469.68)** |
| **Sub2-107** | **m/z=487.19 (C₃₆H₂₅NO=487.60)** | **Sub2-108** | **m/z=466.21 (C₃₄H₁₈D₅NO=466.59)** |
| **Sub2-109** | **m/z=487.19 (C₃₆H₂₅NO=487.60)** | **Sub2-110** | **m/z=487.19 (C₃₆H₂₅NO=487.60)** |
| **Sub2-111** | **m/z=563.22 (C₄₂H₂₉NO=563.70)** | **Sub2-112** | **m/z=487.19 (C₃₆H₂₅NO=487.60)** |
| **Sub2-113** | **m/z=511.19 (C₃₈H₂₅NO=511.62)** | **Sub2-114** | **m/z=511.19 (C₃₈H₂₅NO=511.62)** |
| **Sub2-115** | **m/z=461.18 (C₃₄H₂₃NO=461.56)** | **Sub2-116** | **m/z=501.17 (C₃₆H₂₃NO₂=501.59)** |
| **Sub2-117** | **m/z=441.12 (C₃₀H₁₉NOS=441.55)** | **Sub2-118** | **m/z=537.21 (C₄ₒH₂₇NO=537.66)** |

### Synthesis example of final product

### 1. Synthesis example of P1-2

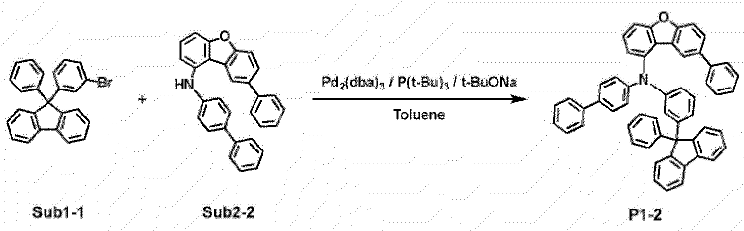

Sub1-1(CAS RN: 1257251-75-4, 15.00 g, 37.75 mmol) and Sub2-2 (15.53 g, 37.75 mmol) were placed in a round-bottom flask and dissolved in toluene (125 mL). Then, Pd₂(dba)₃ (1.03 g, 1.13 mmol), P(t-Bu)₃ (0.45 g, 2.26 mmol) and t-BuONa (7.25 g, 75.50 mmol) were added to the solution and the mixture was stirred at 120 °C. When the reaction was completed, the reaction product was extracted with toluene and water, dried with MgSO₄, and concentrated. Then, the concentrate was then separated using a silica column and recrystallized to obtain 23.7 g (yield: 86.3 %) of the product.

### 2. Synthesis example of P1-75

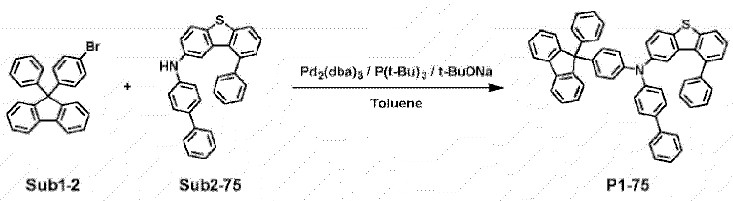

Sub1-2 (15.00 g, 37.75 mmol) and Sub2-75 (16.14 g, 37.75 mmol) were placed in a round-bottom flask and dissolved in toluene (125 mL). Then, Pd₂(dba)₃ (1.03 g, 1.13 mmol), P(t-Bu)₃ (0.45 g, 2.26 mmol) and t-BuONa (7.25 g, 75.50 mmol) were added to the solution and and the same method as the synthesis of P1-2 was carried out to obtain 22.5 g (yield: 80.1 %) of product.

### 3. Synthesis example of P1-76 (This is a reference compound.)

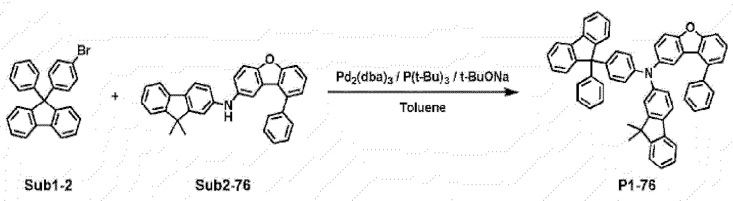

Sub1-2 (15.00 g, 37.75 mmol) and Sub2-76 (17.04 g, 37.75 mmol) were placed in a round-bottom flask and dissolved in toluene (125 mL). Then, Pd₂(dba)₃ (1.03 g, 1.13 mmol), P(t-Bu)₃ (0.45 g, 2.26 mmol) and t-BuONa (7.25 g, 75.50 mmol) were added to the solution and and the same method as the synthesis of P1-2 was carried out to obtain 22.7 g (yield: 78.3 %) of product.

### 4. Synthesis example of P2-50

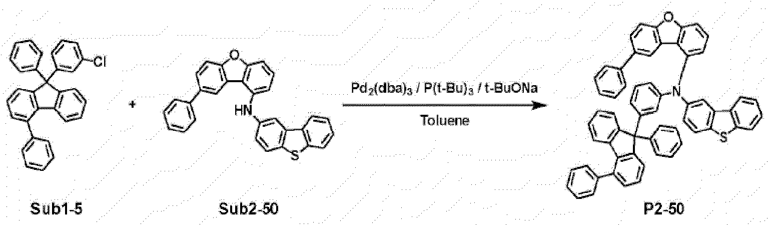

Sub1-5 (15.00 g, 34.96 mmol) and Sub2-50 (15.44 g, 34.96 mmol) were placed in a round-bottom flask and dissolved in toluene (125 mL). Then, Pd₂(dba)₃ (0.96 g, 1.04 mmol), P(t-Bu)₃ (0.42 g, 2.09 mmol) and t-BuONa (6.72 g, 69.93 mmol) were added to the solution and and the same method as the synthesis of P1-2 was carried out to obtain 24.1 g (yield : 82.6 %).

### 5. Synthesis example of P2-91 (This is a reference compound.)

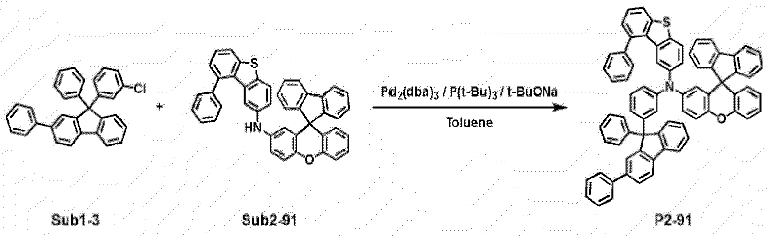

Sub1-3 (15.00 g, 34.96 mmol) and Sub2-91 (21.18 g, 34.96 mmol) were placed in a round-bottom flask and dissolved in toluene (125 mL). Then, Pd₂(dba)₃ (0.96 g, 1.04 mmol), P(t-Bu)₃ (0.42 g, 2.09 mmol) and t-BuONa (6.72 g, 69.93 mmol) were added to the solution and and the same method as the synthesis of P1-2 was carried out to obtain 29.3 g (yield : 83.9 %) of product.

### 6. Synthesis example of P2-105 (This is a reference compound.)

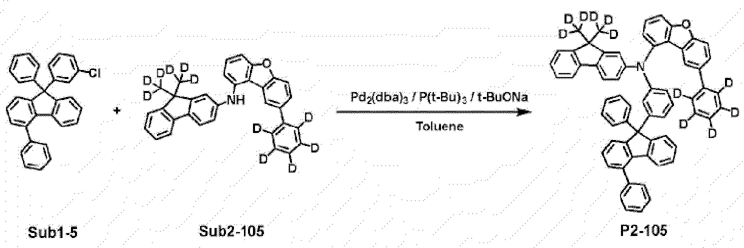

Sub1-5 (15.00 g, 34.96 mmol) and Sub2-105 (16.17 g, 34.96 mmol) were placed in a round-bottom flask and dissolved in toluene (125 mL). Then, Pd₂(dba)₃ (0.96 g, 1.04 mmol), P(t-Bu)₃ (0.42 g, 2.09 mmol) and t-BuONa (6.72 g, 69.93 mmol) were added to the solution and and the same method as the synthesis of P1-2 was carried out to obtain 22.2 g (yield : 74.2 %) of product.

### 7. Synthesis example of P3-6

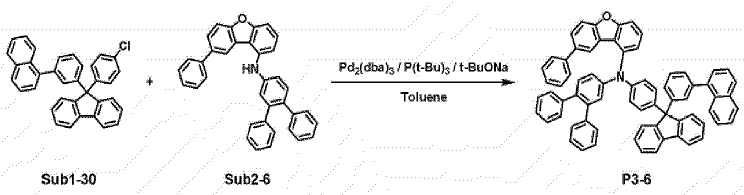

Sub1-30 (15.00 g, 31.31 mmol) and Sub2-6 (15.26 g, 31.31 mmol) were placed in a round-bottom flask and dissolved in toluene (110 mL). Then, Pd₂(dba)₃ (0.86 g, 0.94 mmol), P(t-Bu)₃ (0.38 g, 1.88 mmol) and t-BuONa (6.02 g, 62.70 mmol) were added to the solution and and the same method as the synthesis of P1-2 was carried out to obtain 23.7 g (yield : 81.36 %) of product.

### 8. Synthesis example of P3-16

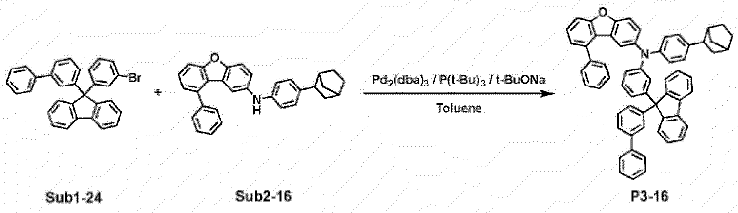

Sub1-24 (15.00 g, 31.68 mmol) and Sub2-16 (13.61 g, 31.68 mmol) were placed in a round-bottom flask and dissolved in toluene (110 mL). Then, Pd₂(dba)₃ (0.87 g, 0.95 mmol), P(t-Bu)₃ (0.38 g, 1.90 mmol) and t-BuONa (6.09 g, 63.37 mmol) were added to the solution and and the same method as the synthesis of P1-2 was carried out to obtain 20.1 g (yield: 77.1 %) of product.

### 9. Synthesis example of P3-100

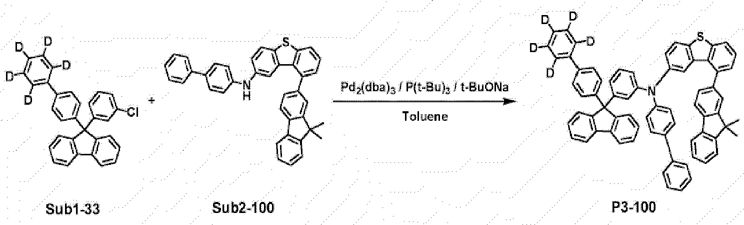

Sub1-33 (15.00 g, 31.35 mmol) and Sub2-100 (17.04 g, 31.35 mmol) were placed in a round-bottom flask and dissolved in toluene (110 mL). Then, Pd₂(dba)₃ (0.86 g, 0.94 mmol), P(t-Bu)₃ (0.38 g, 1.88 mmol) and t-BuONa (6.02 g, 62.70 mmol) were added to the solution and and the same method as the synthesis of P1-2 was carried out to obtain 22.8 g (yield: 77.2 %) of product.

The FD-MS values of the compounds of the present invention manufactured according to the above synthetic examples are as shown in Table 3 below. P1-57 to 64, P1-66 to 68, P1 76-80, P1-90 to 92, P1-96, P1-100, P1-105 to 106, P2-57 to 64, P2-66 to 68, P2 76-80, P2-90 to 92, P2-96, P2-100, P2-105 to 106, P3-57 to 64, P3-66 to 68, P3 76-80, P3-90 to 92, P3-96, P3-100, and P3-105 to 106 are reference compounds.

**[Table 3]**

| **Compound** | **FD-MS** | **Compound** | **FD-MS** |
|---|---|---|---|
| **P1-1** | **m/z=651.26 (C₄₉H₃₃NO=651.81)** | **P1-2** | **m/z=727.29 (C₅₅H₃₇NO=727.91)** |
| **P1-3** | **m/z=727.29 (C₅₅H₃₇NO=727.91)** | **P1-4** | **m/z=727.29 (C₅₅H₃₇NO=727.91)** |
| **P1-5** | **m/z=803.32 (C₆₁H₄₁NO=804.01)** | **P1-6** | **m/z=803.32 (C₆₁H₄₁NO=804.01)** |
| **P1-7** | **m/z=803.32 (C₆₁H₄₁NO=804.01)** | **P1-8** | **m/z=879.35(C₆₇H₄₅NO=880.10)** |
| **P1-9** | **m/z=741.30 (C₅₆H₃₉NO=741.93)** | **P1-10** | **m/z=755.32 (C₅₇H₄₁NO=755.96)** |
| **P1-11** | **m/z=805.33 (C₆₁H₄₃NO=806.02)** | **P1-12** | **m/z=861.40 (C₆₅H₅₁NO=862.13)** |
| **P1-13** | **m/z=839.41 (C₆₃H₅₃NO=840.12)** | **P1-14** | **m/z=783.35 (C₅₉H₄₅NO=784.02)** |
| **P1-15** | **m/z=733.33 (C₅₅H₄₃NO=733.96)** | **P1-16** | **m/z=745.33 (C₅₆H₄₃NO=745.97)** |
| **P1-17** | **m/z=785.37 (C₃₉H₄₇NO=786.03)** | **P1-18** | **m/z=817.30 (C₆₁H₃₉NO₂=817.99)** |
| **P1-19** | **m/z=757.24 (C₅₅H₃₅NOS=757.95)** | **P1-20** | **m/z=853.33 (C₆₅H₄₃NO=854.07)** |
| **P1-21** | **m/z=777.30 (C₅₉H₃₉NO=777.97)** | **P1-22** | **m/z=777.30 (C₅₉H₃₉NO=777.97)** |
| **P1-23** | **m/z=853.33 (C₆₅H₄₃NO=854.07)** | **P1-24** | **m/z=853.33 (C₆₅H₄₃NO=854.07)** |
| **P1-25** | **m/z=783.35 (C₅₉H₄₅NO=784.02)** | **P1-26** | **m/z=809.37 (C₆₁H₄₇NO=810.05)** |
| **P1-27** | **m/z=827.32 (C₆₃H₄₁NO=828.03)** | **P1-28** | **m/z=903.35 (C₆₉H₄₅NO=904.13)** |
| **P1-29** | **m/z=741.27 (C₅₅H₃₅NO₂=741.89)** | **P1-30** | **m/z=741.27 (C₅₅H₃₅NO₂=741.89)** |
| **P1-31** | **m/z=741.27 (C₅₅H₃₅NO₂=741.89)** | **P1-32** | **m/z=741.27 (C₅₅H₃₅NO₂=741.89)** |
| **P1-33** | **m/z=817.30 (C₆₁H₃₉NO₂=817.99)** | **P1-34** | **m/z=817.30 (C₆₁H₃₉NO₂=817.99)** |
| **P1-35** | **m/z=817.30 (C₆₁H₃₉NO₂=817.99)** | **P1-36** | **m/z=817.30 (C₆₁H₃₉NO₂=817.99)** |
| **P1-37** | **m/z=817.30 (C₆₁H₃₉NO₂=817.99)** | **P1-38** | **m/z=791.28 (C₅₉H₃₇NO₂=791.95)** |
| **P1-39** | **m/z=791.28 (C₅₉H₃₇NO₂=791.95)** | **P1-40** | **m/z=797.33 (C₅₉H₄₃NO₂=798.00)** |
| **P1-41** | **m/z=893.33 (C₆₇H₄₃NO₂=894.09)** | **P1-42** | **m/z=969.36 (C₇₃H₄₇NO₂=970.18)** |
| **P1-43** | **m/z=867.31 (C₆₅H₄₁NO₂=868.05)** | **P1-44** | **m/z=867.31 (C₆₇H₄₉NO₂=868.05)** |
| **P1-45** | **m/z=873.36 (C₆₅H₄₇NO₂=874.10)** | **P1-46** | **m/z=899.38 (C₆₇H₄₉NO₂=900.13)** |
| **P1-47** | **m/z=911.38 (C₆₈H₄₉NO₂=912.15)** | **P1-48** | **m/z=951.41 (C₇₁H₅₃NO₂=952.21)** |
| **P1-49** | **m/z=757.24 (C₅₅H₃₅NOS=757.95)** | **P1-50** | **m/z=757.24 (C₅₅H₃₅NOS=757.95)** |
| **P1-51** | **m/z=757.24 (C₅₅H₃₅NOS=757.95)** | **P1-52** | **m/z=757.24 (C₅₅H₃₅NOS=757.95)** |
| **P1-53** | **m/z=833.28 (C₆₁H₃₉NOS=834.05)** | **P1-54** | **m/z=909.31 (C₆₇H₄₃NOS=910.15)** |
| **P1-55** | **m/z=833.28 (C₆₁H₃₉NOS=834.05)** | **P1-56** | **m/z=909.31 (C₆₇H₄₃NOS=910.15)** |
| **P1-57** | **m/z=905.33 (C₆₈H₄₃NO₂=906.10)** | **P1-58** | **m/z=905.33 (C₆₈H₄₃NO₂=906.10)** |
| **P1-59** | **m/z=905.33 (C₆₈H₄₃NO₂=906.10)** | **P1-60** | **m/z=905.33 (C₆₈H₄₃NO₂=906.10)** |
| **P1-61** | **m/z=797.32 (C₅₈H₄₁NO=767.97)** | **P1-62** | **m/z=843.35 (C₆₄H₄₅NO=844.07)** |
| **P1-63** | **m/z=891.35 (C₆₈H₄₅NO=892.11)** | **P1-64** | **m/z=891.35 (C₆₈H₄₅NO=892.11)** |
| **P1-65** | **m/z=891.35 (C₆₈H₄₅NO=892.11)** | **P1-66** | **m/z=889.33 (C₆₈H₄₃NO=890.10)** |
| **P1-67** | **m/z=889.33 (C₆₈H₄₃NO=890.10)** | **P1-68** | **m/z=965.37 (C₇₄H₄₇NO=966.20)** |
| **P1-69** | **m/z=816.31 (C₆₁H₄₀N₂O=817.00)** | **P1-70** | **m/z=816.31 (C₆₁H₄₀N₂O=817.00)** |
| **P1-71** | **m/z=892.35 (C₆₇H₄₄N₂O=893.10)** | **P1-72** | **m/z=816.31 (C₆₁H₄₀N₂O=817.00)** |
| **P1-73** | **m/z=727.29 (C₅₅H₃₇NO=727.91)** | **P1-74** | **m/z=727.29 (C₅₅H₃₇NO=727.91)** |
| **P1-75** | **m/z=743.26 (C₅₅H₃₇NS=743.97)** | **P1-76** | **m/z=767.32 (C₅₈H₄₁NO=767.97)** |
| **P1-77** | **m/z=767.32 (C₅₈H₄₁NO=767.97)** | **P1-78** | **m/z=783.30 (C₅₈H₄₁NS=784.03)** |
| **P1-79** | **m/z=843.35 (C₆₄H₄₅NO=844.07)** | **P1-80** | **m/z=859.33 (C₆₄H₄₅NS=860.13)** |
| **P1-81** | **m/z=741.27 (C₅₅H₃₅NO₂=741.89)** | **P1-82** | **m/z=741.27 (C₅₅H₃₅NO₂=741.89)** |
| **P1-83** | **m/z=741.27 (C₅₅H₃₅NO₂=741.89)** | **P1-84** | **m/z=741.27 (C₅₅H₃₅NO₂=741.89)** |
| **P1-85** | **m/z=817.30 (C₆₁H₃₉NO₂=817.99)** | **P1-86** | **m/z=833.28 (C₆₁H₃₉NOS=834.05)** |
| **P1-87** | **m/z=849.25 (C₆₁H₃₉NS₂=850.11)** | **P1-88** | **m/z=833.28 (C₆₁H₃₉NOS=834.05)** |
| **P1-89** | **m/z=849.25 (C₆₁H₃₉NS₂=850.11)** | **P1-90** | **m/z=905.33 (C₆₈H₄₃NO₂=906.10)** |
| **P1-91** | **m/z=921.31 (C₆₈H₄₃NOS=922.16)** | **P1-92** | **m/z=921.31 (C₆₈H₄₃NOS=922.16)** |
| **P1-93** | **m/z=791.28 (C₅₉H₃₇NO₂=791.95)** | **P1-94** | **m/z=791.28 (C₅₉H₃₇NO₂=791.95)** |
| **P1-95** | **m/z=807.26 (C₅₉H₃₇NOS=808.01)** | **P1-96** | **m/z=817.33 (C₆₂H₄₃NO=818.03)** |
| **P1-97** | **m/z=728.28 (C₅₄H₃₆N₂O=728.90)** | **P1-98** | **m/z=817.30 (C₆₁H₃₉NO₂=817.99)** |
| **P1-99** | **m/z=908.32 (C₆₇H₄₄N₂S=909.16)** | **P1-100** | **m/z=859.33 (C₆₄H₄₅NS=860.13)** |
| **P1-101** | **m/z=983.36 (C₇₄H₄₉NS=984.27)** | **P1-102** | **m/z=732.32 (C₅₅H₃₂D₅NO=732.94)** |
| **P1-103** | **m/z=753.33 (C₅₅H₂₇D₁₀NS=754.03)** | **P1-104** | **m/z=843.34 (C₆₁H₂₉D₁₀NOS=844.11)** |
| **P1-105** | **m/z=778.39 (C₅₈H₃₀D₁₁NO=779.04)** | **P1-106** | **m/z=785.43 (C₅₈H₂₃D₁₈NO=786.08)** |
| **P1-107** | **m/z=803.32 (C₆₁H₄₁NO=804.01)** | **P1-108** | **m/z=782.33 (C₅₉H₃₄D₅NO=783.00)** |
| **P2-1** | **m/z=727.29 (C₅₅H₃₇NO=727.91)** | **P2-2** | **m/z=803.32 (C₆₁H₄₁NO=804.01)** |
| **P2-3** | **m/z=803.32 (C₆₁H₄₁NO=804.01)** | **P2-4** | **m/z=803.32 (C₆₁H₄₁NO=804.01)** |
| **P2-5** | **m/z=955.38 (C₇₃H₄₉NO=956.20)** | **P2-6** | **m/z=955.38 (C₇₃H₄₉NO=956.20)** |
| **P2-7** | **m/z=955.38 (C₇₃H₄₉NO=956.20)** | **P2-8** | **m/z=955.38 (C₇₃H₄₉NO=956.20)** |
| **P2-9** | **m/z=859.38 (C₆₅H₄₉NO=860.11)** | **P2-10** | **m/z=985.43 (C₇₅H₅₅NO=986.27)** |
| **P2-11** | **m/z=979.38 (C₇₅H₄₉NO=980.22)** | **P2-12** | **m/z=1069.39 (C₈₁H₅₁NO₂=1070.30)** |
| **P2-13** | **m/z=1005.45 (C₇₅H₅₉NO₂=1006.30)** | **P2-14** | **m/z=949.39 (C₇₁H₅₁NO₂=950.19)** |
| **P2-15** | **m/z=975.41 (C₇₃H₅₃NO₂=976.23)** | **P2-16** | **m/z=897.40 (C₆₈H₅₁NO=898.16)** |
| **P2-17** | **m/z=911.41 (C₆₉H₅₃NO=912.19)** | **P2-18** | **m/z=859.38 (C₆₅H₄₉NO=860.11)** |
| **P2-19** | **m/z=929.37 (C₇₁H₄₇NO=930.16)** | **P2-20** | **m/z=809.37 (C₆₁H₄₇NO=810.05)** |
| **P2-21** | **m/z=853.33 (C₆₅H₄₃NO=854.07)** | **P2-22** | **m/z=853.33 (C₆₅H₄₃NO=854.07)** |
| **P2-23** | **m/z=929.37 (C₇₁H₄₁NO=930.16)** | **P2-24** | **m/z=929.37 (C₇₁H₄₁NO=930.16)** |
| **P2-25** | **m/z=859.38 (C₆₅H₄₉NO=860.11)** | **P2-26** | **m/z=885.40 (C₆₇H₅₁NO=886.15)** |
| **P2-27** | **m/z=953.37 (C₇₃H₄₇NO=954.19)** | **P2-28** | **m/z=1029.40 (C₇₉H₅₁NO=1030.28)** |
| **P2-29** | **m/z=817.30 (C₆₁H₃₉NO₂=817.99)** | **P2-30** | **m/z=817.30 (C₆₁H₃₉NO₂=817.99)** |
| **P2-31** | **m/z=817.30 (C₆₁H₃₉NO₂=817.99)** | **P2-32** | **m/z=817.30 (C₆₁H₃₉NO₂=817.99)** |
| **P2-33** | **m/z=969.36 (C₇₃H₄₇NO₂=970.18)** | **P2-34** | **m/z=969.36 (C₇₃H₄₇NO₂=970.18)** |
| **P2-35** | **m/z=969.36 (C₇₃H₄₇NO₂=970.18)** | **P2-36** | **m/z=949.39 (C₇₁H₅₁NO₂=950.19)** |
| **P2-37** | **m/z=893.33 (C₆₇H₄₃NO₂=894.09)** | **P2-38** | **m/z=979.38 (C₇₅H₄₉NO=980.22)** |
| **P2-39** | **m/z=867.31 (C₆₅H₄₁NO₂=868.05)** | **P2-40** | **m/z=867.31 (C₆₅H₄₁NO₂=868.05)** |
| **P2-41** | **m/z=1059.37 (C₇₉H₄₉NO₃=1060.27)** | **P2-42** | **m/z=1211.43 (C₉₁H₅₇NO₃=1212.46)** |
| **P2-43** | **m/z=1019.38 (C₇₇H₄₉NO₂=1020.24)** | **P2-44** | **m/z=943.35 (C₇₁H₄₅NO₂=944.15)** |
| **P2-45** | **m/z=949.39 (C₇₁H_{5I}NO₂=950.19)** | **P2-46** | **m/z=975.41 (C₇₃H₅₃NO₂=976.23)** |
| **P2-47** | **m/z=987.41 (C₇₄H₅₃NO₂=988.24)** | **P2-48** | **m/z=1027.44 (C₇₇H₅₇NO₂=1028.31)** |
| **P2-49** | **m/z=833.28 (C₆₁H₃₉NOS=834.05)** | **P2-50** | **m/z=833.28 (C₆₁H₃₉NOS=834.05)** |
| **P2-51** | **m/z=833.28 (C₆₁H₃₉NOS=834.05)** | **P2-52** | **m/z=833.28 (C₆₁H₃₉NOS=834.05)** |
| **P2-53** | **m/z=959.32 (C₇₁H₄₅NOS=960.21)** | **P2-54** | **m/z=1035.35 (C₇₇H₄₉NOS=1036.31)** |
| **P2-55** | **m/z=909.31 (C₆₇H₄₃NOS=910.15)** | **P2-56** | **m/z=985.34 (C₇₃H₄₇NOS=986.25)** |
| **P2-57** | **m/z=981.36 (C₇₄H₄₇NO₂=982.20)** | **P2-58** | **m/z=981.36 (C₇₄H₄₇NO₂=982.20)** |
| **P2-59** | **m/z=981.36 (C₇₄H₄₇NO₂=982.20)** | **P2-60** | **m/z=981.36 (C₇₄H₄₇NO₂=982.20)** |
| **P2-61** | **m/z=843.35 (C₆₄H₄₅NO=844.07)** | **P2-62** | **m/z=919.38 (C₇₀H₄₉NO=920.17)** |
| **P2-63** | **m/z=967.38 (C₇₄H₄₉NO=968.21)** | **P2-64** | **m/z=967.38 (C₇₄H₄₉NO=968.21)** |
| **P2-65** | **m/z=967.38 (C₇₄H₄₉NO=968.21)** | **P2-66** | **m/z=965.37 (C₇₄H₄₇NO=966.20)** |
| **P2-67** | **m/z=965.37 (C₇₄H₄₇NO=966.20)** | **P2-68** | **m/z=1041.40 (C₈₀H₅₁NO=1042.29)** |
| **P2-69** | **m/z=892.35 (C₆₇H₄₄N₂O=893.10)** | **P2-70** | **m/z=968.38 (C₇₃H₄₈N₂O=969.20)** |
| **P2-71** | **m/z=1044.41 (C₇₉H₅₂N₂O=1045.30)** | **P2-72** | **m/z=948.41 (C₇₁H₅₂N₂O=949.21)** |
| **P2-73** | **m/z=803.32 (C₆₁H₄₁NO=804.01)** | **P2-74** | **m/z=803.32 (C₆₁H₄₁NO=804.01)** |
| **P2-75** | **m/z=819.30 (C₆₁H₄₁NS=820.07)** | **P2-76** | **m/z=843.35 (C₆₄H₄₅NO=844.07)** |
| **P2-77** | **m/z=843.35 (C₆₄H₄₅NO=844.07)** | **P2-78** | **m/z=859.33 (C₆₄H₄₅NS=860.13)** |
| **P2-79** | **m/z=969.40 (C₇₄H₅₁NO=970.23)** | **P2-80** | **m/z=935.36 (C₇₀H₄₉NS=936.23)** |
| **P2-81** | **m/z=817.30 (C₆₁H₃₉NO₂=817.99)** | **P2-82** | **m/z=817.30 (C₆₁H₃₉NO₂=817.99)** |
| **P2-83** | **m/z=817.30 (C₆₁H₃₉NO₂=817.99)** | **P2-84** | **m/z=893.33 (C₆₇H₄₃NO₂=894.09)** |
| **P2-85** | **m/z=893.33 (C₆₇H₄₃NO₂=894.09)** | **P2-86** | **m/z=909.31 (C₆₇H₄₃NOS=910.15)** |
| **P2-87** | **m/z=925.28 (C₆₇H₄₃NS₂=926.21)** | **P2-88** | **m/z=909.31 (C₆₇H₄₃NOS=910.15)** |
| **P2-89** | **m/z=925.28 (C₆₇H₄₃NS₂=926.21)** | **P2-90** | **m/z=981.36 (C₇₄H₄₇NO₂=982.20)** |
| **P2-91** | **m/z=997.34 (C₇₄H₄₇NOS=998.26)** | **P2-92** | **m/z=997.34 (C₇₄H₄₇NOS=998.26)** |
| **P2-93** | **m/z=867.31 (C₆₅H₄₁NO₂=868.05)** | **P2-94** | **m/z=917.33 (C₆₉H₄₃NO₂=918.11)** |
| **P2-95** | **m/z=883.29 (C₆₅H₄₁NOS=884.11)** | **P2-96** | **m/z=909.34 (C₆₈H₄₇NS=910.19)** |
| **P2-97** | **m/z=804.31 (C₆₀H₄₀N₂O=804.99)** | **P2-98** | **m/z=893.33 (C₆₇H₄₃NO₂=894.09)** |
| **P2-99** | **m/z=984.35 (C₇₃H₄₈N₂S=985.26)** | **P2-100** | **m/z=935.36 (C₇₀H₄₉NS=936.23)** |
| **P2-101** | **m/z=1064.42 (C₈₀H₄₈D₅NS=1065.40)** | **P2-102** | **m/z=813.38 (C₆₁H₃₁D₁₀NO=814.07)** |
| **P2-103** | **m/z=822.44 (C₆₁H₂₂D₁₉NO=823.12** | **P2-104** | **m/z=924.40 (C₆₇H₂₈D₁₅NOS=925.24)** |
| **P2-105** | **m/z=854.42 (C₆₄H₃₄D₁₁NO=855.14)** | **P2-106** | **m/z=866.49 (C₆₄H₂₂D₂₃NO=867.21)** |
| **P2-107** | **m/z=879.35 (C₆₇H₄₅NO=880.10)** | **P2-108** | **m/z=858.37 (C₆₅H₃₈D₅NO=859.10)** |
| **P3-1** | **m/z=727.29 (C₅₅H₃₇NO=727.91)** | **P3-2** | **m/z=803.32 (C₆₁H₄₁NO=804.01)** |
| **P3-3** | **m/z=803.32 (C₆₁H₄₁NO=804.01)** | **P3-4** | **m/z=803.32 (C₆₁H₄₁NO=804.01)** |
| **P3-5** | **m/z=879.35 (C₆₇H₄₅NO=880.10)** | **P3-6** | **m/z=929.37 (C₇₁H₄₇NO=930.16)** |
| **P3-7** | **m/z=929.37 (C₇₁H₄₇NO=930.16)** | **P3-8** | **m/z=955.38 (C₇₃H₄₉NO=956.20)** |
| **P3-9** | **m/z=1005.40 (C₇₇H₅₁NO=1006.26)** | **P3-10** | **m/z=959.41 (C₇₃H₅₃NO=960.23)** |
| **P3-11** | **m/z=1035.35 (C₇₇H₄₉NOS=1036.31)** | **P3-12** | **m/z=929.37 (C₇₁H₄₇NO=930.16)** |
| **P3-13** | **m/z=915.44 (C₆₉H₅₇NO=916.22)** | **P3-14** | **m/z=859.38 (C₆₅H₄₉NO=860.11)** |
| **P3-15** | **m/z=809.37 (C₆₁H₄₇NO=810.05)** | **P3-16** | **m/z=821.37 (C₆₂H₄₇NO=822.06)** |
| **P3-17** | **m/z=861.40 (C₆₅H₅₁NO=862.13)** | **P3-18** | **m/z=909.40 (C₆₉H₅₁NO=910.17)** |
| **P3-19** | **m/z=929.37 (C₇₁H₄₇NO=930.16)** | **P3-20** | **m/z=859.38 (C₆₅H₄₉NO=860.11)** |
| **P3-21** | **m/z=853.33 (C₆₅H₄₃NO=854.07)** | **P3-22** | **m/z=853.33 (C₆₅H₄₃NO=854.07)** |
| **P3-23** | **m/z=929.37 (C₇₁H₄₇NO=930.16)** | **P3-24** | **m/z=929.37 (C₇₁H₄₇NO=930.16)** |
| **P3-25** | **m/z=859.38 (C₆₅H₄₉NO=860.11)** | **P3-26** | **m/z=885.40 (C₆₇H₅₁NO=886.15)** |
| **P3-27** | **m/z=903.35 (C₆₉H₄₅NO=904.13)** | **P3-28** | **m/z=979.38 (C₇₅H₄₉NO=980.22)** |
| **P3-29** | **m/z=817.30 (C₆₁H₃₉NO₂=817.99)** | **P3-30** | **m/z=817.30 (C₆₁H₃₉NO₂=817.99)** |
| **P3-31** | **m/z=817.30 (C₆₁H₃₉NO₂=817.99)** | **P3-32** | **m/z=817.30 (C₆₁H₃₉NO₂=817.99)** |
| **P3-33** | **m/z=893.33 (C₆₇H₄₃NO₂=894.09)** | **P3-34** | **m/z=893.33 (C₆₇H₄₃NO₂=894.09)** |
| **P3-35** | **m/z=893.33 (C₆₇H₄₃NO₂=894.09)** | **P3-36** | **m/z=893.33 (C₆₇H₄₃NO₂=894.09)** |
| **P3-37** | **m/z=893.33 (C₆₇H₄₃NO₂=894.09)** | **P3-38** | **m/z=899.38 (C₆₇H₄₉NO₂=900.13)** |
| **P3-39** | **m/z=917.33 (C₆₉H₄₃NO₂=918.11)** | **P3-40** | **m/z=973.39 (C₇₃H₆₁NO₂=974.22)** |
| **P3-41** | **m/z=1045.39 (C₇₉H₅₁NO₂=1046.28)** | **P3-42** | **m/z=1045.39 (C₇₉H₅₁NO₂=1046.28)** |
| **P3-43** | **m/z=943.35 (C₇₁H₄₅NO₂=944.15)** | **P3-44** | **m/z=993.36 (C₇₅H₄₇NO₂=994.21)** |
| **P3-45** | **m/z=949.39 (C₇₁H₅₁NO₂=950.19)** | **P3-46** | **m/z=975.41 (C₇₃H₅₃NO₂=976.23)** |
| **P3-47** | **m/z=987.41 (C₇₄H₅₃NO₂=988.24)** | **P3-48** | **m/z=1027.44 (C₇₇H₅₇NO₂=1028.31)** |
| **P3-49** | **m/z=833.28 (C₆₁H₃₉NOS=834.05)** | **P3-50** | **m/z=833.28 (C₆₁H₃₉NOS=834.05)** |
| **P3-51** | **m/z=833.28 (C₆₁H₃₉NOS=834.05)** | **P3-52** | **m/z=833.28 (C₆₁H₃₉NOS=834.05)** |
| **P3-53** | **m/z=909.31 (C₆₇H₄₃NOS=910.15)** | **P3-54** | **m/z=985.34 (C₇₃H₄₇NOS=986.25)** |
| **P3-55** | **m/z=909.31 (C₆₇H₄₃NOS=910.15)** | **P3-56** | **m/z=985.34 (C₇₃H₄₇NOS=986.25)** |
| **P3-57** | **m/z=981.36 (C₇₄H₄₇NO₂=982.20)** | **P3-58** | **m/z=981.36 (C₇₄H₄₇NO₂=982.20)** |
| **P3-59** | **m/z=981.36 (C₇₄H₄₇NO₂=982.20)** | **P3-60** | **m/z=981.36 (C₇₄H₄₇NO₂=982.20)** |
| **P3-61** | **m/z=843.35 (C₆₄H₄₅NO=844.07)** | **P3-62** | **m/z=919.38 (C₇₀H₄₉NO=920.17)** |
| **P3-63** | **m/z=967.38 (C₇₄H₄₉NO=968.21)** | **P3-64** | **m/z=967.38 (C₇₄H₄₉NO=968.21)** |
| **P3-65** | **m/z=967.38 (C₇₄H₄₉NO=968.21)** | **P3-66** | **m/z=965.37 (C₇₄H₄₇NO=966.20)** |
| **P3-67** | **m/z=965.37 (C₇₄H₄₇NO=966.20)** | **P3-68** | **m/z=1041.40 (C₈₀H₅₁NO=1042.29)** |
| **P3-69** | **m/z=892.35 (C₆₇H₄₄N₂O=893.10)** | **P3-70** | **m/z=892.35 (C₆₇H₄₄N₂O=893.10)** |
| **P3-71** | **m/z=968.38 (C₇₃H₄₈N₂O=969.20)** | **P3-72** | **m/z=892.35 (C₆₇H₄₄N₂O=893.10)** |
| **P3-73** | **m/z=803.32 (C₆₁H₄₁NO=804.01)** | **P3-74** | **m/z=803.32 (C₆₁H₄₁NO=804.01)** |
| **P3-75** | **m/z=819.30 (C₆₁H₄₁NS=820.07)** | **P3-76** | **m/z=843.35 (C₆₄H₄₅NO=844.07)** |
| **P3-77** | **m/z=843.35 (C₆₄H₄₅NO=844.07)** | **P3-78** | **m/z=859.33 (C₆₄H₄₅NS=860.13)** |
| **P3-79** | **m/z=919.38 (C₇₀H₄₉NO=920.17)** | **P3-80** | **m/z=935.36 (C₇₀H₄₉NS=936.23)** |
| **P3-81** | **m/z=817.30 (C₆₁H₃₉NO₂=817.99)** | **P3-82** | **m/z=817.30 (C₆₁H₃₉NO₂=817.99)** |
| **P3-83** | **m/z=817.30 (C₆₁H₃₉NO₂=817.99)** | **P3-84** | **m/z=893.33 (C₆₇H₄₃NO₂=894.09)** |
| **P3-85** | **m/z=893.33 (C₆₇H₄₃NO₂=894.09)** | **P3-86** | **m/z=909.31 (C₆₇H₄₃NOS=910.15)** |
| **P3-87** | **m/z=925.28 (C₆₇H₄₃NS₂=926.21)** | **P3-88** | **m/z=909.31 (C₆₇H₄₃NOS=910.15)** |
| **P3-89** | **m/z=925.28 (C₆₇H₄₃NS₂=926.21)** | **P3-90** | **m/z=981.36 (C₇₄H₄₇NO₂=982.20)** |
| **P3-91** | **m/z=997.34 (C₇₄H₄₇NOS=998.26)** | **P3-92** | **m/z=997.34 (C₇₄H₄₇NOS=998.26)** |
| **P3-93** | **m/z=867.31 (C₆₅H₄₁NO₂=868.05)** | **P3-94** | **m/z=867.31 (C₆₅H₄₁NO₂=868.05)** |
| **P3-95** | **m/z=883.29 (C₆₅H₄₁NOS=884.11)** | **P3-96** | **m/z=893.33 (C₆₇H₄₃NO₂=894.09)** |
| **P3-97** | **m/z=804.31 (C₆₀H₄₀N₂O=804.99)** | **P3-98** | **m/z=893.33 (C₆₇H₄₃NO₂=894.09)** |
| **P3-99** | **m/z=984.35 (C₇₃H₄₈N₂S=985.26)** | **P3-100** | **m/z=940.39 (C₇₀H₄₄D₅NS=941.26)** |
| **P3-101** | **m/z=1064.42 (C₈₀H₄₈D₅NS=1065.40)** | **P3-102** | **m/z=813.38 (C₆₁H₃₁D₁₀NO=814.07)** |
| **P3-103** | **m/z=834.39 (C₆₁H₂₆D₁₅NS=835.16)** | **P3-104** | **m/z=924.40 (C₆₇H₂₈D₁₅NOS=925.24)** |
| **P3-105** | **m/z=859.45 (C₆₄H₂₉D₁₆NO=860.17)** | **P3-106** | **m/z=866.49 (C₆₄H₂₂D₂₃NO=867.21)** |
| **P3-107** | **m/z=879.35 (C₆₇H₄₅NO=880.10)** | **P3-108** | **m/z=858.37 (C₆₅H₃₈D₅NO=859.10)** |

### Fabrication and Evaluation of Organic Electric Element

### [Test Example 1] Green organic electroluminescent element (light-emitting auxiliary layer)

After vacuum-depositing 4,4',4"-tris[2-naphthyl(phenyl)amino]triphenylamine (hereinafter abbreviated as "2-TNATA") on an ITO layer (anode) formed on a glass substrate to form a hole injection layer of 60 nm thickness, a hole transport layer of 60 nm thickness was formed by vacuum-depositing N,N'-bis(1-naphthalenyl)-N,N'-bisphenyl-(1,1'-biphenyl)-4,4'-diamine (hereinafter abbreviated as "NPB") on the hole injection layer.

Subsequently, a light-emitting auxiliary layer of 20 nm thickness was formed by vacuum-depositing the compound P1-2 of the present invention on the hole transport layer and 4,4'-N,N'-dicarbazole-biphenyl (hereinafter abbreviated as "CBP") as a host material and tris(2-phenylpyridine)-iridium (hereinafter abbreviated as "Ir(ppy)₃)") as a dopant material in a weight ratio of 95:5 were deposited on the light-emitting auxiliary layer to form a light-emitting layer of 30 nm thickness.

Next, (1,1'-bisphenyl-4-olato)bis(2-methyl-8-quinolinolato)aluminum (hereinafter abbreviated as " BAlq") was vacuum-deposited to form a hole blocking layer of 10 nm thickness on the light-emitting layer, and tris-(8-hydroxyquinoline)aluminum (hereinafter abbreviated as "Alq₃") was vacuum-deposited to form a an electron transport layer of 40 nm thickness on the hole blocking layer. Thereafter, LiF was deposited to form an electron injection layer of 0.2 nm thickness on the electron transport layer, and then Al was deposited to form a cathode of 150 nm thickness on the electron injection layer.

### [Test Example 2] to [Test Example 15]

The organic electroluminescent elements were fabricated in the same manner as described in Example 1 except that the compounds of the present invention described in the following Table 4, instead of compound P1-2, were used as material of a light-emitting auxiliary layer.

### [Comparative Example 1] to [Comparative Example 3]

The organic electroluminescent elements were fabricated in the same manner as described in Example 1 except that Comparative Compounds 1 to 3, instead of compound P1-2, were used as material of a light-emitting auxiliary layer.

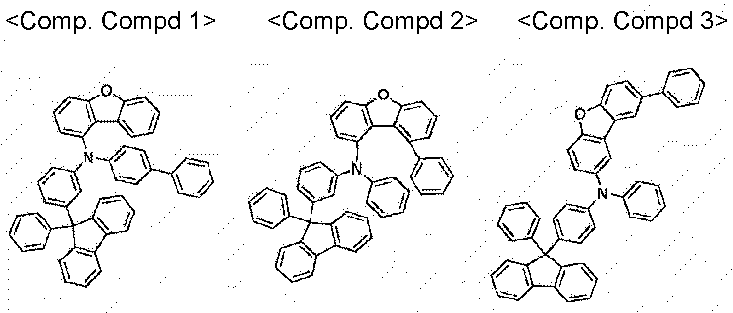

A forward bias DC voltage was applied to the organic electroluminescent elements manufactured in Test Examples 1 to 15 and Comparative Examples 1 to 3, and electroluminescence (EL) characteristics were measured with Photo Research's PR-650 and lifetime(T95) was measured with a lifetime measuring device manufactured by Mc Science company at 5000 cd/m² standard luminance. The measurement results are shown in Table 4 below.

**[Table 4]**

| | **Compound** | **Voltage (V)** | **Current Density (mA/cm²)** | **Brightness (cd/m²)** | **Efficiency (cd/A)** | **Lifetime T(95)** |
|---|---|---|---|---|---|---|
| **comp.Ex (1)** | **Comp. compd 1** | **6.3** | **23.1** | **5000.0** | **21.6** | **82.5** |
| **comp.Ex (2)** | **Comp. compd 2** | **5.9** | **16.6** | **5000.0** | **30.1** | **92.6** |
| **comp.Ex (3)** | **Comp. compd 3** | **6.0** | **17.7** | **5000.0** | **28.3** | **100.7** |
| **Test Ex.(1)** | **P1-2** | **4.9** | **11.7** | **5000.0** | **42.7** | **123.0** |
| **Test Ex.(2)** | **P1-6** | **4.9** | **11.5** | **5000.0** | **43.4** | **123.2** |
| **Test Ex.(3)** | **P1-12** | **4.8** | **11.4** | **5000.0** | **43.9** | **122.9** |
| **Test Ex.(4)** | **P1-16** | **4.9** | **12.0** | **5000.0** | **41.8** | **123.4** |
| **Test Ex.(5)** | **P1-30** | **5.0** | **11.9** | **5000.0** | **42.1** | **125.0** |
| **Test Ex.(6)** | **P1-33** | **4.9** | **11.5** | **5000.0** | **43.4** | **124.8** |
| **Test Ex.(7)** | **P1-40** | **4.8** | **11.7** | **5000.0** | **42.8** | **122.9** |
| **Test Ex.(8)** | **P1-53** | **4.9** | **11.5** | **5000.0** | **43.4** | **124.3** |
| **Test Ex.(9)** | **P1-57** | **5.0** | **11.6** | **5000.0** | **42.3** | **123.6** |
| **Test Ex.(10)** | **P1-73** | **4.9** | **12.5** | **5000.0** | **40.1** | **121.2** |
| **Test Ex.(11)** | **P1-75** | **5.1** | **12.2** | **5000.0** | **40.9** | **121.9** |
| **Test Ex.(12)** | **P1-86** | **5.0** | **12.1** | **5000.0** | **41.3** | **122.3** |
| **Test Ex.(13)** | **P1-87** | **5.1** | **12.3** | **5000.0** | **40.5** | **121.7** |
| **Test Ex.(14)** | **P1-93** | **4.8** | **11.6** | **5000.0** | **43.2** | **124.1** |
| **Test Ex.(15)** | **P1-105** | **4.9** | **11.6** | **5000.0** | **43.2** | **125.7** |

| | | | | | | |
|---|---|---|---|---|---|---|
| Test Ex. (9) and (15) are reference examples. | | | | | | |

From the above Table 4, it can be seen that not only can the driving voltage of an organic electroluminescent element be lowered, but also the efficiency and lifespan are significantly improved when an organic electroluminescent element is manufactured using the material for an organic electroluminescent element of the present invention as a light-emitting auxiliary layer material, compared to when Comparative Compound 1 to Comparative Compound 3 are used.

Comparative Compound 1 to Comparative Compound 3 and the compound of the present invention have the same structure in which an amine group is bonded to the phenyl of 9,9-diphenyl-9H-fluorene, but there is a difference in the substituent bonded to the benzene moiety of dibenzofuran which is substituted on the amine group.

Comparative Compound 1 is similar to the compound in which A in Formula 1 of the present invention is Formula A-1, but the compound of the present invention is different in that Ar², not hydrogen, is necessarily substituted at a specific position of the benzene moiety of dibenzofuran in Formula A-1, whereas Comparative Compounds 1 and 2 have a difference in that the substituent corresponding to Ar² is hydrogen.

In addition, Comparative Compound 3 is similar to the compound in which A in Formula 1 of the present invention is Formula A-2, but the compound of the present invention is different in that Ar³, not hydrogen, is necessarily substituted at a specific position of the benzene moiety of dibenzofuran in Formula A-2, whereas Comparative Compound 3 has a difference in that the substituent corresponding to Ar³ is hydrogen.

The compound of the present invention has an amine group and Ar² or Ar³ substituted at an asymmetric position with respect to dibenzofuran. That is, an amine group is substituted at position 1 of dibenzofuran and Ar² is substituted at position 8, or an amine group is substituted at position 2 and Ar³ is substituted at position 9.

On the other hand, it can be seen that Comparative Compounds 2 and 3 have amine groups and phenyl groups substituted at positions symmetrical with respect to dibenzofuran. That is, Comparative Compound 2 has an amine group substituted at position 1 of dibenzofuran and a phenyl group substituted at position 9, and Comparative Compound 3 has an amine group substituted at position 2 of dibenzofuran and a phenyl group substituted at position 8.

Due to these differences, when the compound of the present invention is used as a light-emitting auxiliary layer material, the driving voltage, efficiency, and lifespan are all significantly improved compared to the comparative compounds.

This can be explained by the LUMO values of Comparative Compound 2, Comparative Compound 3, and the compounds of the present invention. Referring to Table 5 below, it can be seen that the LUMO values of the compounds of the present invention, in which the amine group and phenyl are asymmetrically substituted, is higher than that of Comparative Compounds 2 and 3, in which the amine group and phenyl are symmetrically substituted with respect to dibenzofuran.

**[Table 5]**

| | **Comp. compd 2** | **Comp. compd 3** | **P1-1** | **P1-73** |
|---|---|---|---|---|
| **LUMO (eV)** | **-1.089** | **-1.066** | **-1.028** | **-1.004** |

Therefore, since the compound of the present invention has a higher LUMO value, it has an improved blocking ability for electrons passing from the light-emitting layer to the light-emitting auxiliary layer than the comparative compound 2 or comparative compound 3, so that light emission can be prevented from occurring at the interface between the light-emitting layer and an adjacent layer rather than the light-emitting layer, and deterioration of the interface can be prevented. As a result, when the compound of the present invention is used as a light-emitting auxiliary layer material, the lifespan, efficiency, and color purity of the element appear to be improved.

These results suggest that even if the core structure is similar, the properties of the compound, such as hole characteristics, luminous efficiency characteristics, energy levels (LUMO, HOMO levels, T1 levels), hole injection and mobility characteristics, and electron blocking characteristics, may differ depending on the type of substituent and the bonding position of the substituent, which may lead to completely different element results.

### [Test Example 16] to [Test Example 28] Green organic electroluminescent element (light-emitting auxiliary layer)

The organic electroluminescent elements were fabricated in the same manner as described in Example 1 except that the compounds of the present invention described in the following Table 5, instead of compound P1-2, were used as material of a light-emitting auxiliary layer.

### [Comparative Example 4]

The organic electroluminescent elements were fabricated in the same manner as described in Example 1 except that one of Comparative Compound 4, instead of compound P1-2, was used as material of a light-emitting auxiliary layer.

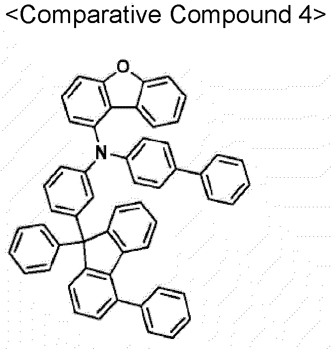

A forward bias DC voltage was applied to the electroluminescent elements manufactured in Test Examples 16 to 30 and Comparative Examples 1 and 4, and electroluminescence (EL) characteristics were measured with Photo Research's PR-650 and lifetime(T95) was measured with a lifetime measuring device manufactured by Mc Science company at 5000 cd/m² standard luminance. The measurement results are shown in Table 6 below.

**[Table 6]**

| | **Compound** | **Voltage (V)** | **Current Density (mA/cm²)** | **Brightness (cd/m²)** | **Efficiency (cd/A)** | **Lifetime T(95)** |
|---|---|---|---|---|---|---|
| **comp.Ex (1)** | **Comp. compd 1** | **6.3** | **23.1** | **5000.0** | **21.6** | **82.5** |
| **comp.Ex (4)** | **Comp. compd 4** | **6.2** | **20.2** | **5000.0** | **24.7** | **103.1** |
| **Test Ex.(16)** | **P2-1** | **5.2** | **12.4** | **5000.0** | **40.4** | **131.6** |
| **Test Ex.(17)** | **P2-5** | **5.3** | **12.8** | **5000.0** | **39.2** | **132.0** |
| **Test Ex.(18)** | **P2-8** | **5.2** | **12.4** | **5000.0** | **40.2** | **131.2** |
| **Test Ex.(19)** | **P2-14** | **5.2** | **12.3** | **5000.0** | **40.6** | **130.3** |
| **Test Ex.(20)** | **P2-32** | **5.1** | **12.0** | **5000.0** | **41.7** | **132.4** |
| **Test Ex.(21)** | **P2-35** | **5.1** | **12.1** | **5000.0** | **41.3** | **131.8** |
| **Test Ex.(22)** | **P2-39** | **5.2** | **11.9** | **5000.0** | **41.8** | **133.1** |
| **Test Ex.(23)** | **P2-58** | **5.2** | **12.2** | **5000.0** | **41.0** | **132.1** |
| **Test Ex.(24)** | **P2-61** | **5.1** | **12.3** | **5000.0** | **40.7** | **132.4** |
| **Test Ex.(25)** | **P2-78** | **5.4** | **12.9** | **5000.0** | **38.7** | **129.0** |
| **Test Ex.(26)** | **P2-88** | **5.4** | **12.7** | **5000.0** | **39.5** | **129.8** |
| **Test Ex.(27)** | **P2-98** | **5.3** | **12.4** | **5000.0** | **40.2** | **130.3** |
| **Test Ex.(28)** | **P2-102** | **5.2** | **12.1** | **5000.0** | **41.4** | **133.5** |

| | | | | | | |
|---|---|---|---|---|---|---|
| Test Ex. (23) and (25) are reference examples. | | | | | | |

From the above Table 6, it can be seen that when a green organic electroluminescent element is manufactured using the material for an organic electroluminescent element of the present invention as a light-emitting auxiliary layer material, not only can the driving voltage of the organic electroluminescent element be lowered, but also the light-emitting efficiency and lifespan can be significantly improved, compared to when Comparative Compound 1 or Comparative Compound 4 is used.

Comparative compounds 1 and 4 have very similar structures, but they differ in that Comparative Compound 1 has a hydrogen bonded at the 4-position of 9,9-diphenyl-9H-fluorene, whereas Comparative Compound 4 has a phenyl substituted at the 4-position.

Comparing Comparative Examples 1 and 4, it can be seen that in the case of Comparative Example 4, the characteristics of the element, especially the lifespan, are significantly improved. Therefore, it can be seen that even if a compound has a similar structure, when a compound in which a substituent other than hydrogen is substituted on the benzene moiety of 9,9-diphenyl-9H-fluorene, like Comparative Compound 4, is used as a light-emitting auxiliary layer material, it is advantageous in improving the lifespan of the element.

The compounds of the present invention used in the examples of Table 6 above is the same as Comparative Compound 4 in that a substituent other than hydrogen is substituted on the benzene moiety of 9,9-diphenyl-9H-fluorene. However, Comparative Compound 4 differs from the compound of the present invention in that an amine group is substituted at position 1 on dibenzofuran, but hydrogen is bonded at position 8.

That is, the compounds of the present invention used in the above examples has substituents that are asymmetrically substituted on both benzene rings of dibenzofuran, such as an amine group substituted at position 1 and Ar² substituted at position 8, or an amine group substituted at position 2 and Ar³ substituted at position 9 of dibenzofuran, whereas the comparative compound 4 has an amine group substituted only on one benzene ring of dibenzofuran and the other benzene ring is not substituted with a substituent other than hydrogen.

It can be confirmed from Table 6 above that there is a large difference in hole mobility depending on the presence or absence of these substituents and the bonding position, and that this difference can ultimately affect the element characteristics.

In addition, it appears that by using the compound of the present invention as a material for the light-emitting auxiliary layer, the HOMO or LUMO energy level of the compound of the present invention has an appropriate value between each energy level of the hole transport layer and the light-emitting layer, so that holes and electrons achieve charge balance and light emission occurs within the light-emitting layer rather than at the interface of the hole transport layer, and thus efficiency and lifespan of the element are maximized.

### [Test Example 29] to [Test Example 41] Green organic electroluminescent element (light-emitting auxiliary layer)

The organic electroluminescent elements were fabricated in the same manner as described in Example 1 except that the compounds of the present invention described in the following Table 6, instead of compound P1-2, were used as material of a light-emitting auxiliary layer.

### [Comparative Example 5]

The organic electroluminescent elements were fabricated in the same manner as described in Example 1 except that one of Comparative Compound 5, instead of compound P1-2, was used as material of a light-emitting auxiliary layer.

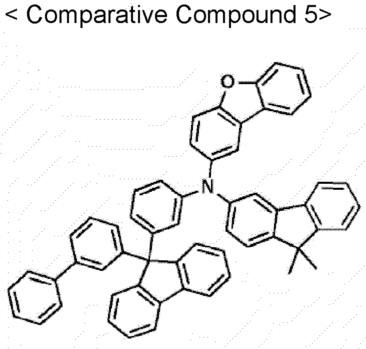

A forward bias DC voltage was applied to the electroluminescent elements manufactured in Test Examples 29 to 41 and Comparative Examples 1 and 5, and electroluminescence (EL) characteristics were measured with Photo Research's PR-650 and lifetime(T95) was measured with a lifetime measuring device manufactured by Mc Science company at 5000 cd/m² standard luminance. The measurement results are shown in Table 7 below.

**[Table 7]**

| | **Compound** | **Voltage (V)** | **Current Density (mA/cm²)** | **Brightness (cd/m²)** | **Efficiency (cd/A)** | **Lifetime T(95)** |
|---|---|---|---|---|---|---|
| **comp.Ex (1)** | **Comp. compd 1** | **6.3** | **23.1** | **5000.0** | **21.6** | **82.5** |
| **comp.Ex (5)** | **Comp. compd 5** | **6.1** | **16.1** | **5000.0** | **31.0** | **88.7** |
| **Test Ex.(29)** | **P3-1** | **5.2** | **10.9** | **5000.0** | **45.7** | **126.0** |
| **Test Ex.(30)** | **P3-7** | **5.1** | **10.8** | **5000.0** | **46.1** | **126.4** |
| **Test Ex.(31)** | **P3-12** | **5.2** | **10.8** | **5000.0** | **46.5** | **126.1** |
| **Test Ex.(32)** | **P3-21** | **5.1** | **10.9** | **5000.0** | **46.0** | **126.3** |
| **Test Ex.(33)** | **P3-32** | **5.1** | **10.3** | **5000.0** | **48.5** | **127.5** |
| **Test Ex.(34)** | **P3-48** | **5.0** | **10.6** | **5000.0** | **47.2** | **127.6** |
| **Test Ex.(35)** | **P3-53** | **5.0** | **10.4** | **5000.0** | **48.2** | **128.1** |
| **Test Ex.(36)** | **P3-56** | **5.1** | **10.5** | **5000.0** | **47.8** | **127.0** |
| **Test Ex.(37)** | **P3-80** | **5.2** | **11.1** | **5000.0** | **45.1** | **124.9** |
| **Test Ex.(38)** | **P3-82** | **5.2** | **11.2** | **5000.0** | **44.7** | **124.9** |
| **Test Ex.(39)** | **P3-83** | **5.2** | **11.2** | **5000.0** | **44.6** | **124.2** |
| **Test Ex.(40)** | **P3-96** | **5.1** | **10.3** | **5000.0** | **48.5** | **124.5** |
| **Test Ex.(41)** | **P3-104** | **5.2** | **10.4** | **5000.0** | **48.0** | **128.3** |

| | | | | | | |
|---|---|---|---|---|---|---|
| Test Ex. (37) and (40) are reference examples. | | | | | | |

From the above Table 7, it can be seen that when a green organic electroluminescent element is manufactured using the material for an organic electroluminescent element of the present invention as a light-emitting auxiliary layer material, not only can the driving voltage of the organic electroluminescent element be lowered, but also the light-emitting efficiency and lifespan can be significantly improved, compared to when Comparative Compound 1 or Comparative Compound 5 is used.

It can be seen that the element characteristics are better when Comparative Compound 5 is used as a light-emitting auxiliary layer material than when Comparative Compound 1 is used, and that the element characteristics differ depending on whether a substituent is further substituted on the phenyl at position 9 of 9,9-diphenyl-9H-fluorenyl group and the type of substituent substituted on the amine group.

The compounds of the present invention used in the examples of Table 7 are the same as Comparative Compound 5 in that one of the phenyls at position 9 of the 9,9-diphenyl-9H-fluorenyl group is substituted with a substituent other than hydrogen, but there is a difference in whether or not dibenzofuran is substituted on the amine group substituted on the other phenyl. That is, while the compounds of the present invention have an amine group substituted at position 1 of dibenzofuran and Ar² substituted at position 8, or an amine group substituted at position 2 of dibenzofuran and Ar³ substituted at position 9, Comparative Compound 5 differs in that an amine group is substituted at position 2 of dibenzofuran and a hydrogen is bonded at position 9.

From that the characteristics of the element are improved when the compound of the present invention is used as a light-emitting auxiliary layer material due to these differences, it can be seen that when a substituent is substituted at a specific position of dibenzofuran, the chemical and physical characteristics of the compound change, which also affects the performance of the element.

Therefore, by summarizing Tables 4, 6 and 7, it can be seen that among the amine compounds substituted with 9,9-diphenyl-9H-fluorenyl group and dibenzofuran, a compound in which an amine group is substituted on one benzene ring of dibenzofuran and a substituent is substituted on the other benzene ring of dibenzofuran is more suitable as a light-emitting auxiliary layer material, and a structure in which the amine group substituted on one benzene ring and the substituent substituted on the other benzene ring is substituted in asymmetrical positions(the structure in which when an amine group is substituted at position 1, another substituent is substituted at position 8, and when an amine group is substituted at position 2, another substituent is substituted at position 9.) is more suitable.

Although the exemplary embodiments of the present invention have been described for illustrative purposes, those skilled in the art to which the present invention pertains will be capable of various modifications without departing from the essential characteristics of the present invention. The scope of the present invention shall be construed on the basis of the accompanying claims, and it shall be construed that all of the technical arts included within the claimed scope belong to the present invention.

## Claims

1. A compound of Formula 1: 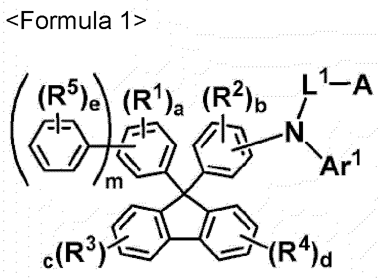 in Formula 1,
A is Formula A-1 or Formula A-2, 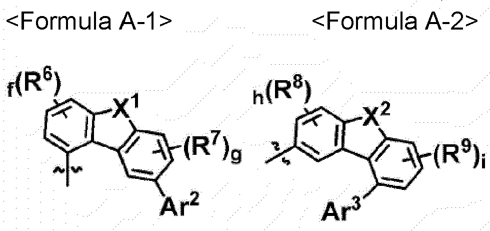
R¹ to R⁹ are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₆₀ aryloxy group, and -L'-N(Rₐ)(R_{b}), and adjacent groups may be bonded to each other to form a ring,
a is an integer of 0 to 5, b to d are each an integer of 0 to 4, e is an integer of 0 to 5, m is an integer of 0 to 2, f and i are each an integer of 0 to 3,
X¹ and X² are O or S,
L¹ and L' are each independently selected from the group consisting of a single bond, a C₆-C₆₀ arylene group, a fluorenylene group, a C₃-C₆₀ aliphatic ring group, and a C₂-C₆₀ heterocyclic group containing at least one heteroatom of O, N, S, Si and P,
Ar², Ar³, Rₐ and R_{b} are each independently selected from the group consisting of a C₆-C₆₀ aryl group, a fluorenyl group, a C₃-C₆₀ aliphatic ring group, and a C₂-C₆₀ heterocyclic group containing at least one heteroatom of O, N, S, Si and P, and
the aryl group, the arylene group, the fluorenyl group, the fluorenylene group, the heterocyclic group, the aliphatic ring group, the alkyl group, the alkenyl group, the alkynyl group, the alkoxyl group, the aryloxyl group, and the ring formed by adjacent groups may be each optionally substituted with one or more substituents selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₃₀ aryloxy group, a C₁-C₂₀ alkylthio group, and a C₆-C₃₀ arylthio group, and
wherein Ar¹ is selected from the group consisting of Formula Ar-1 to Ar-6: 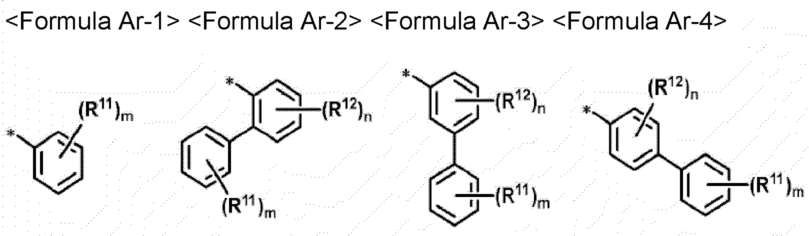 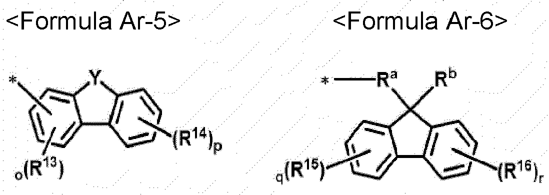
in Formula Ar-1 to Formula Ar-6,
R¹¹ to R¹⁶ are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and adjacent groups may be bonded to each other to form a C₆-C₁₄ aromatic ring group,
m' is an integer of 0 to 5, n, p, q and r are each an integer of 0 to 4, o is an integer of 0 to 3, and when each of these is an integer of 2 or more, each of R¹³s, each of R¹⁴s, each of R¹⁵s, each of R¹⁶s are the same as or different from each other,
Y is S, O, or N(R₃),
R^{a} is selected from the group consisting of a single bond, a C₁-C₂₀ alkylene group, a C₆-C₃₀ arylene group, a fluorenylene group, a C₂-C₃₀ heterocyclic group comprising at least one heteroatom selected from the group consisting of O, N, S, Si and P, and a C₃-C₃₀ aliphatic ring group, and
R₃ and R^{b} are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group.

2. The compound of claim 1, wherein Formula 1 is represented by one of Formula 2-1 to Formula 2-4: 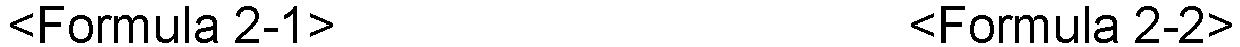 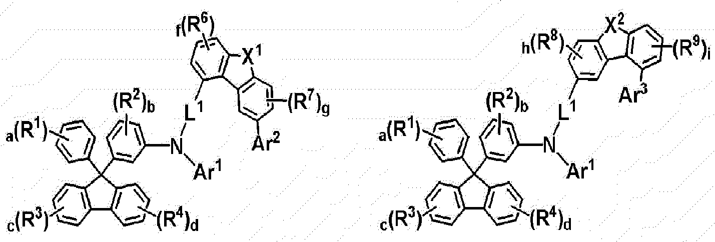 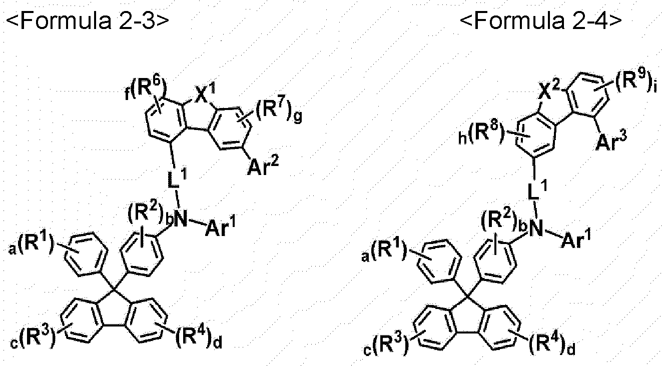 in Formula 2-1 to Formula 2-4, R¹ to R⁴, R⁶ to R⁹, X¹, X², L¹, Ar¹ to Ar³, a to d, f to i are the same as defined in claim 1.

3. The compound of claim 1, wherein Formula 1 is represented by one of Formula 3-1 to Formula 3-4: 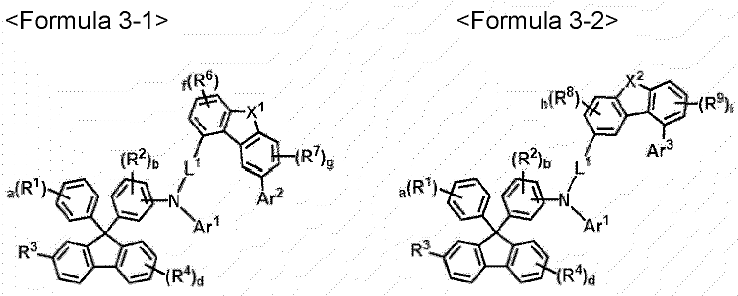 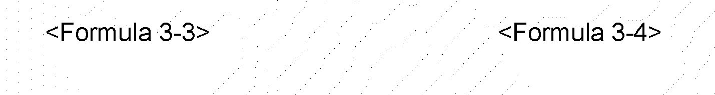 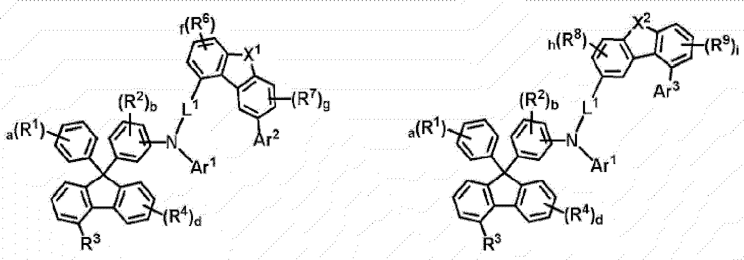 in Formula 3-1 to Formula 3-4,
R¹, R², R⁴, R⁶ to R⁹, X¹, X², L¹, Ar¹ to Ar³, a, b, d, f to i are the same as defined in claim 1, and
R³ is selected from the group consisting of deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₆₀ aryloxy group, and -L'-N(Rₐ)(R_{b}).

4. The compound of claim 1, wherein Formula 1 is represented by one of Formula 4-1 to Formula 4-8: 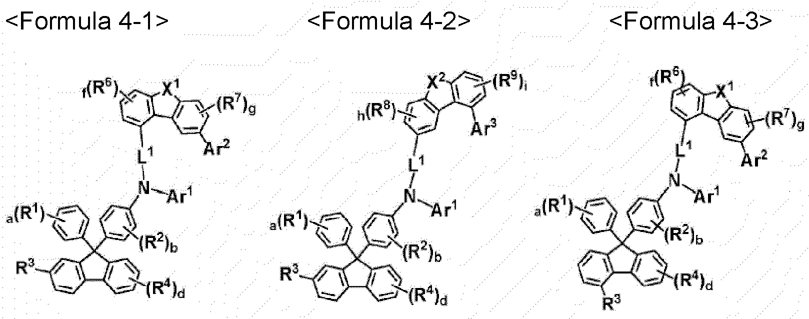 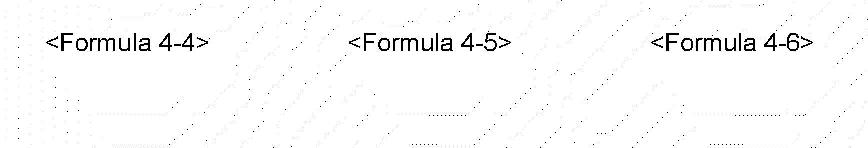 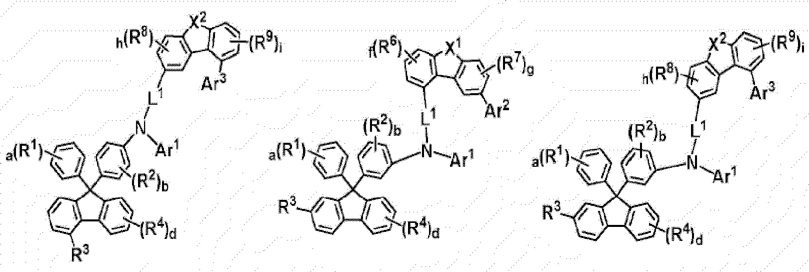 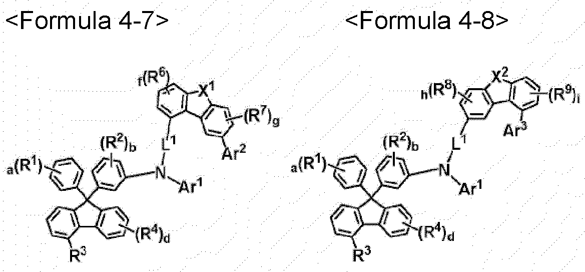
in Formula 4-1 to Formula 4-8, R¹, R², R⁴, R⁶ to R⁹, X¹, X², L¹, Ar¹ to Ar³, a, b, d, f to i are the same as defined in claim 1, and
R₃ is selected from the group consisting of deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, and -L'-N(Rₐ)(R_{b}).

5. The compound of claim 1, wherein Formula 1 is represented by Formula 5-1 or Formula 5-2: 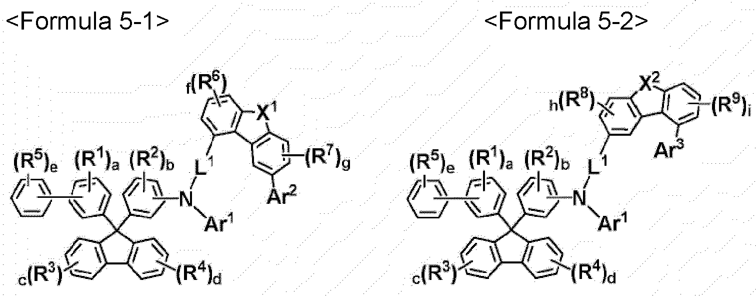 in Formula 5-1 and Formula 5-2, R¹ to R⁹, X¹, X², L¹, Ar¹ to Ar³, a to i are the same as defined in claim 1.

6. The compound of claim 1, wherein Formula 1 is represented by Formula 6-1 or Formula 6-2: 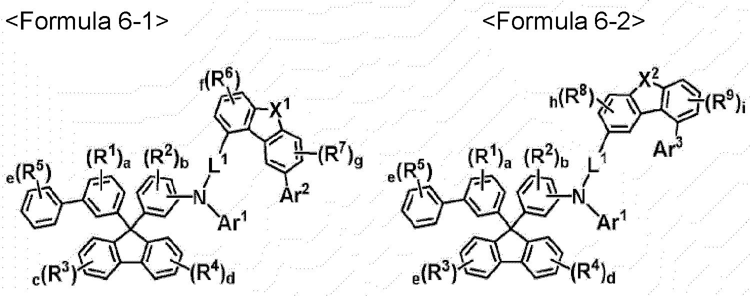 in Formula 6-1 and Formula 6-2, R¹ to R⁹, X¹, X², L¹, Ar¹ to Ar³, a to i are the same as defined in claim 1.

7. The compound of claim 1, wherein Formula 1 is represented by one of Formula 7-1 to Formula 7-4: 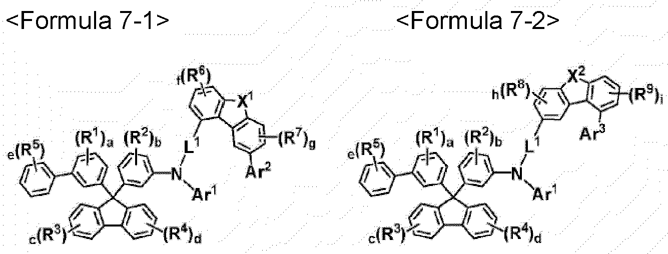 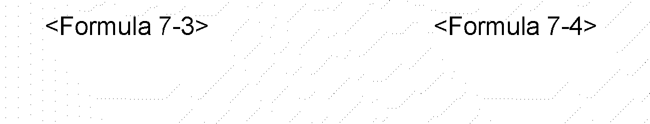 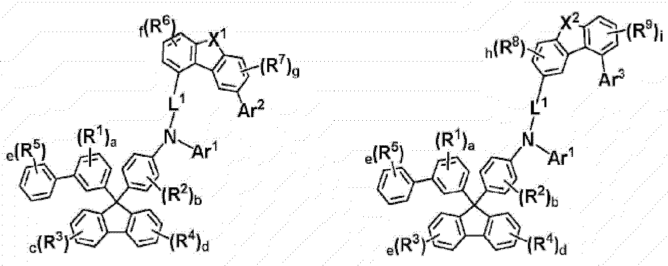 in Formula 7-1 to Formula 7-4, R¹ to R⁹, X¹, X², L¹, Ar¹ to Ar³, a to i are the same as defined in claim 1.

8. The compound of claim 1, wherein at least one of Ar² and Ar³ is selected from the group consisting of Formula Ar-1 to Ar-6: 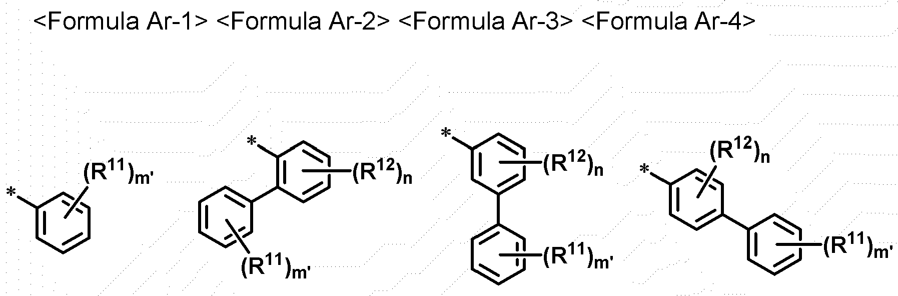 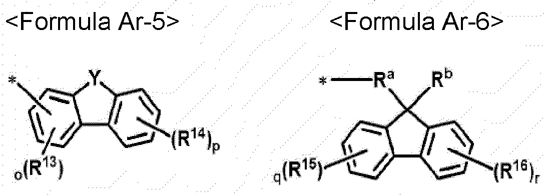 in Formula Ar-1 to Formula Ar-6,
R¹¹ to R¹⁶ are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and adjacent groups may be bonded to each other to form a ring,
m' is an integer of 0 to 5, n, p, q and r are each an integer of 0 to 4, o is an integer of 0 to 3, and when each of these is an integer of 2 or more, each of R¹³s, each of R¹⁴s, each of R¹⁵s, each of R¹⁶s are the same as or different from each other,
Y is S, O, C(R₁)(R₂) or N(R₃),
R^{a} is selected from the group consisting of a single bond, a C₁-C₂₀ alkylene group, a C₆-C₃₀ arylene group, a fluorenylene group, a C₂-C₃₀ heterocyclic group comprising at least one heteroatom selected from the group consisting of O, N, S, Si and P, and a C₃-C₃₀ aliphatic ring group, and
R₁ to R₃, R^{b} are each independently selected from the group consisting of deuterium, halogen, a cyano group, a nitro group, siloxane group, a C₆-C₃₀ aryl group, a fluorenyl group, a C₂-C₃₀ heterocyclic group comprising at least one heteroatom selected from the group consisting of O, N, S, Si and P, a C₃-C₃₀ aliphatic ring group, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, and a C₆-C₂₀ arylthio group, and R₁ and R₂ may be bonded to each other to form a ring.

9. The compound of claim 1, wherein both Ar² and Ar³ are a C₆-C₆₀ aryl group.

10. The compound of claim 1, wherein the compound represented by Formula 1 is one of the following compounds:

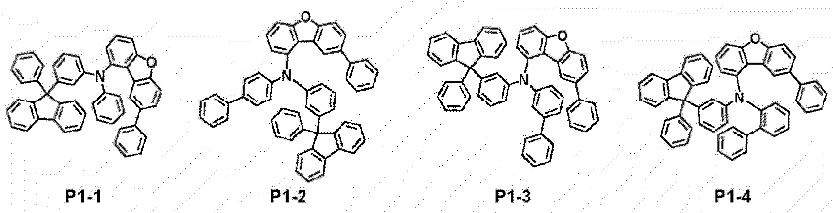

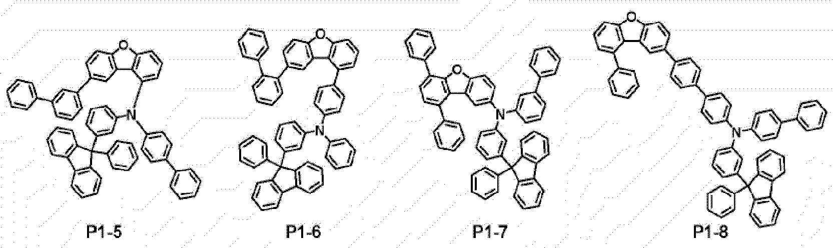

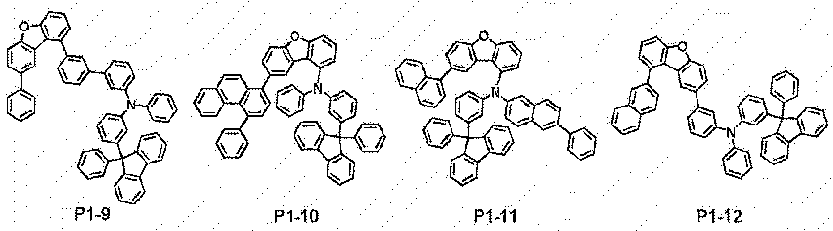

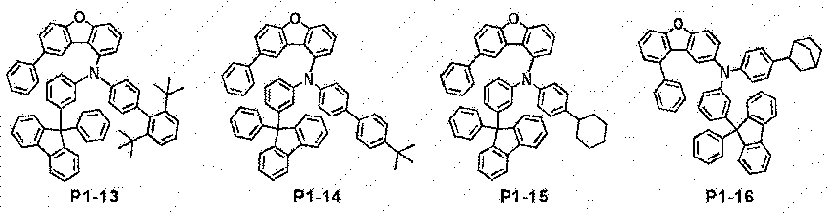

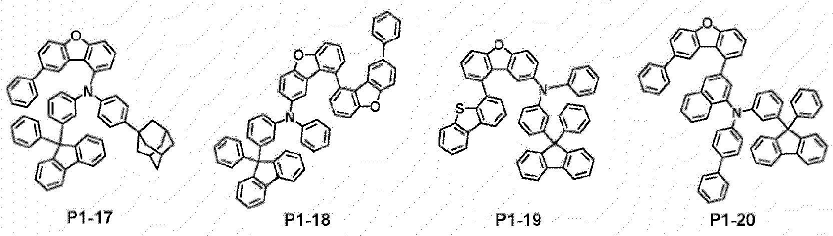

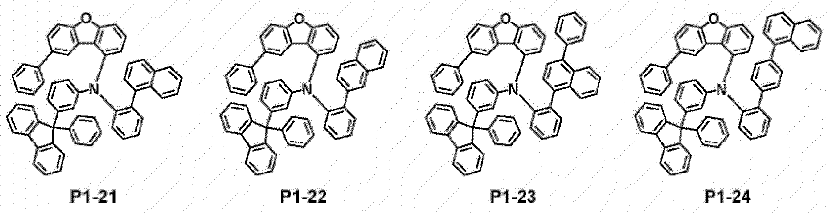

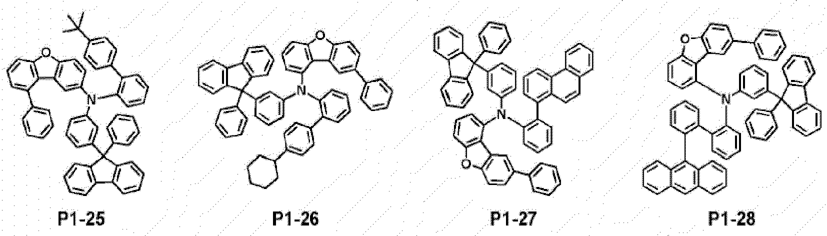

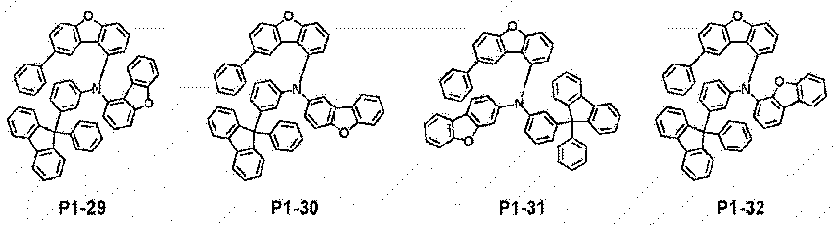

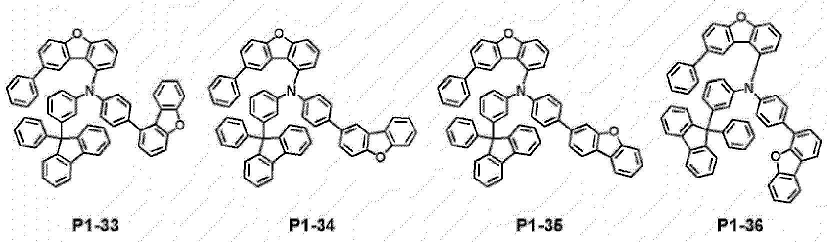

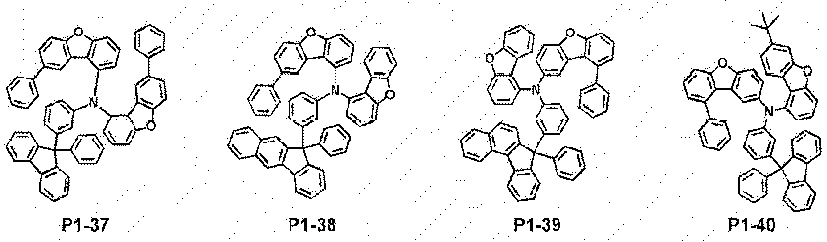

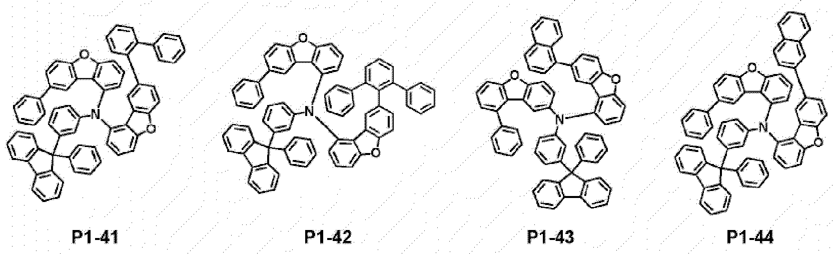

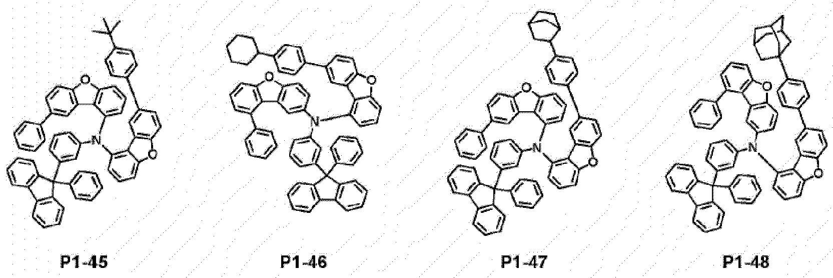

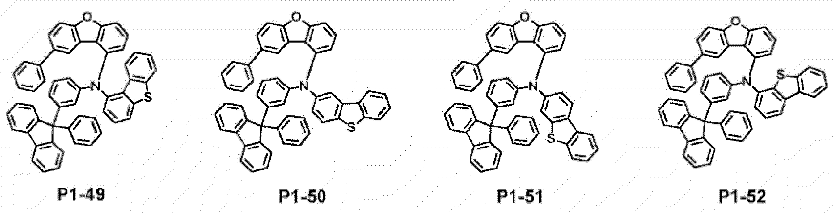

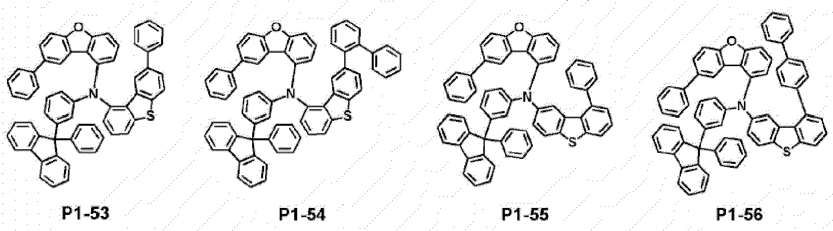

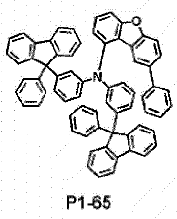

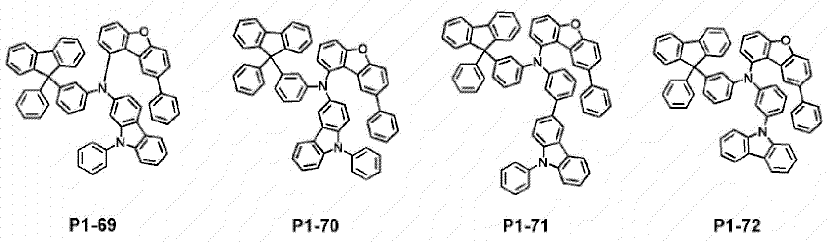

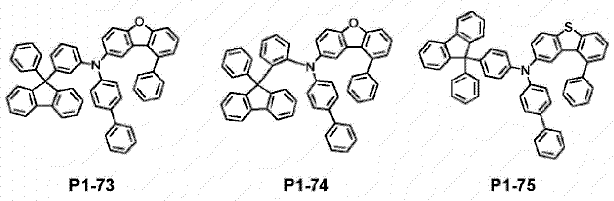

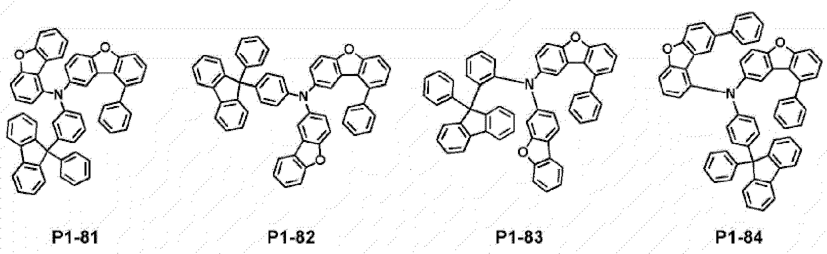

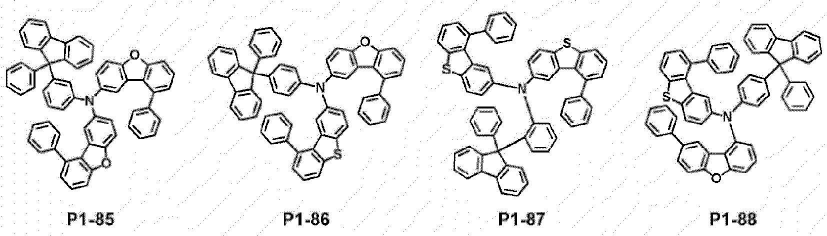

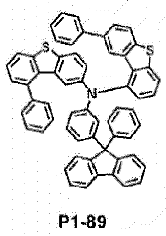

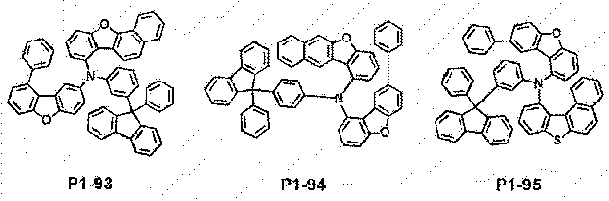

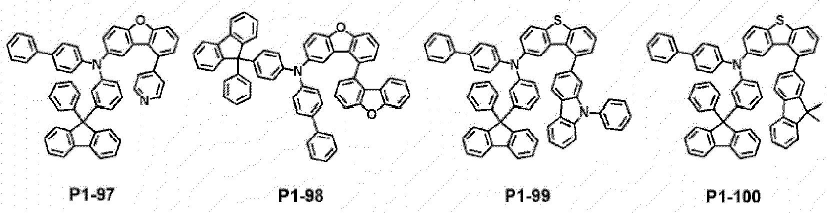

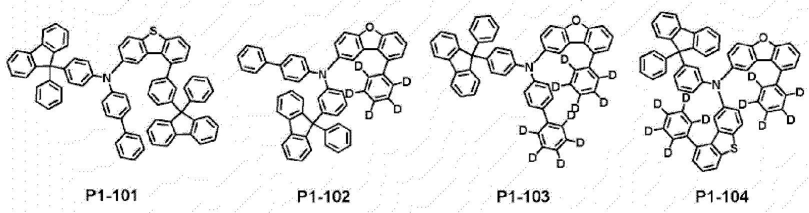

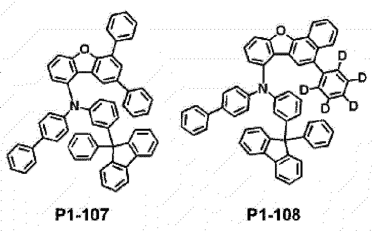

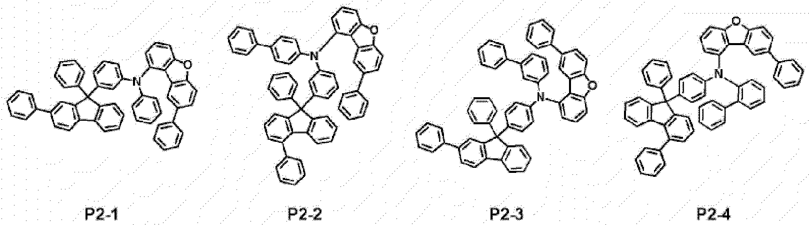

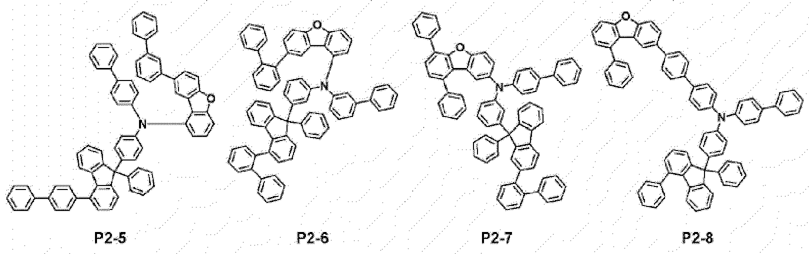

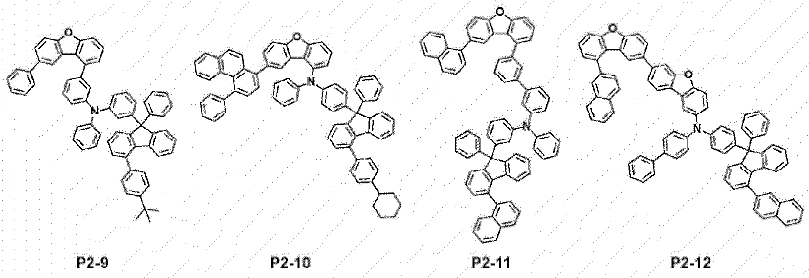

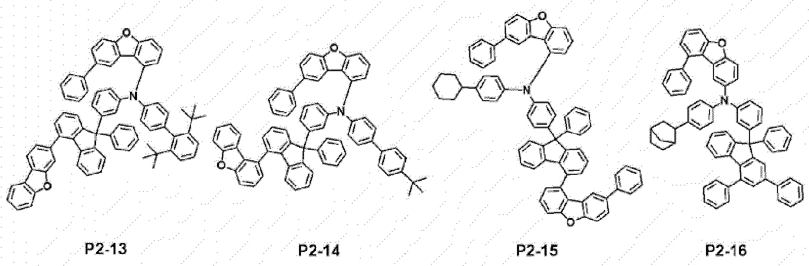

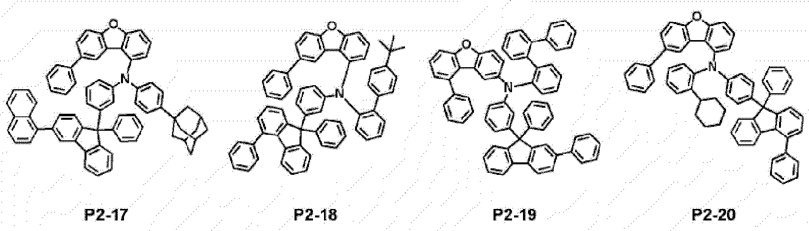

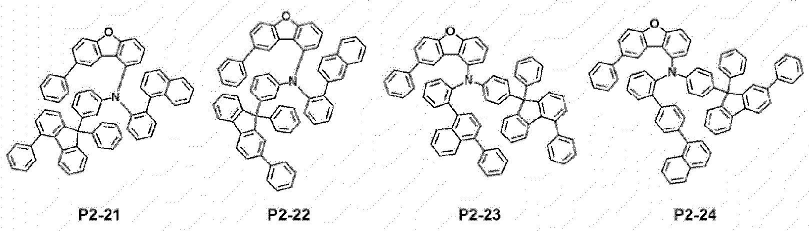

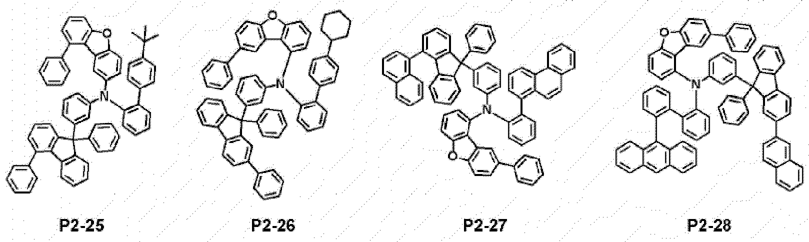

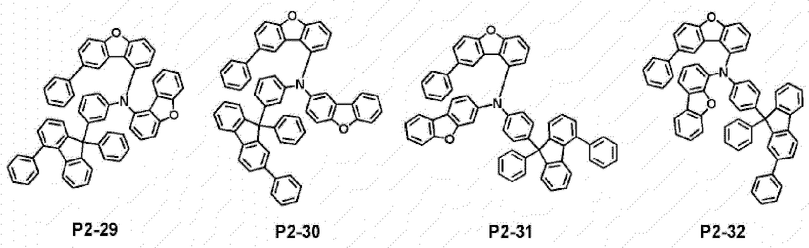

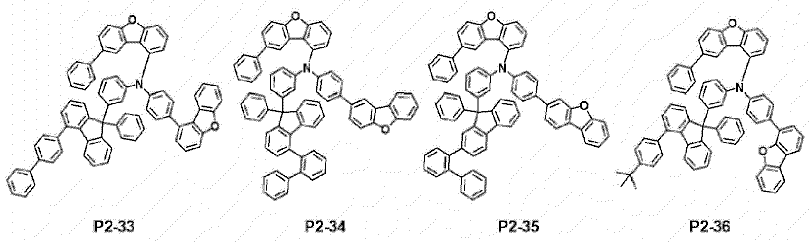

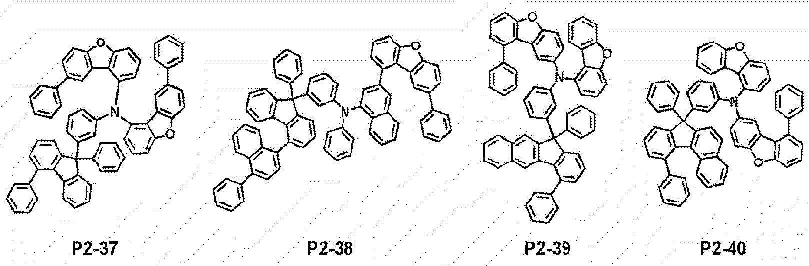

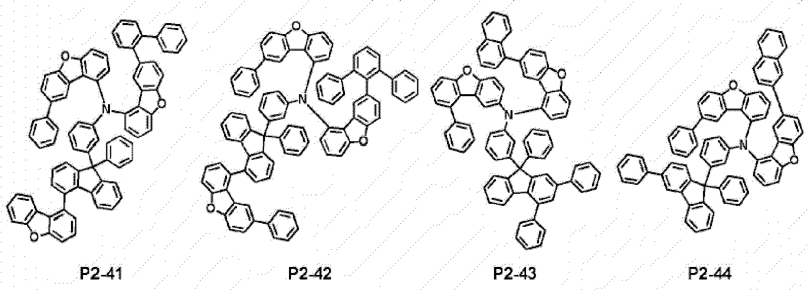

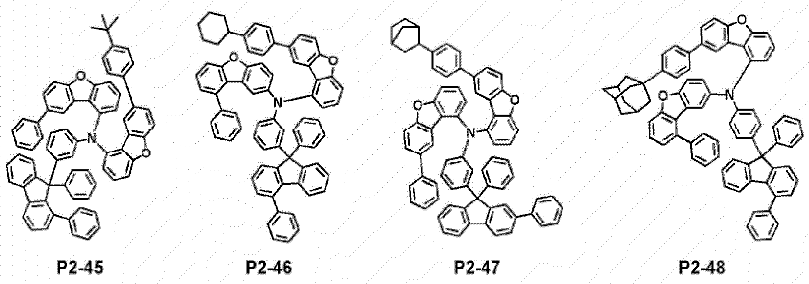

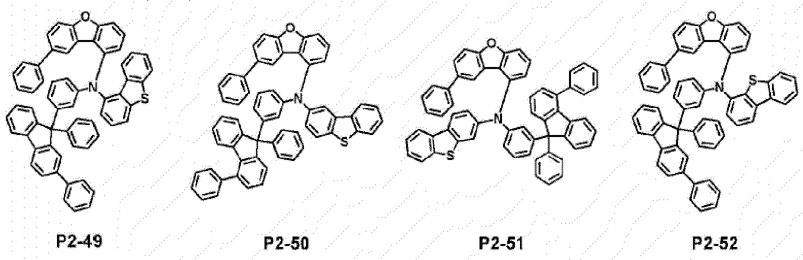

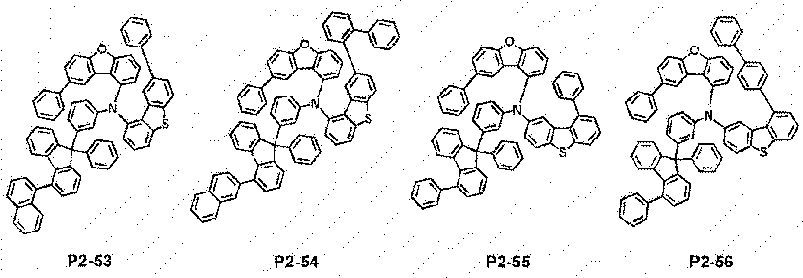

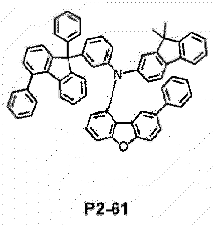

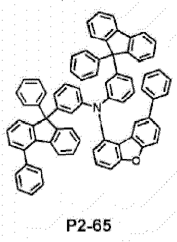

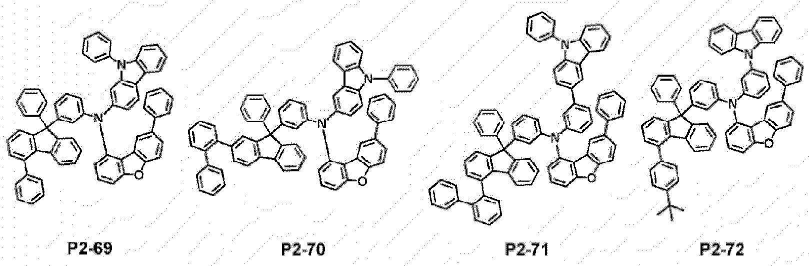

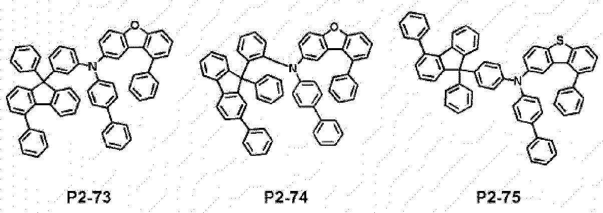

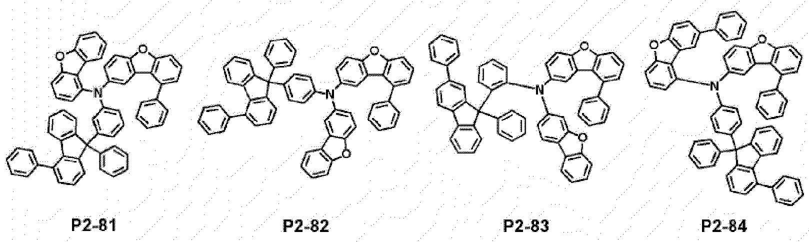

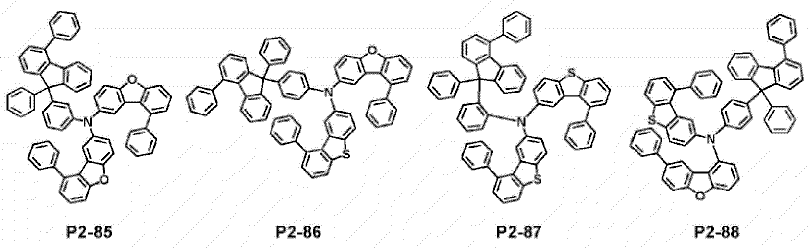

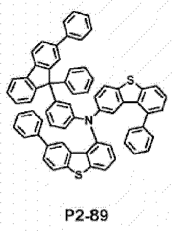

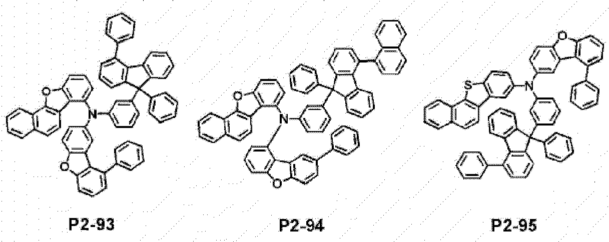

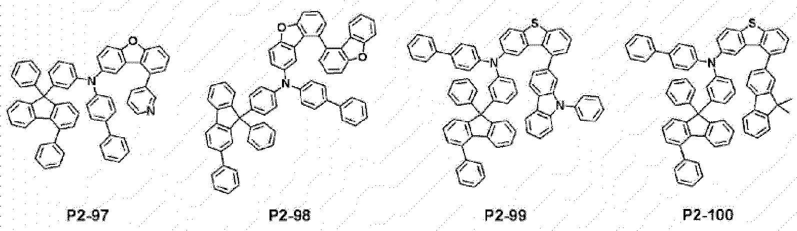

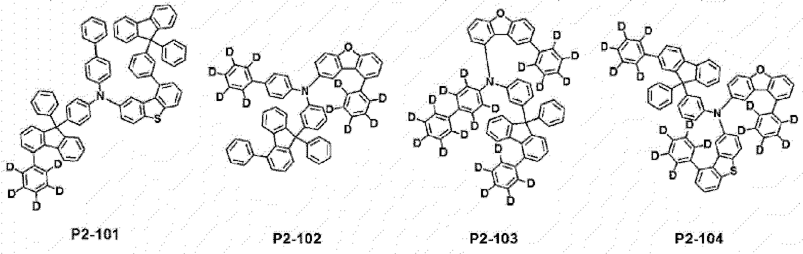

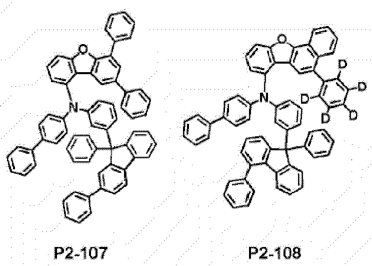

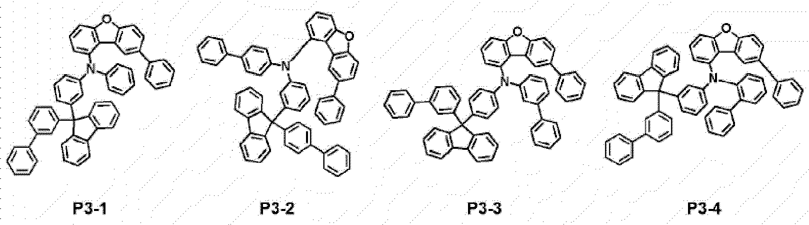

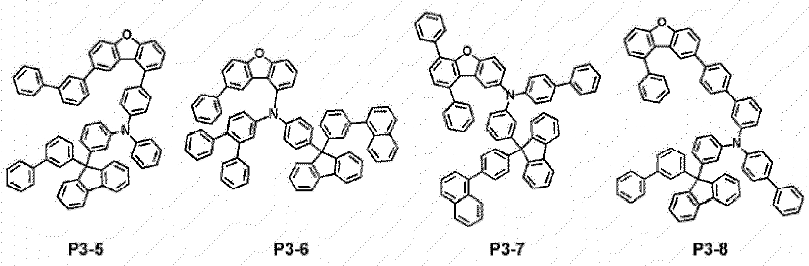

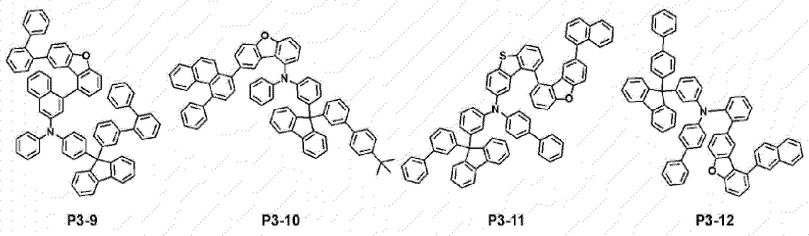

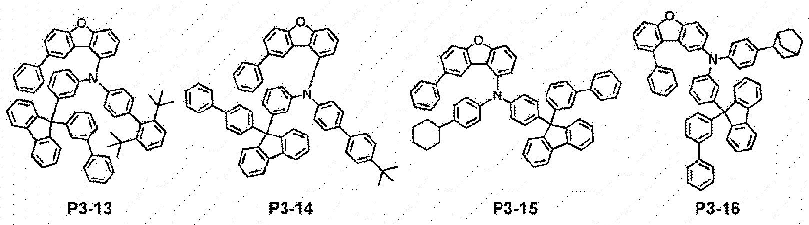

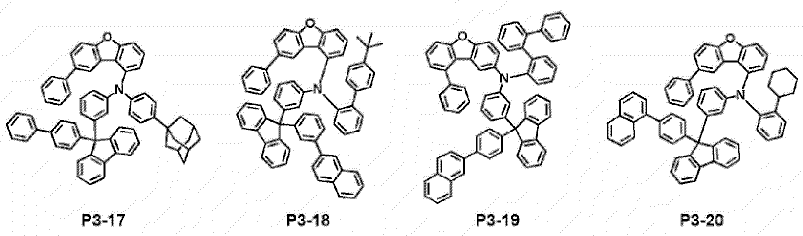

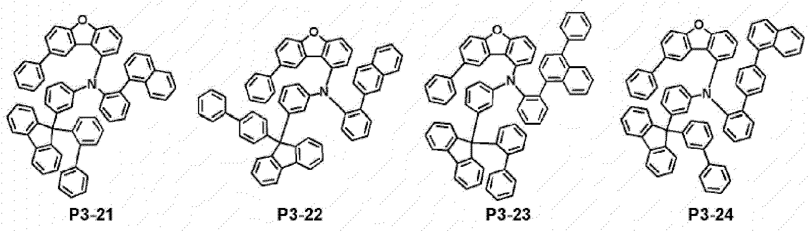

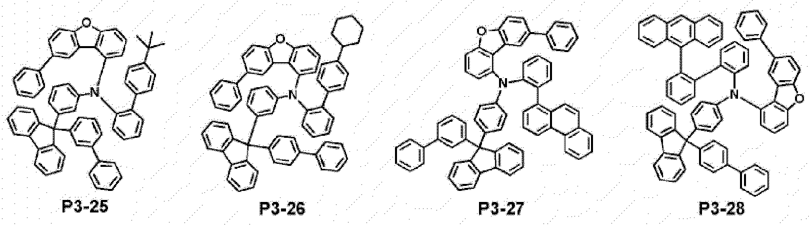

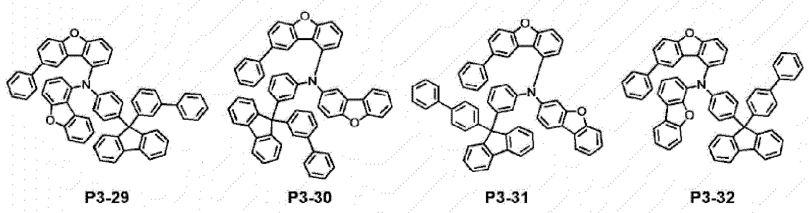

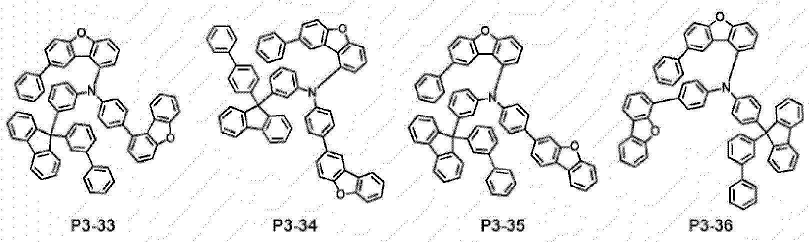

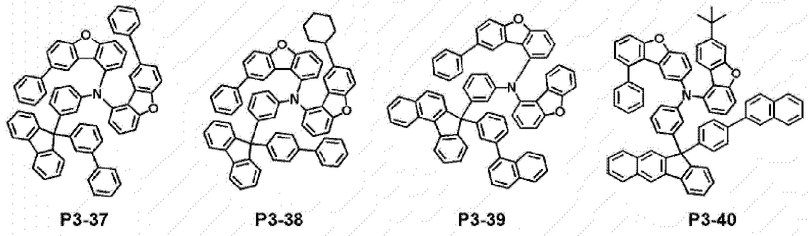

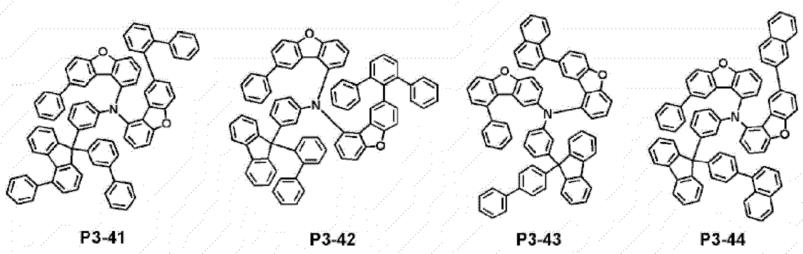

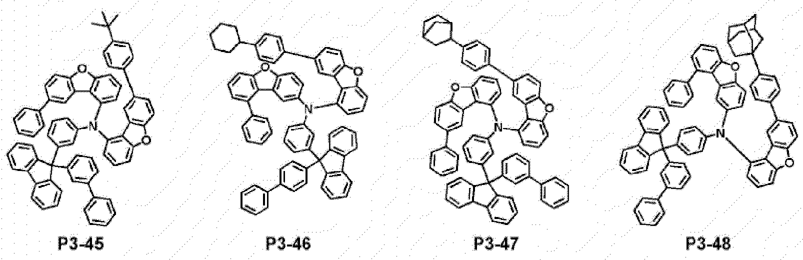

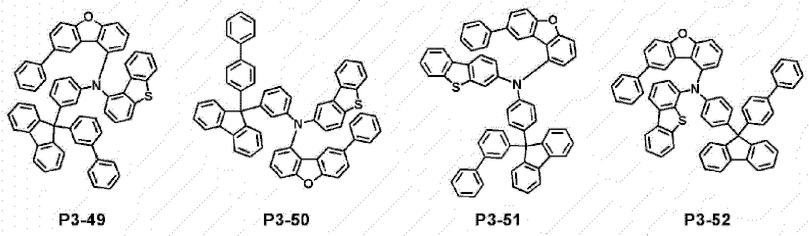

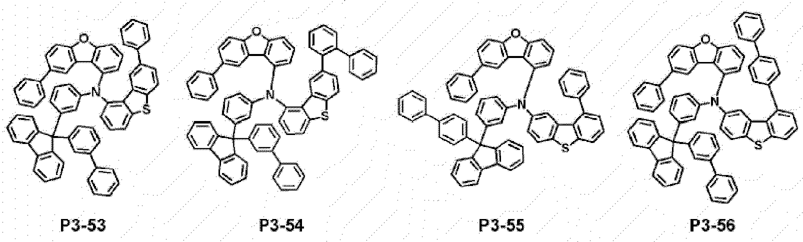

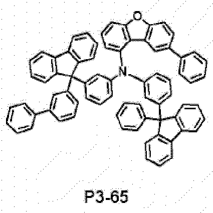

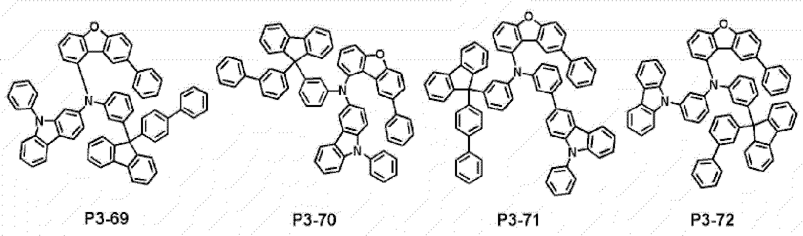

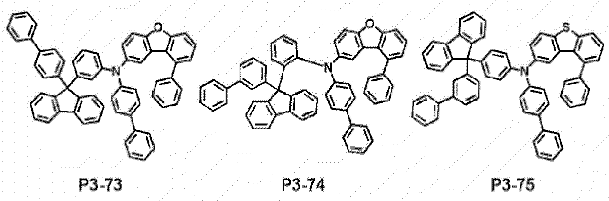

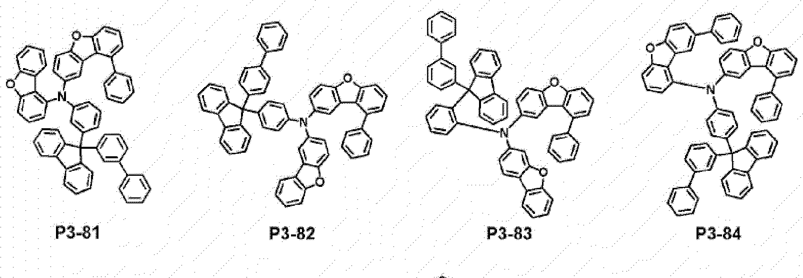

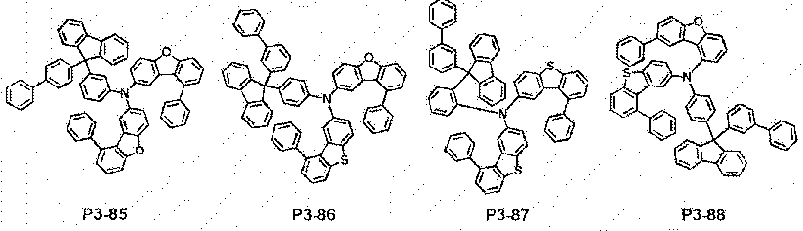

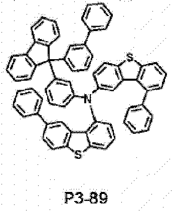

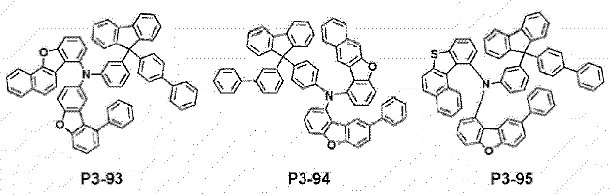

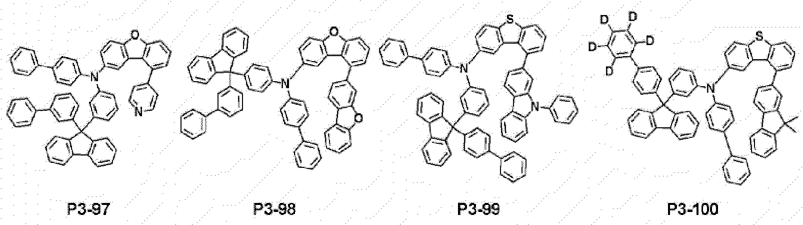

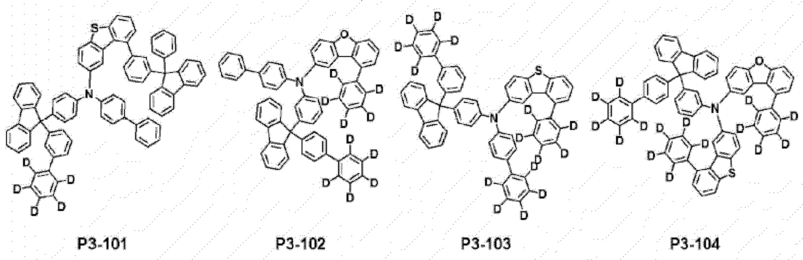

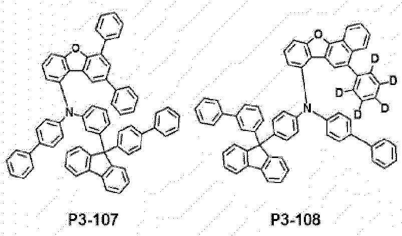

11. An organic electric element comprising a first electrode, a second electrode, and an organic material layer between the first electrode and the second electrode, wherein the organic material layer comprises the compound of Formula 1 of claim 1.

12. The organic electric element of claim 11, wherein the organic material layer further comprises a light-emitting layer between the first electrode and the second electrode, and a light-emitting auxiliary layer between the light-emitting layer and the first electrode, and the light-emitting auxiliary layer comprises the compound.

13. The organic electric element of claim 11, wherein the organic material layer comprises two or more stacks, and the two or more stacks each comprise a hole transport layer, a light-emitting layer and an electron transport layer formed sequentially on the first electrode.

14. The organic electric element of claim 13, wherein the organic material layer further comprises a charge generation layer between the two or more stacks.

15. The organic electric element of claim 11, wherein the organic electric element further comprises a layer for improving luminous efficiency, and the layer for improving luminous efficiency is formed on one side of both sides of the first electrode or the second, and the one side not facing the organic material layer.

16. An electronic device comprising a display device and a control unit for driving the display device, wherein the display device comprises the organic electric element of claim 11.

17. The electronic device of claim 16, wherein the organic electric element is selected from the group consisting of an organic electroluminescent element, an organic solar cell, an organic photo conductor, an organic transistor, an element for monochromatic illumination and a quantum dot display.

## Patentansprüche

1. Verbindung der Formel 1: 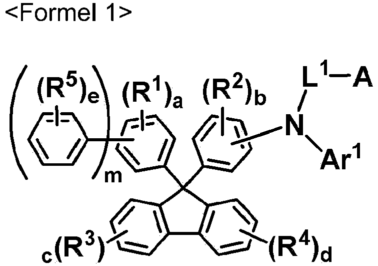 wobei in Formel 1
A Formel A-1 oder Formel A-2 ist, 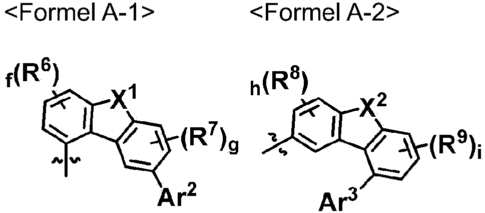
R¹ bis R⁹ jeweils unabhängig ausgewählt sind aus der Gruppe bestehend aus Wasserstoff, Deuterium, Halogen, einer Cyanogruppe, einer Nitrogruppe, einer C₆-C₆₀-Arylgruppe, einer Fluorenylgruppe, einer heterocyclischen C₂-C₆₀-Gruppe, die mindestens ein Heteroatom von O, N, S, Si und P enthält, einer aliphatischen C₃-C₆₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₆₀-Aryloxygruppe und -L'-N(Rₐ)(R_{b}), und benachbarte Gruppen aneinander gebunden sein können, um einen Ring zu bilden,
a eine ganze Zahl von 0 bis 5 ist, b bis d jeweils eine ganze Zahl von 0 bis 4 sind, e eine ganze Zahl von 0 bis 5 ist, m eine ganze Zahl von 0 bis 2 ist, f und i jeweils eine ganze Zahl von 0 bis 3 sind,
X¹ und X² O oder S sind,
L' und L' jeweils unabhängig ausgewählt sind aus der Gruppe bestehend aus einer Einfachbindung, einer C₆-C₆₀-Arylengruppe, einer Fluorenylengruppe, einer aliphatischen C₃-C₆₀-Ringgruppe und einer heterocyclischen C₂-C₆₀-Gruppe, die mindestens ein Heteroatom von O, N, S, Si und P enthält,
Ar², Ar³, Rₐ und R_{b} jeweils unabhängig ausgewählt sind aus der Gruppe bestehend aus einer C₆-C₆₀-Arylgruppe, einer Fluorenylgruppe, einer aliphatischen C₃-C₆₀-Ringgruppe und einer heterocyclischen C₂-C₆₀-Gruppe, die mindestens ein Heteroatom von O, N, S, Si und P enthält, und
die Arylgruppe, die Arylengruppe, die Fluorenylgruppe, die Fluorenylengruppe, die heterocyclische Gruppe, die aliphatische Ringgruppe, die Alkylgruppe, die Alkenylgruppe, die Alkinylgruppe, die Alkoxylgruppe, die Aryloxylgruppe und der durch benachbarte Gruppen gebildete Ring jeweils gegebenenfalls mit einem oder mehreren Substituenten substituiert sein können, ausgewählt aus der Gruppe bestehend aus Deuterium, Halogen, einer Cyanogruppe, einer Nitrogruppe, einer Siloxangruppe, einer C₆-C₃₀-Arylgruppe, einer Fluorenylgruppe, einer heterocyclischen C₂-C₃₀-Gruppe, die mindestens ein Heteroatom umfasst, ausgewählt aus der Gruppe bestehend aus O, N, S, Si und P, einer aliphatischen C₃-C₃₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₃₀-Aryloxygruppe, einer C₁-C₂₀-Alkylthiogruppe und einer C₆-C₃₀-Arylthiogruppe, und
wobei Ar¹ ausgewählt ist aus der Gruppe bestehend aus Formel Ar-1 bis Ar-6: 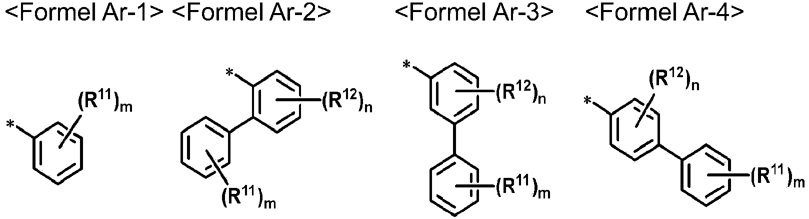 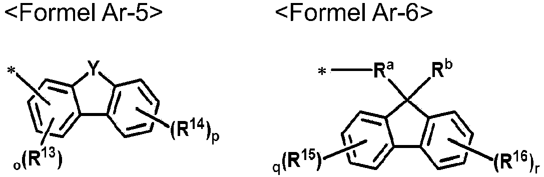
wobei in Formel Ar-1 bis Formel Ar-6,
R¹¹ bis R¹⁶ jeweils unabhängig ausgewählt sind aus der Gruppe bestehend aus Deuterium, Halogen, einer Cyanogruppe, einer Nitrogruppe, einer Siloxangruppe, einer C₆-C₃₀-Arylgruppe, einer Fluorenylgruppe, einer heterocyclischen C₂-C₃₀-Gruppe, die mindestens ein Heteroatom umfasst, ausgewählt aus der Gruppe bestehend aus O, N, S, Si und P, einer aliphatischen C₃-C₃₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₂₀-Aryloxygruppe, einer C₁-C₂₀-Alkylthiogruppe und einer C₆-C₂₀-Arylthiogruppe, und benachbarte Gruppen aneinander gebunden sein können, um eine aromatische C₆-C₁₄-Ringgruppe zu bilden,
m' eine ganze Zahl von 0 bis 5 ist, n, p, q und r jeweils eine ganze Zahl von 0 bis 4 sind, o eine ganze Zahl von 0 bis 3 ist, und wenn jedes von diesen eine ganze Zahl von 2 oder mehr ist, jedes von R¹³, jedes von R¹⁴, jedes von R¹⁵, jedes von R¹⁶ gleich oder verschieden voneinander sind,
Y S, O oder N(R₃) ist,
R^{a} ausgewählt ist aus der Gruppe bestehend aus einer Einfachbindung, einer C₁-C₂₀-Alkylengruppe, einer C₆-C₃₀-Arylengruppe, einer Fluorenylengruppe, einer heterocyclischen C₂-C₃₀-Gruppe, die mindestens ein Heteroatom umfasst, ausgewählt aus der Gruppe bestehend aus O, N, S, Si und P, und einer aliphatischen C₃-C₃₀-Ringgruppe, und
R₃ und R^{b} jeweils unabhängig ausgewählt sind aus der Gruppe bestehend aus Deuterium, Halogen, einer Cyanogruppe, einer Nitrogruppe, einer Siloxangruppe, einer C₆-C₃₀-Arylgruppe, einer Fluorenylgruppe, einer heterocyclischen C₂-C₃₀-Gruppe, die mindestens ein Heteroatom umfasst, ausgewählt aus der Gruppe bestehend aus O, N, S, Si und P, einer aliphatischen C₃-C₃₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₂₀-Aryloxygruppe, einer C₁-C₂₀-Alkylthiogruppe und einer C₆-C₂₀-Arylthiogruppe.

2. Verbindung gemäß Anspruch 1, wobei Formel 1 durch eine der Formeln 2-1 bis 2-4 dargestellt ist: 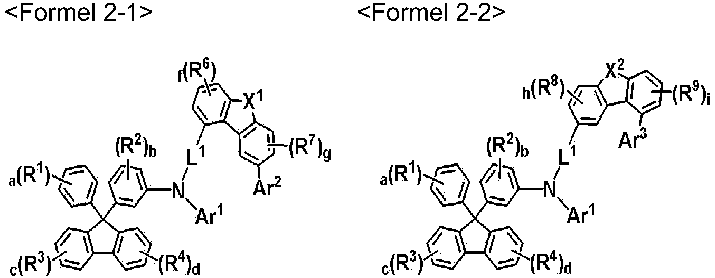 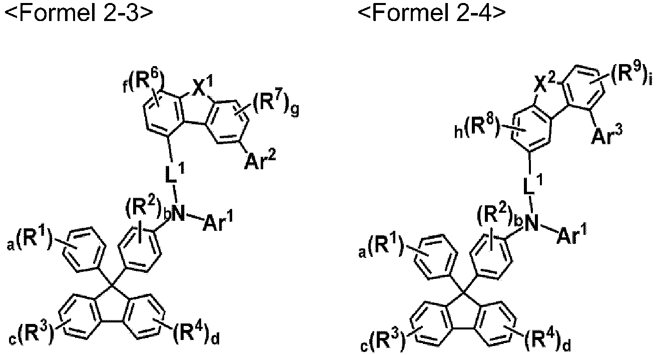 **in Formel** 2-1 bis 2-4 sind R¹ bis R⁴, R⁶ bis R⁹, X¹, X², L¹, Ar¹ bis Ar³, a bis d, f bis i dieselben wie in Anspruch 1 festgelegt.

3. Verbindung gemäß Anspruch 1, wobei Formel 1 durch eine der Formeln 3-1 bis 3-4 dargestellt ist: 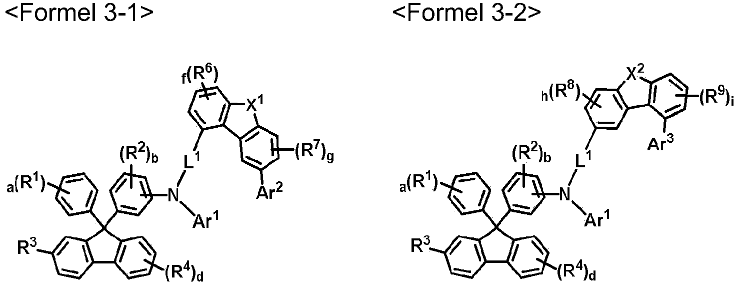 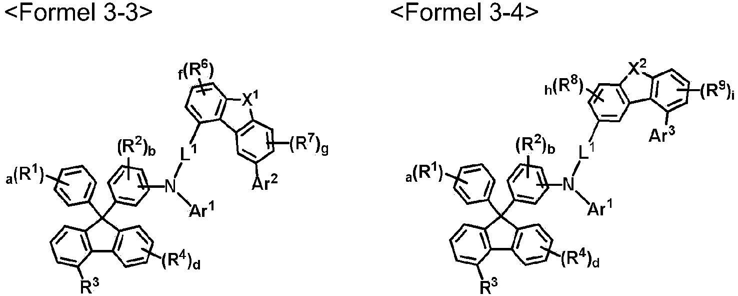 in Formel 3-1 bis 3-4 sind
R¹, R², R⁴, R⁶ bis R⁹, X¹, X², L¹, Ar¹ bis Ar³, a, b, d, f bis i dieselben wie in Anspruch 1 festgelegt, und
R₃ ist ausgewählt aus der Gruppe bestehend aus Deuterium, Halogen, einer Cyanogruppe, einer Nitrogruppe, einer C₆-C₆₀-Arylgruppe, einer Fluorenylgruppe, einer heterocyclischen C₂-C₆₀-Gruppe, die mindestens ein Heteroatom von O, N, S, Si und P enthält, einer aliphatischen C₃-C₆₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₆₀-Aryloxygruppe und -L'-N(Rₐ)(R_{b}).

4. Verbindung gemäß Anspruch 1, wobei Formel 1 durch eine der Formeln 4-1 bis 4-8 dargestellt ist: 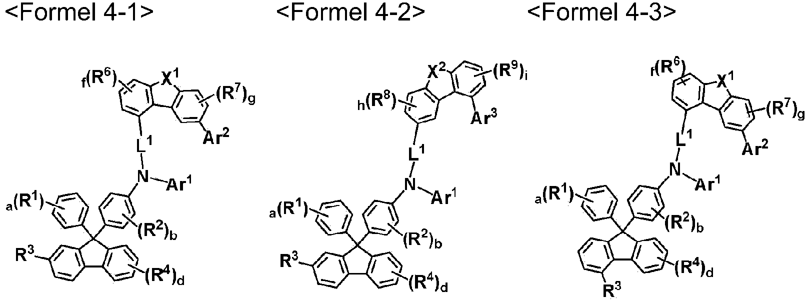 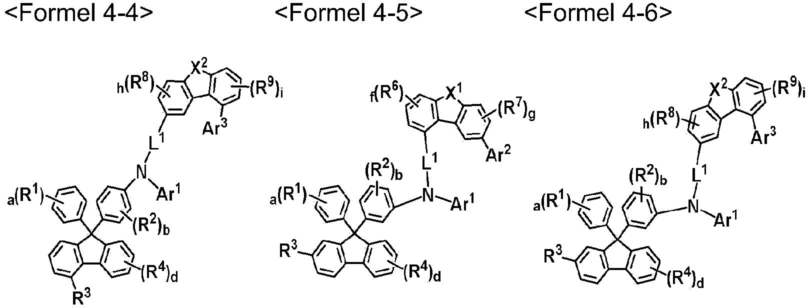 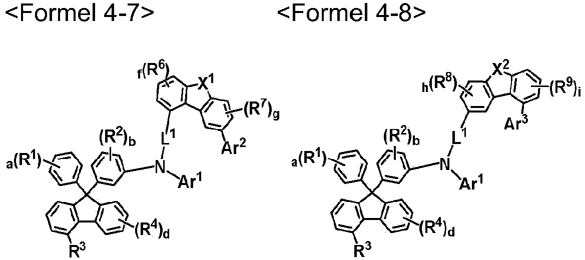
in Formel 4-1 bis 4-8 sind R¹, R², R⁴, R⁶ bis R⁹, X¹, X², L¹, Ar¹ bis Ar³, a, b, d, f bis i dieselben wie in Anspruch 1 festgelegt, und
R₃ ist ausgewählt aus der Gruppe bestehend aus Deuterium, Halogen, einer Cyanogruppe, einer Nitrogruppe, einer C₆-C₆₀-Arylgruppe, einer Fluorenylgruppe, einer heterocyclischen C₂-C₆₀-Gruppe, die mindestens ein Heteroatom von O, N, S, Si und P enthält, einer aliphatischen C₃-C₆₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₂₀-Aryloxygruppe und -L'-N(Rₐ)(R_{b}).

5. Verbindung gemäß Anspruch 1, wobei Formel 1 durch Formel 5-1 oder Formel 5-2 dargestellt ist: 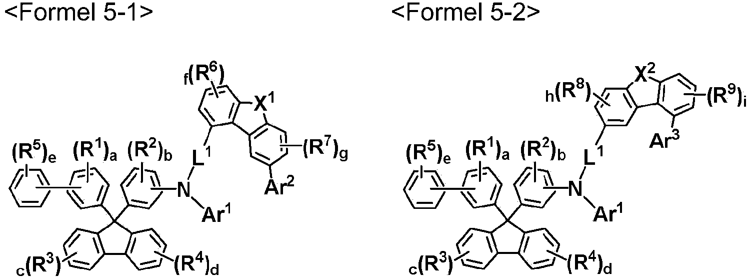 in Formel 5-1 und 5-2 sind R¹ bis R⁹, X¹, X², L¹, Ar¹ bis Ar³, a bis i dieselben wie in Anspruch 1 festgelegt.

6. Verbindung gemäß Anspruch 1, wobei Formel 1 durch Formel 6-1 oder Formel 6-2 dargestellt ist: 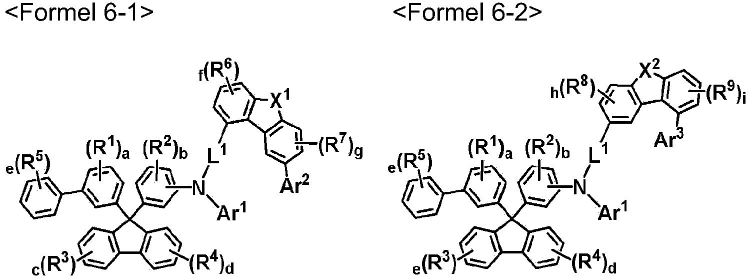 in Formel 6-1 und 6-2 sind R¹ bis R⁹, X¹, X², L¹, Ar¹ bis Ar³, a bis i dieselben wie in Anspruch 1 festgelegt.

7. Verbindung gemäß Anspruch 1, wobei Formel 1 durch eine der Formeln 7-1 bis 7-4 dargestellt ist: 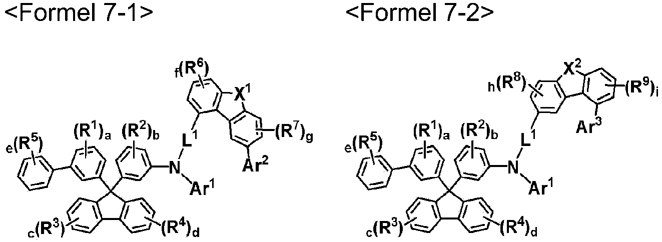 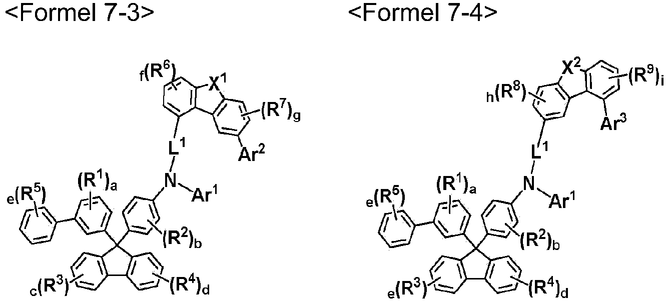 in Formel 7-1 bis 7-4 sind R¹ bis R⁹, X¹, X², L¹, Ar¹ bis Ar³, a bis i dieselben wie in Anspruch 1 festgelegt.

8. Verbindung gemäß Anspruch 1, wobei mindestens eines von Ar² und Ar³ ausgewählt ist aus der Gruppe bestehend aus Formel Ar-1 bis Ar-6: 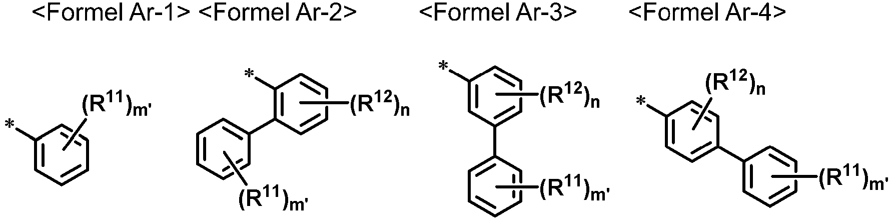 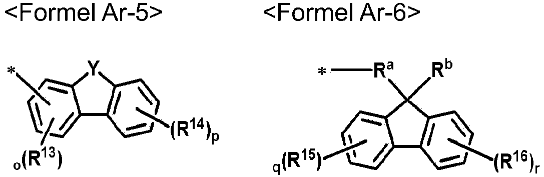 wobei in Formel Ar-1 bis Formel Ar-6,
R¹¹ bis R¹⁶ jeweils unabhängig ausgewählt sind aus der Gruppe bestehend aus Deuterium, Halogen, einer Cyanogruppe, einer Nitrogruppe, einer Siloxangruppe, einer C₆-C₃₀-Arylgruppe, einer Fluorenylgruppe, einer heterocyclischen C₂-C₃₀-Gruppe, die mindestens ein Heteroatom umfasst, ausgewählt aus der Gruppe bestehend aus O, N, S, Si und P, einer aliphatischen C₃-C₃₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₂₀-Aryloxygruppe, einer C₁-C₂₀-Alkylthiogruppe und einer C₆-C₂₀-Arylthiogruppe, und benachbarte Gruppen aneinander gebunden sein können, um einen Ring zu bilden,
m' eine ganze Zahl von 0 bis 5 ist, n, p, q und r jeweils eine ganze Zahl von 0 bis 4 sind, o eine ganze Zahl von 0 bis 3 ist, und wenn jedes von diesen eine ganze Zahl von 2 oder mehr ist, jedes von R¹³, jedes von R¹⁴, jedes von R¹⁵, jedes von R¹⁶ gleich oder verschieden voneinander sind,
Y S, O, C(R₁)(R₂) oder N(R₃) ist,
R^{a} ausgewählt ist aus der Gruppe bestehend aus einer Einfachbindung, einer C₁-C₂₀-Alkylengruppe, einer C₆-C₃₀-Arylengruppe, einer Fluorenylengruppe, einer heterocyclischen C₂-C₃₀-Gruppe, die mindestens ein Heteroatom umfasst, ausgewählt aus der Gruppe bestehend aus O, N, S, Si und P, und einer aliphatischen C₃-C₃₀-Ringgruppe, und
R₁ bis R₃, R^{b} jeweils unabhängig ausgewählt sind aus der Gruppe bestehend aus Deuterium, Halogen, einer Cyanogruppe, einer Nitrogruppe, einer Siloxangruppe, einer C₆-C₃₀-Arylgruppe, einer Fluorenylgruppe, einer heterocyclischen C₂-C₃₀-Gruppe, die mindestens ein Heteroatom umfasst, ausgewählt aus der Gruppe bestehend aus O, N, S, Si und P, einer aliphatischen C₃-C₃₀-Ringgruppe, einer C₁-C₂₀-Alkylgruppe, einer C₂-C₂₀-Alkenylgruppe, einer C₂-C₂₀-Alkinylgruppe, einer C₁-C₂₀-Alkoxylgruppe, einer C₆-C₂₀-Aryloxygruppe, einer C₁-C₂₀-Alkylthiogruppe und einer C₆-C₂₀-Arylthiogruppe, und R₁ und R₂ aneinander gebunden sein können, um einen Ring zu bilden.

9. Verbindung gemäß Anspruch 1, wobei sowohl Ar² als auch Ar³ eine C₆-C₆₀-Arylgruppe sind.

10. Verbindung gemäß Anspruch 1, wobei die durch Formel 1 dargestellte Verbindung eine der folgenden Verbindungen ist:

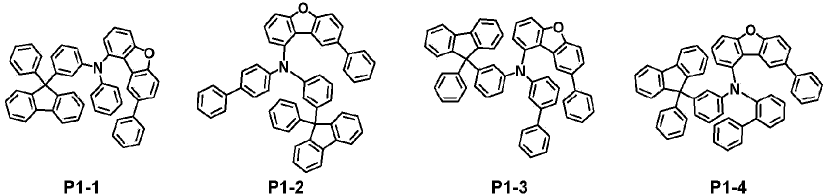

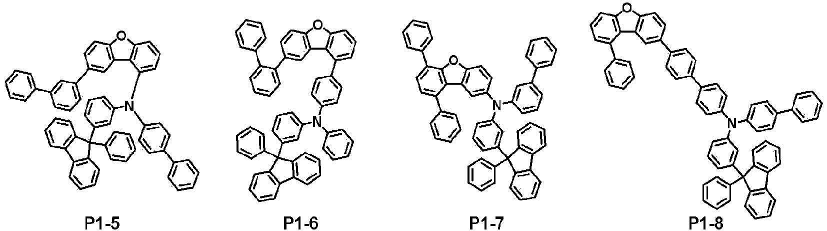

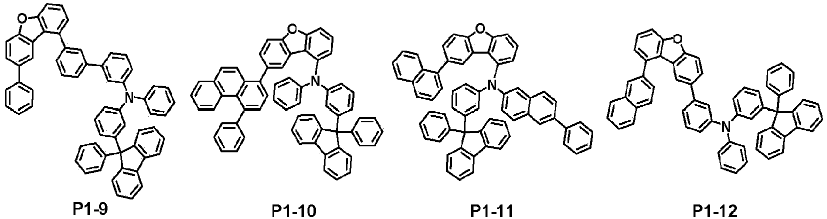

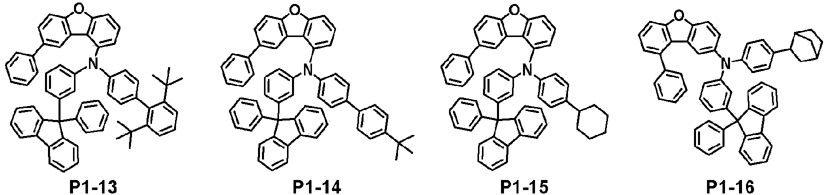

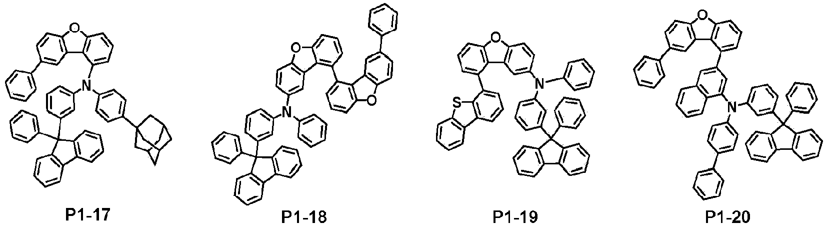

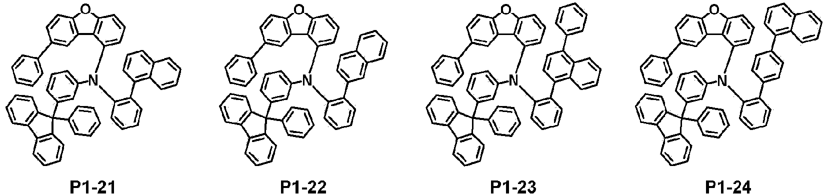

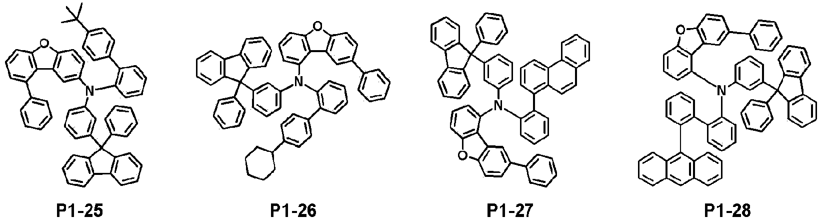

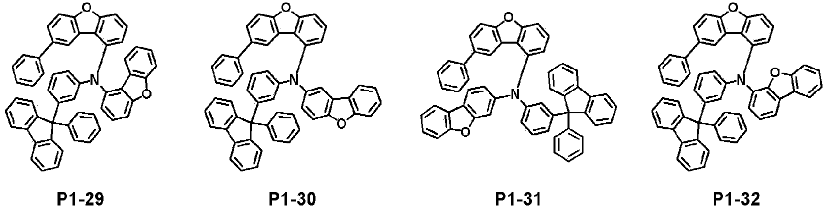

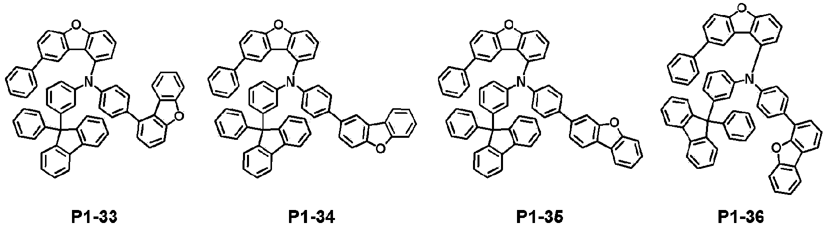

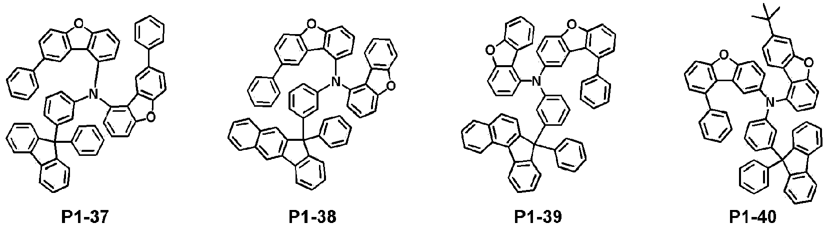

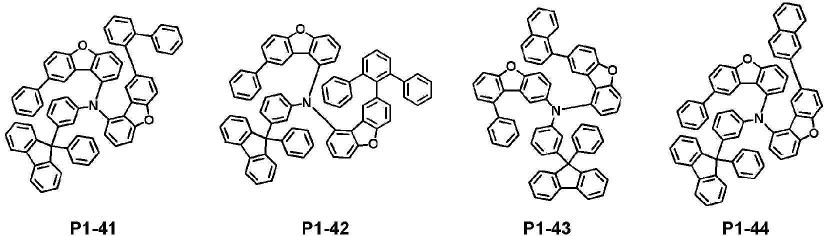

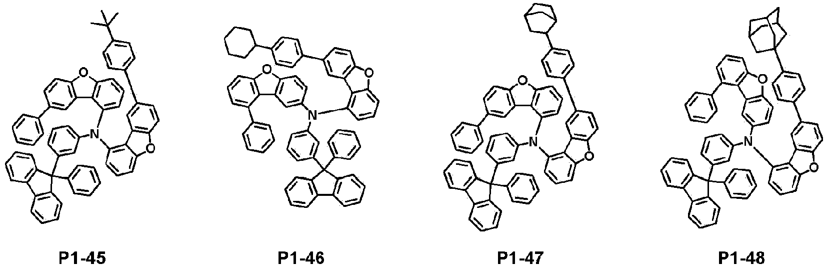

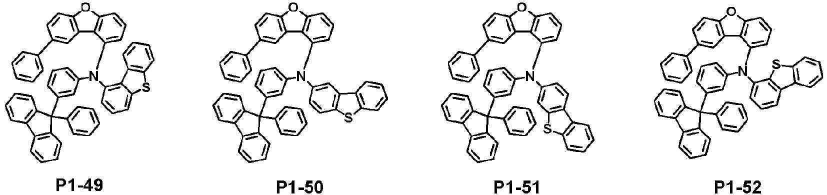

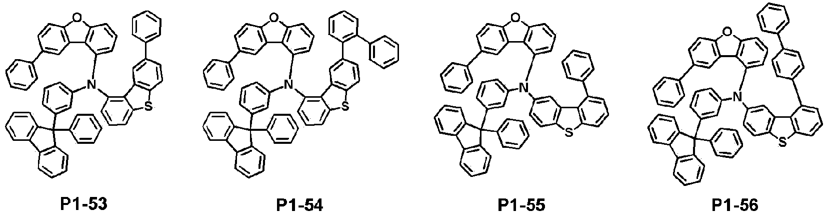

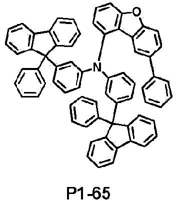

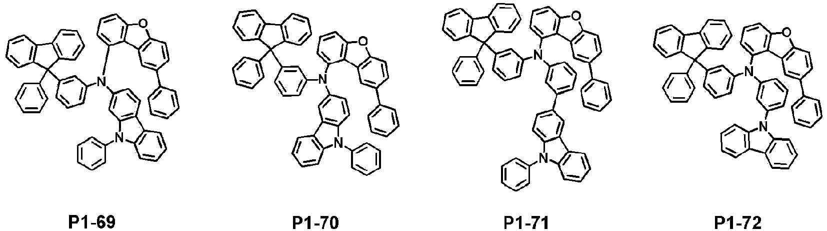

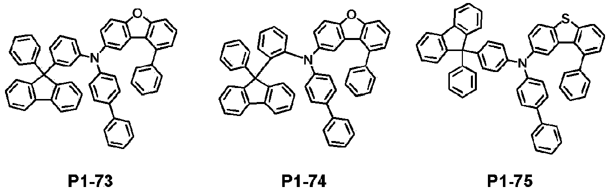

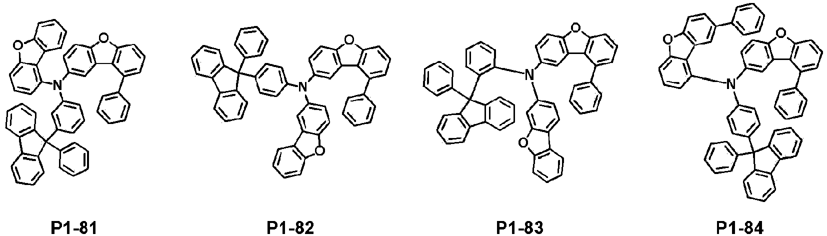

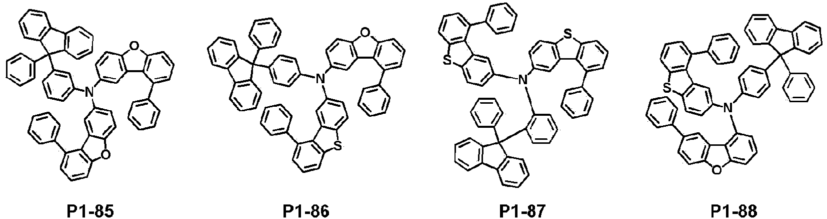

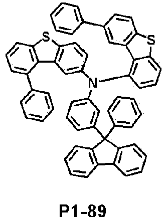

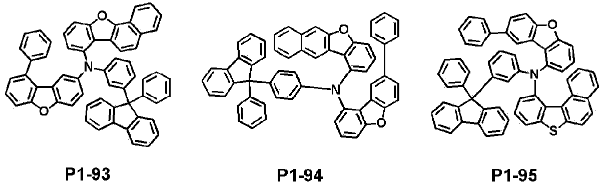

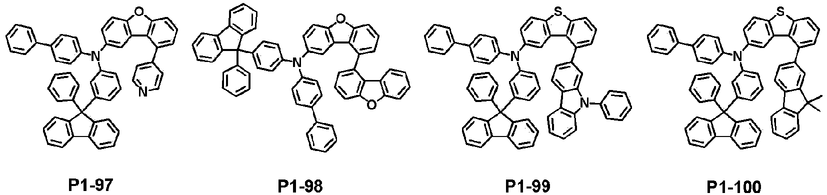

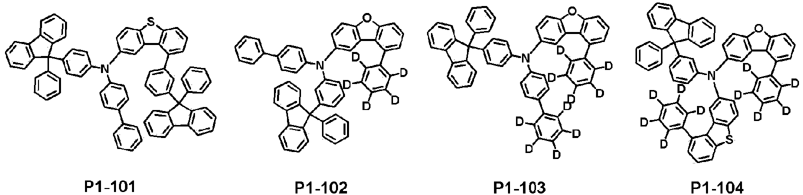

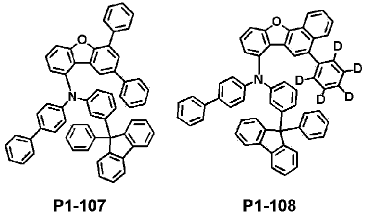

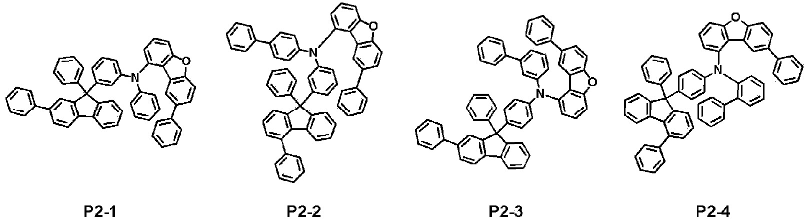

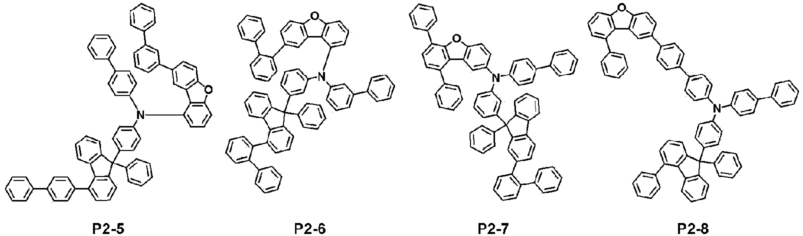

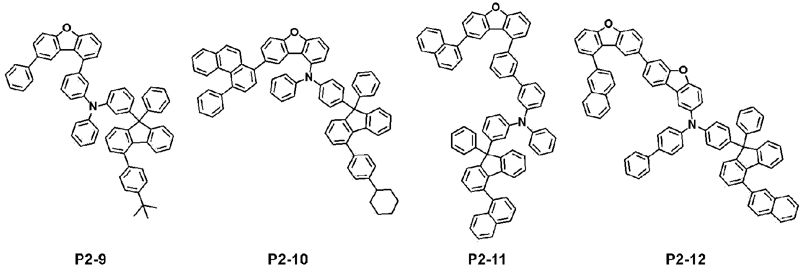

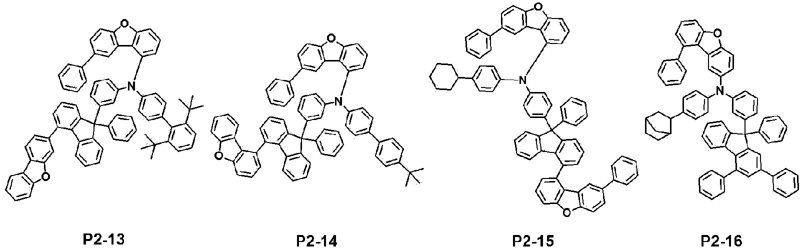

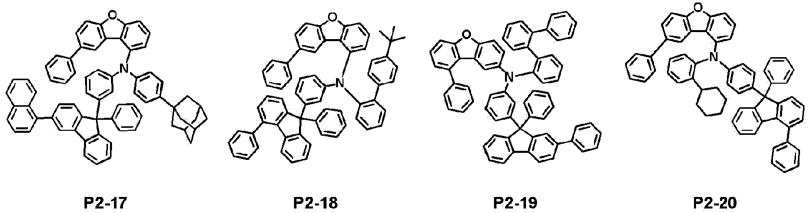

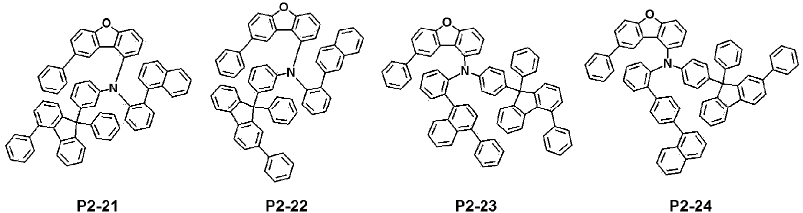

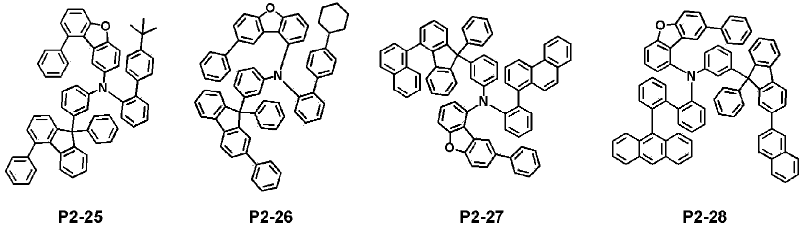

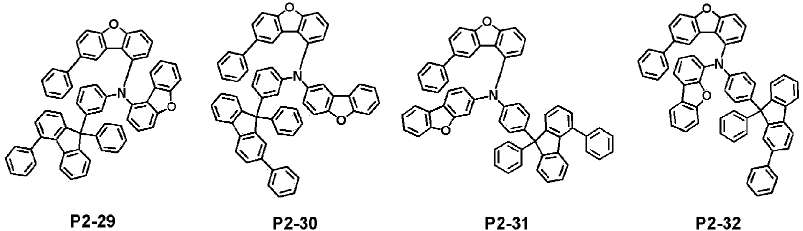

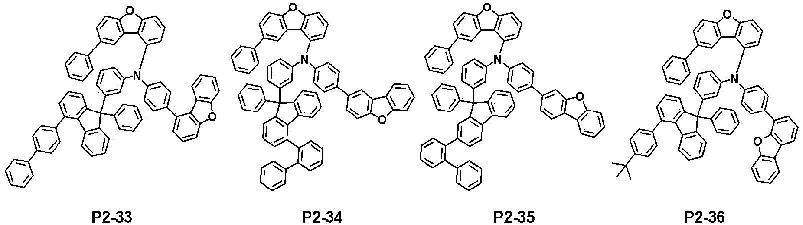

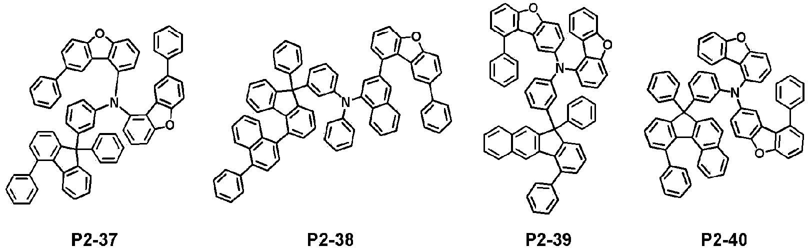

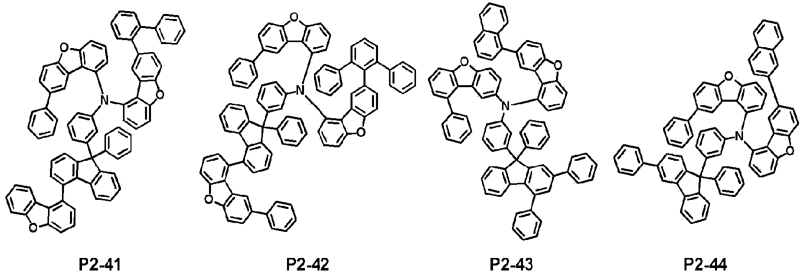

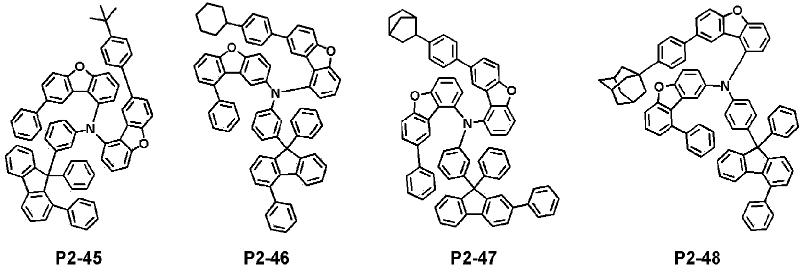

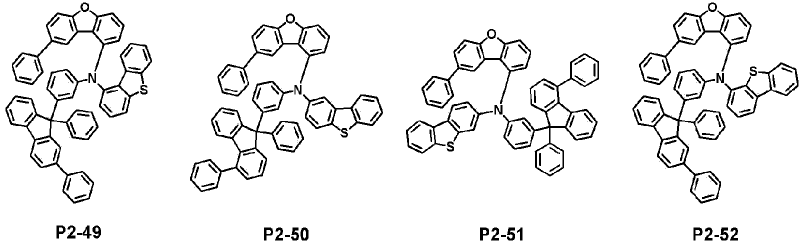

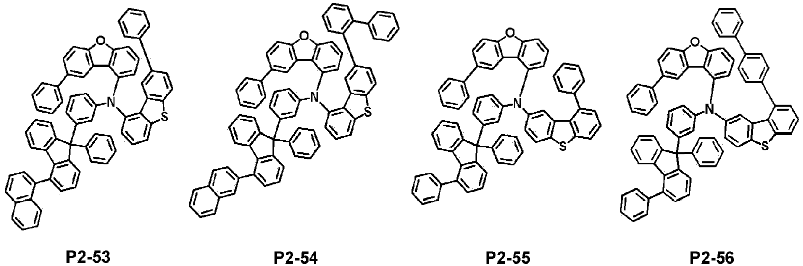

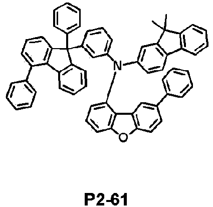

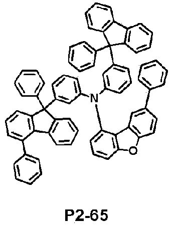

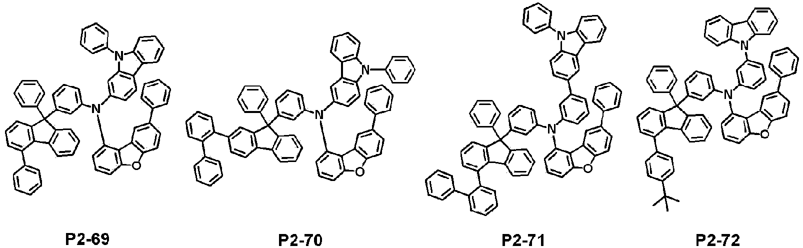

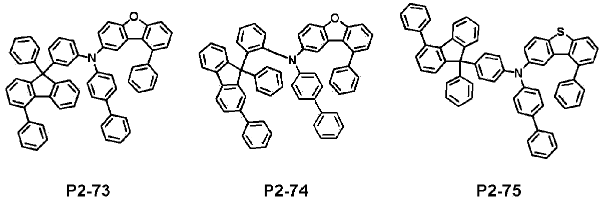

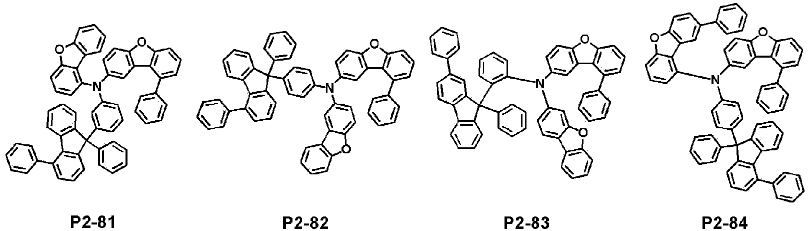

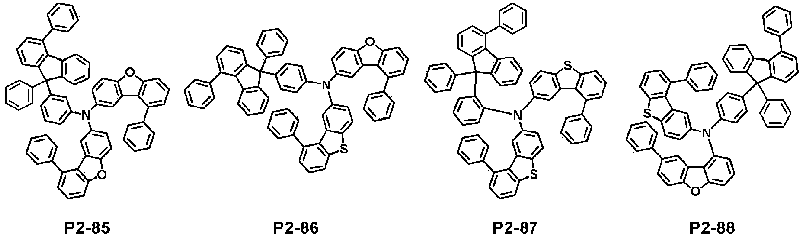

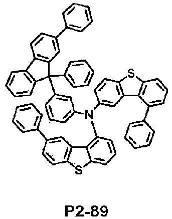

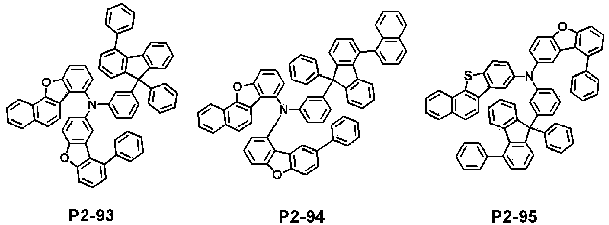

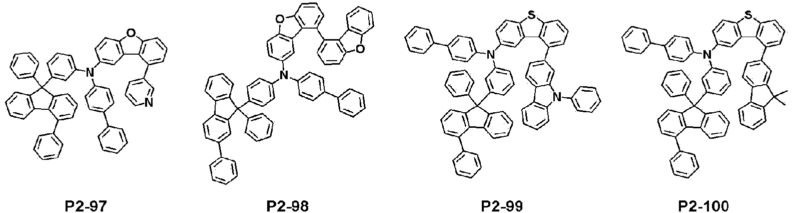

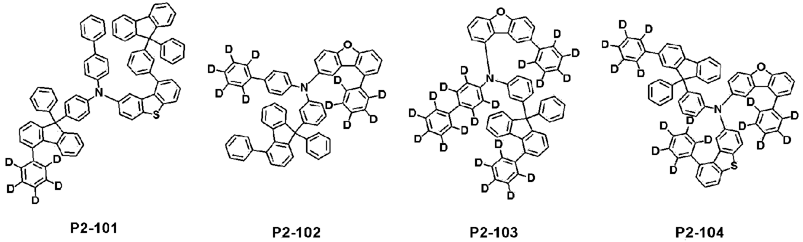

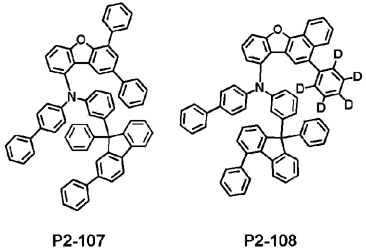

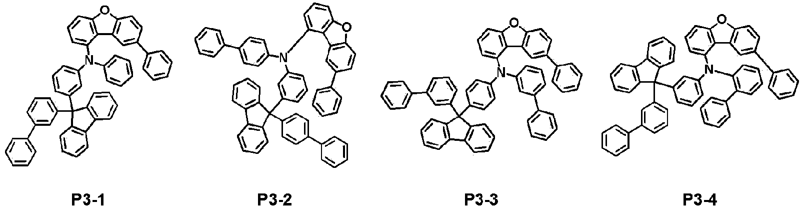

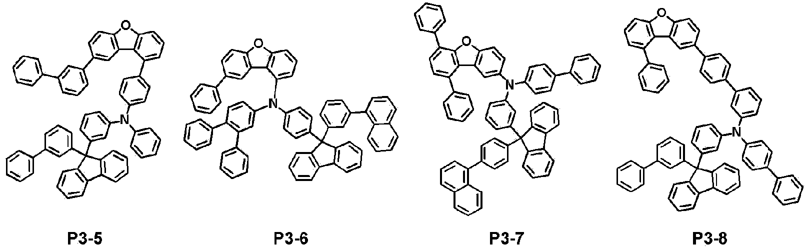

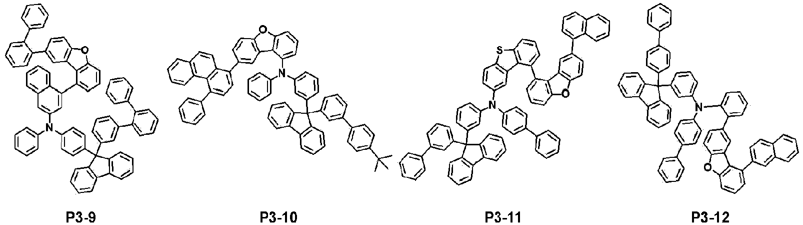

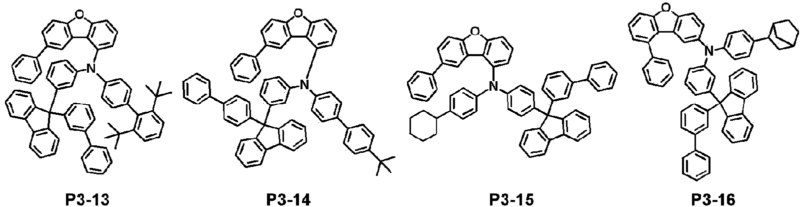

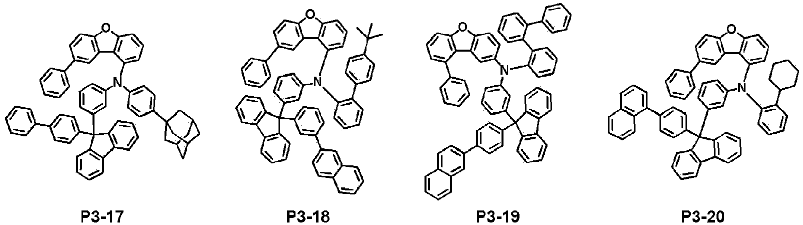

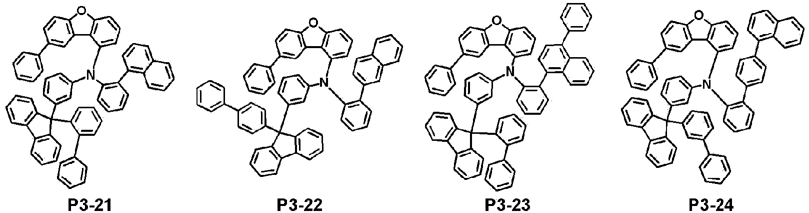

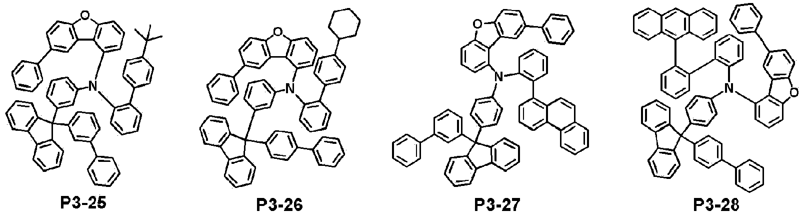

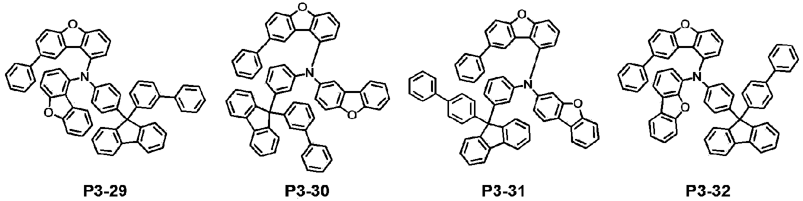

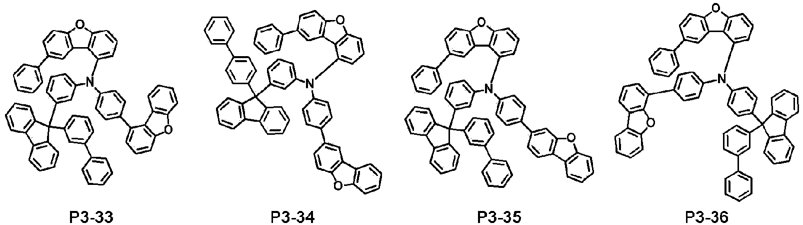

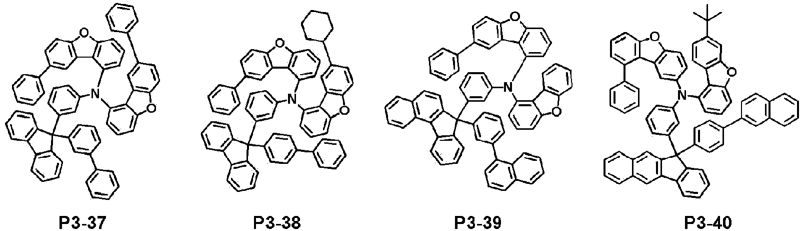

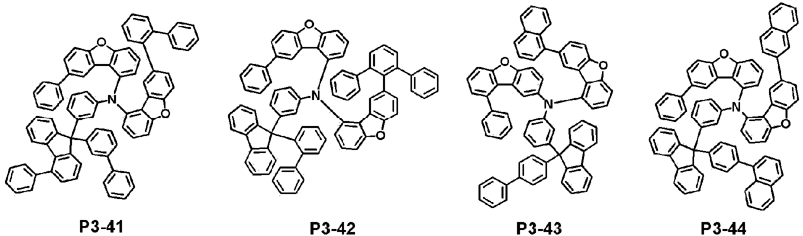

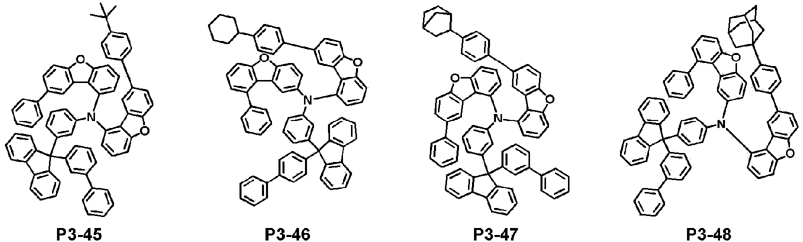

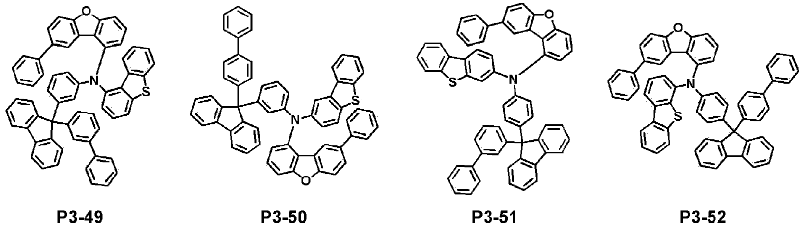

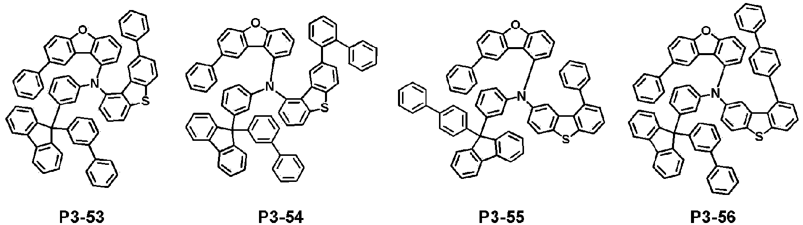

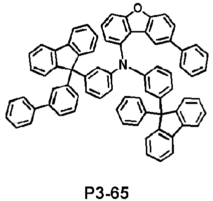

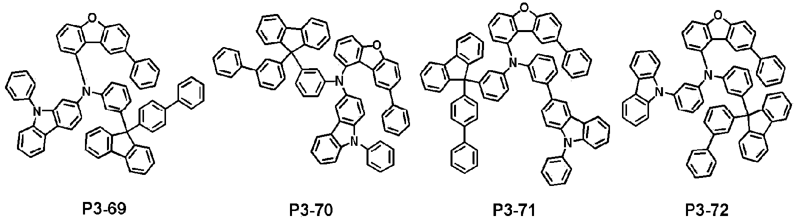

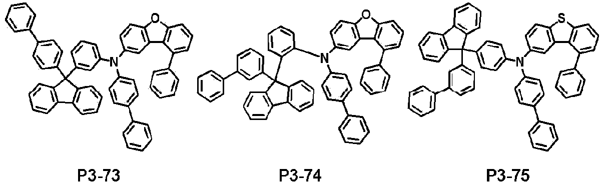

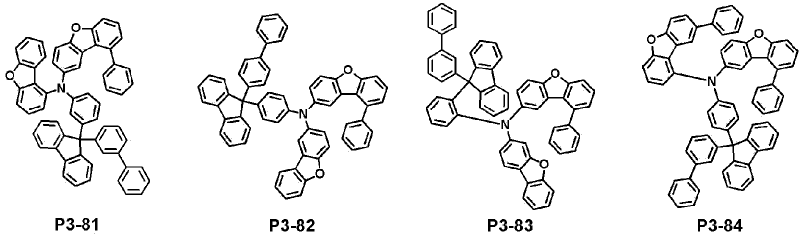

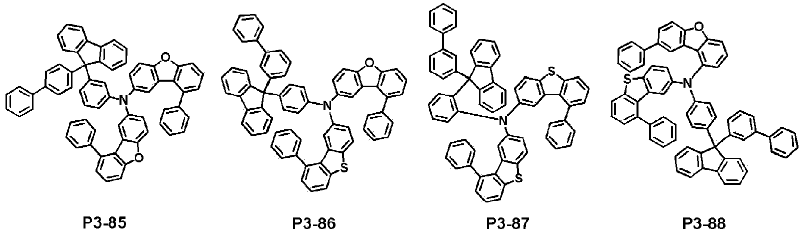

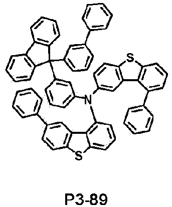

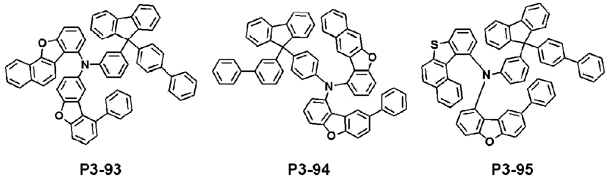

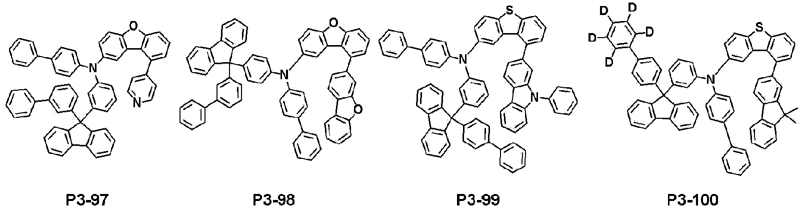

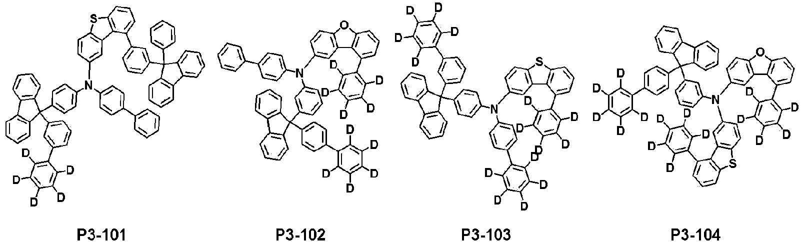

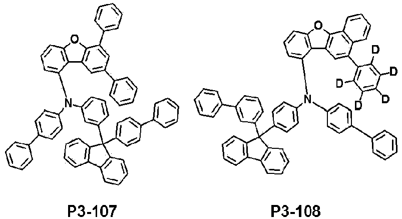

11. Organisches elektrisches Element, umfassend eine erste Elektrode, eine zweite Elektrode und eine organische Materialschicht zwischen der ersten Elektrode und der zweiten Elektrode, wobei die organische Materialschicht die Verbindung der Formel 1 gemäß Anspruch 1 umfasst.

12. Organisches elektrisches Element gemäß Anspruch 11, wobei die organische Materialschicht ferner eine lichtemittierende Schicht zwischen der ersten Elektrode und der zweiten Elektrode und eine lichtemittierende Hilfsschicht zwischen der lichtemittierenden Schicht und der ersten Elektrode umfasst und die lichtemittierende Hilfsschicht die Verbindung umfasst.

13. Organisches elektrisches Element gemäß Anspruch 11, wobei die organische Materialschicht zwei oder mehr Stapel umfasst und die zwei oder mehr Stapel jeweils eine Lochtransportschicht, eine lichtemittierende Schicht und eine Elektronentransportschicht, die aufeinanderfolgend auf der ersten Elektrode ausgebildet sind, umfassen.

14. Organisches elektrisches Element gemäß Anspruch 13, wobei die organische Materialschicht ferner eine Ladungserzeugungsschicht zwischen den zwei oder mehr Stapeln umfasst.

15. Organisches elektrisches Element gemäß Anspruch 11, wobei das organische elektrische Element ferner eine Schicht zum Verbessern der Lichtausbeute umfasst und die Schicht zum Verbessern der Lichtausbeute auf einer Seite von beiden Seiten der ersten Elektrode oder der zweiten Elektrode ausgebildet ist, und die eine Seite nicht der organischen Materialschicht zugewandt ist.

16. Elektronische Vorrichtung, umfassend eine Anzeigevorrichtung und eine Steuereinheit zum Ansteuern der Anzeigevorrichtung, wobei die Anzeigevorrichtung das organische elektrische Element gemäß Anspruch 11 umfasst.

17. Elektronische Vorrichtung gemäß Anspruch 16, wobei das organische elektrische Element ausgewählt ist aus der Gruppe bestehend aus einem organischen Elektrolumineszenzelement, einer organischen Solarzelle, einem organischen Fotoleiter, einem organischen Transistor, einem Element zur monochromatischen Beleuchtung und einem Quantenpunktdisplay.

## Revendications

1. Composé de formule 1 : 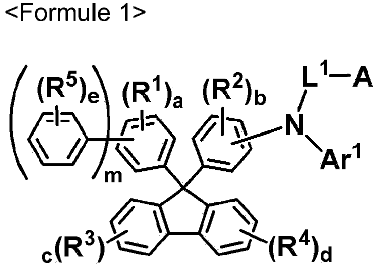 dans la formule 1,
A est la formule A-1 ou la formule A-2, 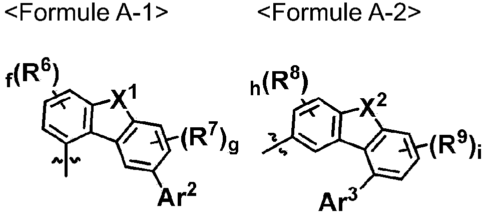
R¹ à R⁹ sont chacun indépendamment choisis dans le groupe constitué par l'hydrogène, le deutérium, un halogène, un groupe cyano, un groupe nitro, un groupe aryle en C₆-C₆₀, un groupe fluorényle, un groupe hétérocyclique en C₂-C₆₀ contenant au moins un hétéroatome parmi O, N, S, Si et P, un groupe cyclique aliphatique en C₃-C₆₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₆₀, et -L'-N(Rₐ)(R_{b}), et des groupes adjacents peuvent être liés les uns aux autres pour former un cycle,
a est un nombre entier de 0 à 5, b à d sont chacun un nombre entier de 0 à 4, e est un nombre entier de 0 à 5, m est un nombre entier de 0 à 2, f et i sont chacun un nombre entier de 0 à 3,
X¹ et X² sont O ou S,
L¹ et L' sont chacun indépendamment choisis dans le groupe constitué par une liaison simple, un groupe arylène en C₆-C₆₀, un groupe fluorénylène, un groupe cyclique aliphatique en C₃-C₆₀, et un groupe hétérocyclique en C₂-C₆₀ contenant au moins un hétéroatome parmi O, N, S, Si et P,
Ar², Ar³, R^{a} et R_{b} sont chacun indépendamment choisis dans le groupe constitué par un groupe aryle en C₆-C₆₀, un groupe fluorényle, un groupe cyclique aliphatique en C₃-C₆₀, et un groupe hétérocyclique en C₂-C₆₀ contenant au moins un hétéroatome parmi O, N, S, Si et P, et
le groupe aryle, le groupe arylène, le groupe fluorényle, le groupe fluorénylène, le groupe hétérocyclique, le groupe cyclique aliphatique, le groupe alkyle, le groupe alcényle, le groupe alcynyle, le groupe alcoxyle, le groupe aryloxyle, et le cycle formé par des groupes adjacents peuvent chacun être éventuellement substitués par un ou plusieurs substituants choisis dans le groupe constitué par le deutérium, un halogène, un groupe cyano, un groupe nitro, un groupe siloxane, un groupe aryle en C₆-C₃₀, un groupe fluorényle, un groupe hétérocyclique en C₂-C₃₀ comprenant au moins un hétéroatome choisi dans le groupe constitué par O, N, S, Si et P, un groupe cyclique aliphatique en C₃-C₃₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₃₀, un groupe alkylthio en C₁-C₂₀, et un groupe arylthio en C₆-C₃₀, et
où Ar¹ est choisi dans le groupe constitué par les formules Ar-1 à Ar-6 : 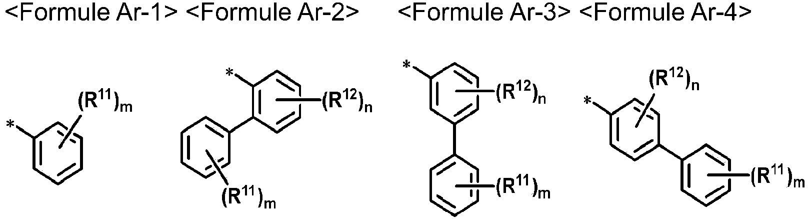 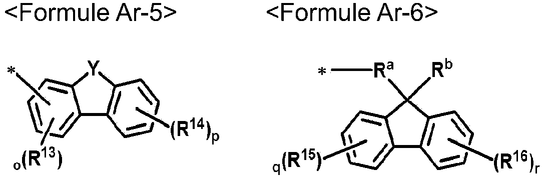
dans les formules Ar-1 à Ar-6,
R¹¹ à R¹⁰ sont chacun indépendamment choisis dans le groupe constitué par le deutérium, un halogène, un groupe cyano, un groupe nitro, un groupe siloxane, un groupe aryle en C₆-C₃₀, un groupe fluorényle, un groupe hétérocyclique en C₂-C₃₀ comprenant au moins un hétéroatome choisi dans le groupe constitué par O, N, S, Si et P, un groupe cyclique aliphatique en C₃-C₃₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₂₀, un groupe alkylthio en C₁-C₂₀, et un groupe arylthio en C₆-C₂₀, et des groupes adjacents peuvent être liés les uns aux autres pour former un groupe cyclique aromatique en C₆-C₁₄,
m' est un nombre entier de 0 à 5, n, p, q et r sont chacun un nombre entier de 0 à 4, o est un nombre entier de 0 à 3, et lorsque chacun de ceux-ci est un nombre entier de 2 ou plus, chacun des R¹³, chacun des R¹⁴, chacun des R¹⁵, chacun des R¹⁰ sont identiques ou différents les uns des autres,
Y est S, O, ou N(R₃),
R^{a} est choisi dans le groupe constitué par une liaison simple, un groupe alkylène en C₁-C₂₀, un groupe arylène en C₆-C₃₀, un groupe fluorénylène, un groupe hétérocyclique en C₂-C₃₀ comprenant au moins un hétéroatome choisi dans le groupe constitué par O, N, S, Si et P, et un groupe cyclique aliphatique en C₃-C₃₀, et
R₃ et R^{b} sont chacun indépendamment choisis dans le groupe constitué par le deutérium, un halogène, un groupe cyano, un groupe nitro, un groupe siloxane, un groupe aryle en C₆-C₃₀, un groupe fluorényle, un groupe hétérocyclique en C₂-C₃₀ comprenant au moins un hétéroatome choisi dans le groupe constitué par O, N, S, Si et P, un groupe cyclique aliphatique en C₃-C₃₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₂₀, un groupe alkylthio en C₁-C₂₀, et un groupe arylthio en C₆-C₂₀.

2. Composé selon la revendication 1, où la formule 1 est représentée par l'une des formules 2-1 à 2-4 : 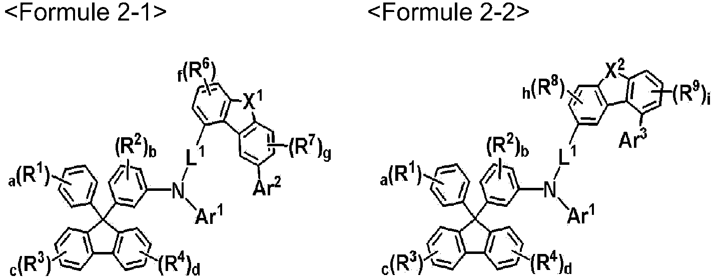 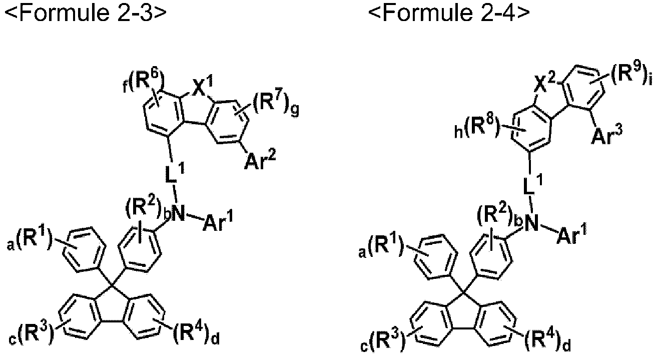 dans les formules 2-1 à 2-4, R¹ à R⁴, R⁶ à R⁹, X¹, X², L¹, Ar¹ à Ar³, a à d, f à i sont tels que définis dans la revendication 1.

3. Composé selon la revendication 1, où la formule 1 est représentée par l'une des formules 3-1 à 3-4 : 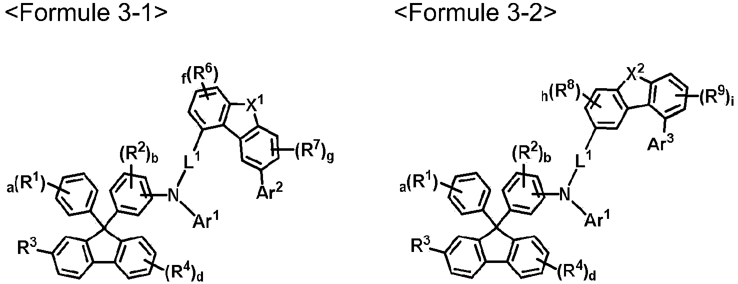 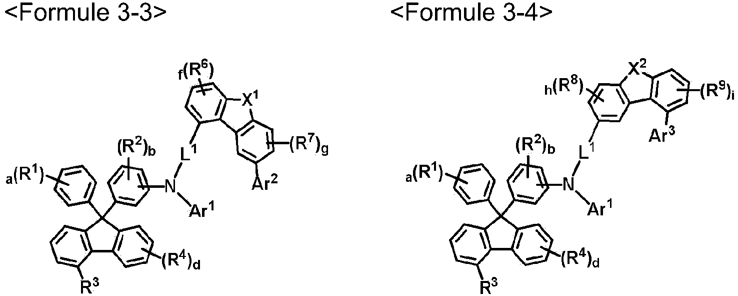 dans les formules 3-1 à 3-4,
R¹, R², R⁴, R⁶ à R⁹, X¹, X², L¹, Ar¹ à Ar³, a, b, d, f à i sont tels que définis dans la revendication 1, et
R³ est choisi dans le groupe constitué par le deutérium, un halogène, un groupe cyano, un groupe nitro, un groupe aryle en C₆-C₆₀, un groupe fluorényle, un groupe hétérocyclique en C₂-C₆₀ contenant au moins un hétéroatome parmi O, N, S, Si et P, un groupe cyclique aliphatique en C₃-C₆₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₆₀, et -L'-N(Rₐ)(R_{b}).

4. Composé selon la revendication 1, où la formule 1 est représentée par l'une des formules 4-1 à 4-8 : 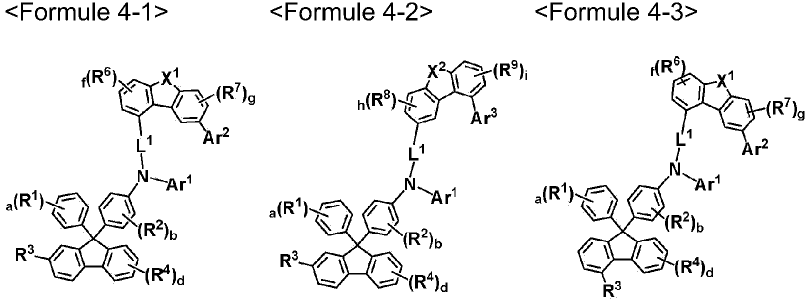 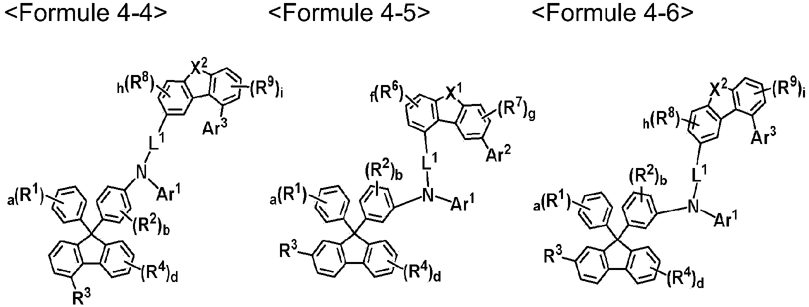 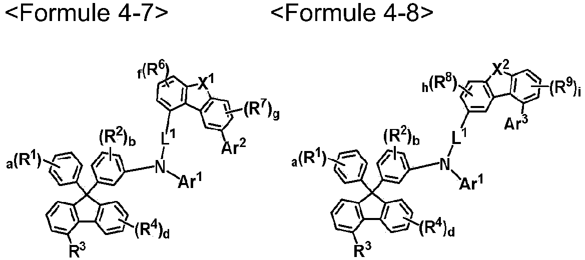
dans les formules 4-1 à 4-8, R¹, R², R⁴, R⁶ à R⁹, X¹, X², L¹, Ar¹ à Ar³, a, b, d, f à i sont tels que définis dans la revendication 1, et
R³ est choisi dans le groupe constitué par le deutérium, un halogène, un groupe cyano, un groupe nitro, un groupe aryle en C₆-C₆₀, un groupe fluorényle, un groupe hétérocyclique en C₂-C₆₀ contenant au moins un hétéroatome parmi O, N, S, Si et P, un groupe cyclique aliphatique en C₃-C₆₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₂₀, et -L'-N(Rₐ)(R_{b}).

5. Composé selon la revendication 1, où la formule 1 est représentée par la formule 5-1 ou la formule 5-2 : 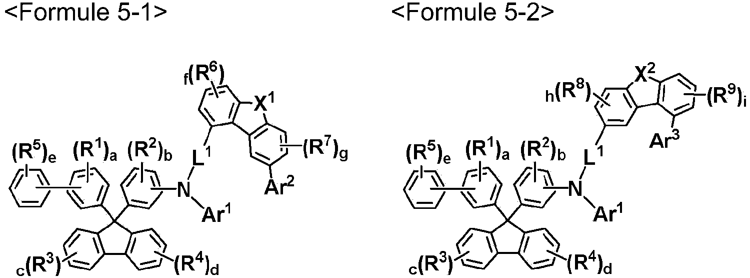 dans les formules 5-1 et 5-2, R¹ à R⁹, X¹, X², L¹, Ar¹ à Ar³, a à i sont tels que définis dans la revendication 1.

6. Composé selon la revendication 1, où la formule 1 est représentée par la formule 6-1 ou la formule 6-2 : 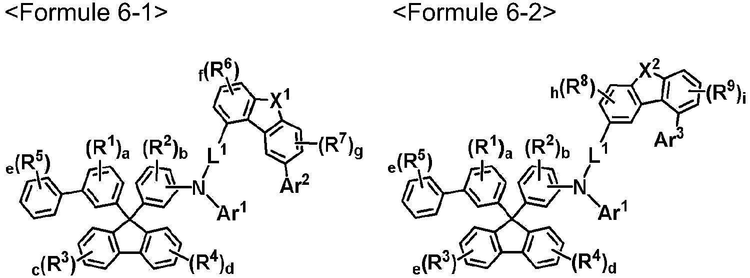 dans les formules 6-1 et 6-2, R¹ à R⁹, X¹, X², L¹, Ar¹ à Ar³, a à i sont tels que définis dans la revendication 1.

7. Composé selon la revendication 1, où la formule 1 est représentée par l'une des formules 7-1 à 7-4 : 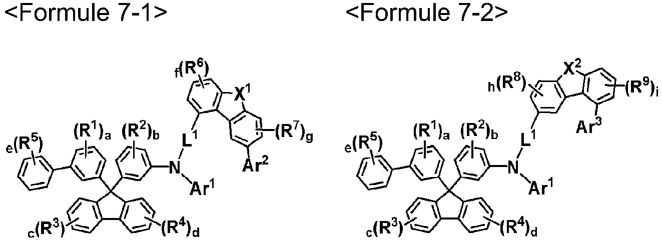 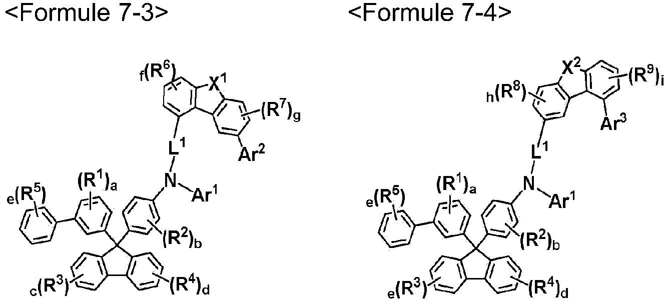 dans les formules 7-1 à 7-4, R¹ à R⁹, X¹, X², L¹, Ar¹ à Ar³, a à i sont tels que définis dans la revendication 1.

8. Composé selon la revendication 1, où au moins l'un parmi Ar² et Ar³ est choisi dans le groupe constitué par les formules Ar-1 à Ar-6 : 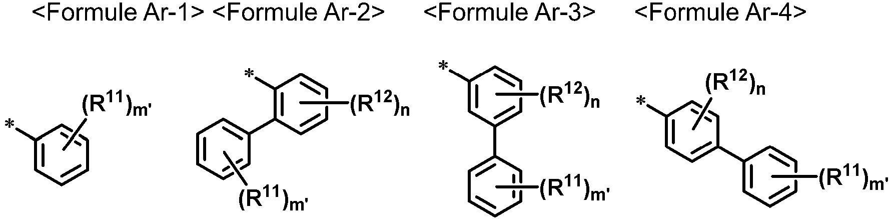 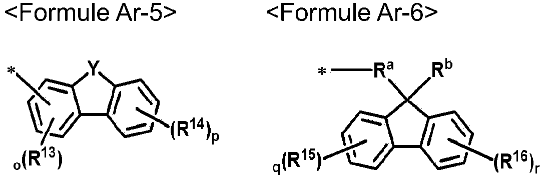 dans les formules Ar-1 à Ar-6,
R¹¹ à R¹⁶ sont chacun indépendamment choisis dans le groupe constitué par le deutérium, un halogène, un groupe cyano, un groupe nitro, un groupe siloxane, un groupe aryle en C₆-C₃₀, un groupe fluorényle, un groupe hétérocyclique en C₂-C₃₀ comprenant au moins un hétéroatome choisi dans le groupe constitué par O, N, S, Si et P, un groupe cyclique aliphatique en C₃-C₃₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₂₀, un groupe alkylthio en C₁-C₂₀, et un groupe arylthio en C₆-C₂₀, et des groupes adjacents peuvent être liés les uns aux autres pour former un cycle,
m' est un nombre entier de 0 à 5, n, p, q et r sont chacun un nombre entier de 0 à 4, o est un nombre entier de 0 à 3, et lorsque chacun de ceux-ci est un nombre entier de 2 ou plus, chacun des R¹³, chacun des R¹⁴, chacun des R¹⁵, chacun des R¹⁶ sont identiques ou différents les uns des autres,
Y est S, O, C(R₁)(R₂) ou N(R₃),
R^{a} est choisi dans le groupe constitué par une liaison simple, un groupe alkylène en C₁-C₂₀, un groupe arylène en C₆-C₃₀, un groupe fluorénylène, un groupe hétérocyclique en C₂-C₃₀ comprenant au moins un hétéroatome choisi dans le groupe constitué par O, N, S, Si et P, et un groupe cyclique aliphatique en C₃-C₃₀, et
R₁ à R₃, R^{b} sont chacun indépendamment choisis dans le groupe constitué par le deutérium, un halogène, un groupe cyano, un groupe nitro, un groupe siloxane, un groupe aryle en C₆-C₃₀, un groupe fluorényle, un groupe hétérocyclique en C₂-C₃₀ comprenant au moins un hétéroatome choisi dans le groupe constitué par O, N, S, Si et P, un groupe cyclique aliphatique en C₃-C₃₀, un groupe alkyle en C₁-C₂₀, un groupe alcényle en C₂-C₂₀, un groupe alcynyle en C₂-C₂₀, un groupe alcoxyle en C₁-C₂₀, un groupe aryloxy en C₆-C₂₀, un groupe alkylthio en C₁-C₂₀, et un groupe arylthio en C₆-C₂₀, et R₁ et R₂ peuvent être liés l'un à l'autre pour former un cycle.

9. Composé selon la revendication 1, où à la fois Ar² et Ar³ sont un groupe aryle en C₆-C₆₀.

10. Composé selon la revendication 1, où le composé représenté par la formule 1 est l'un des composés suivants :

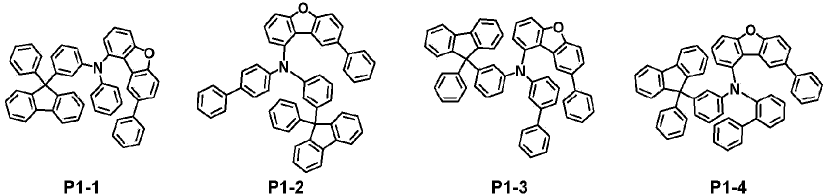

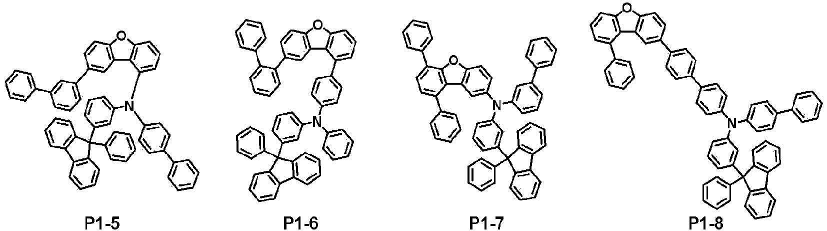

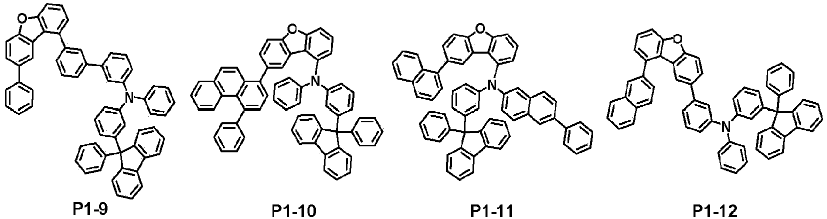

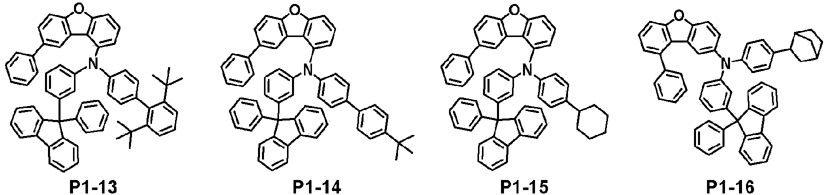

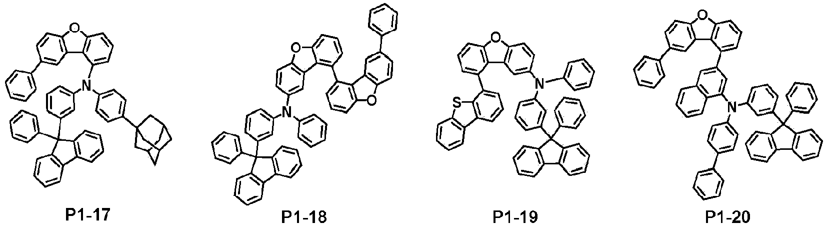

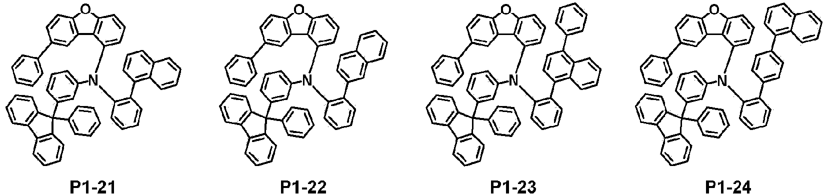

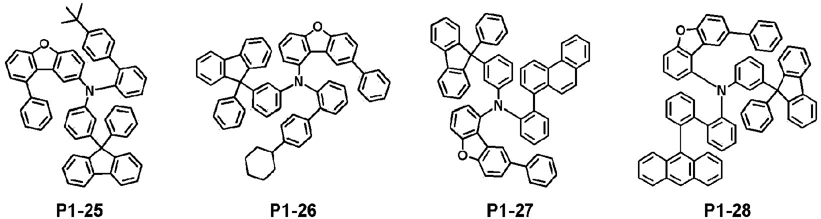

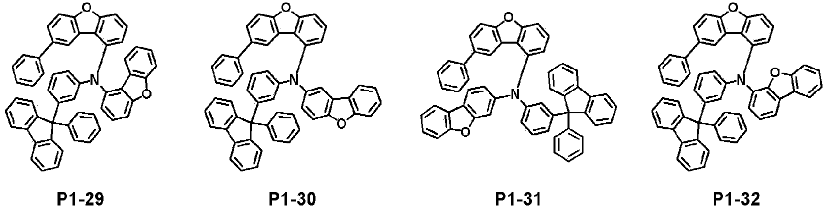

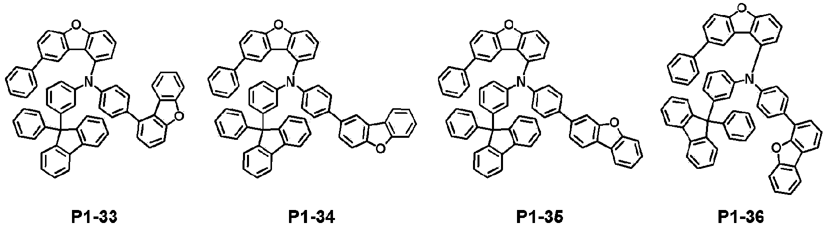

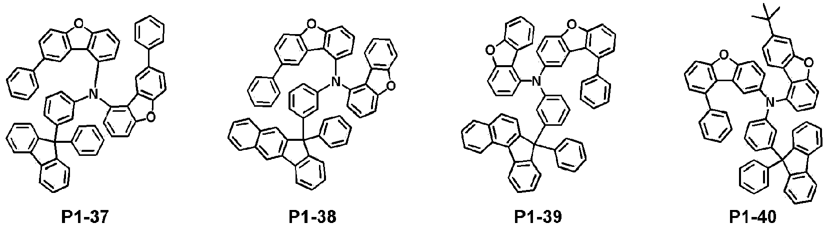

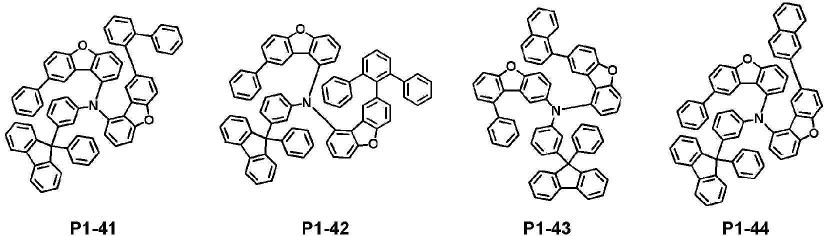

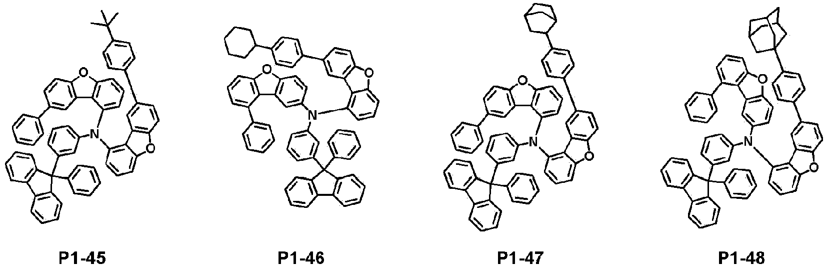

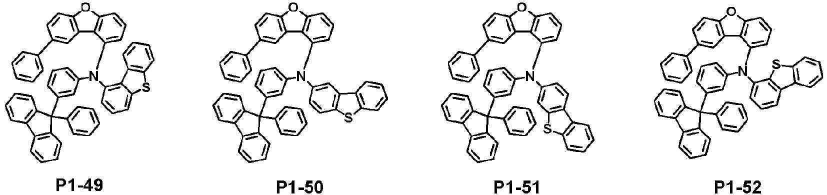

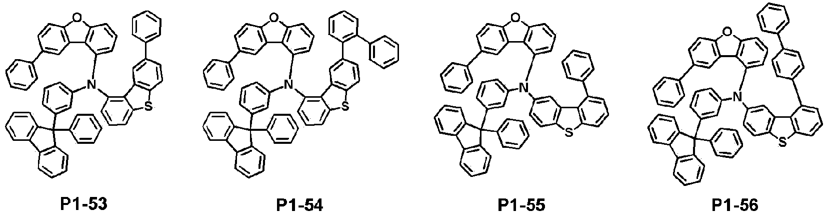

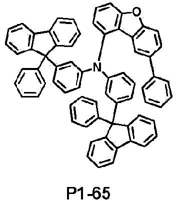

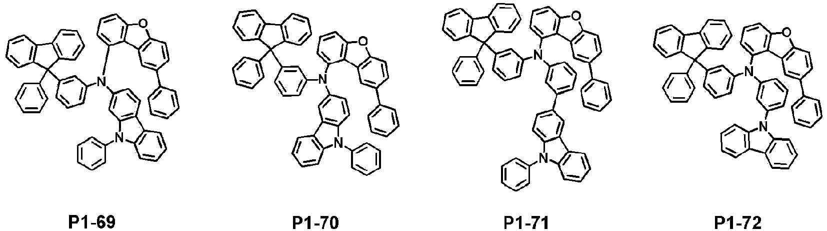

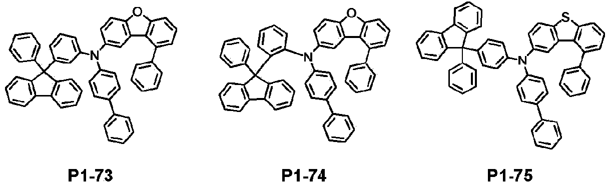

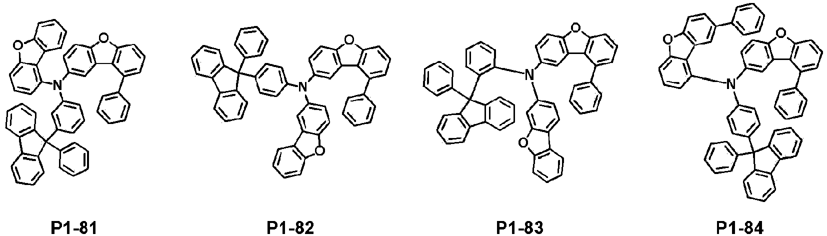

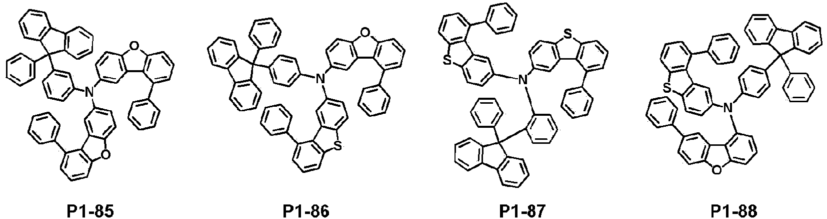

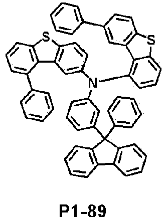

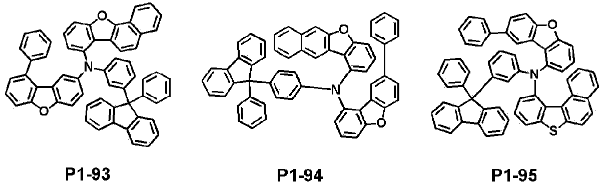

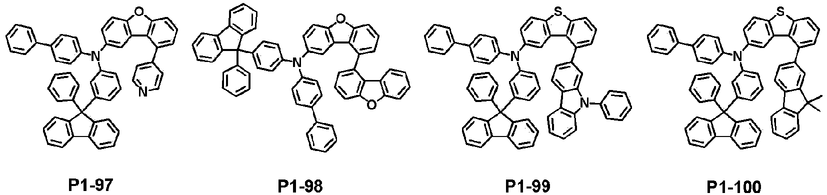

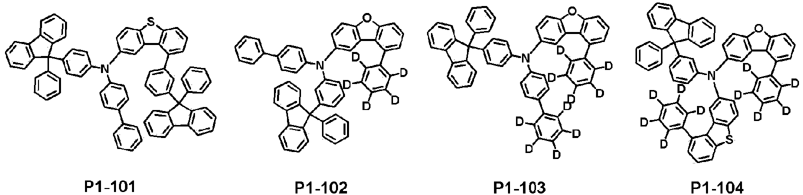

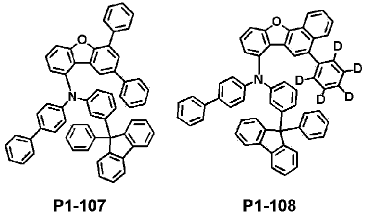

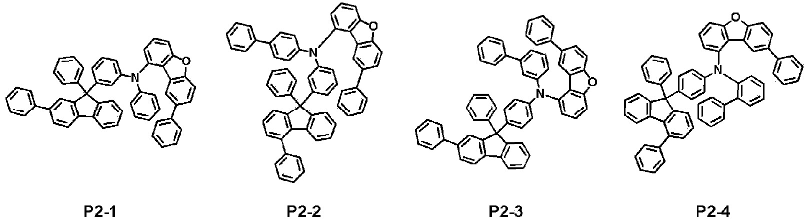

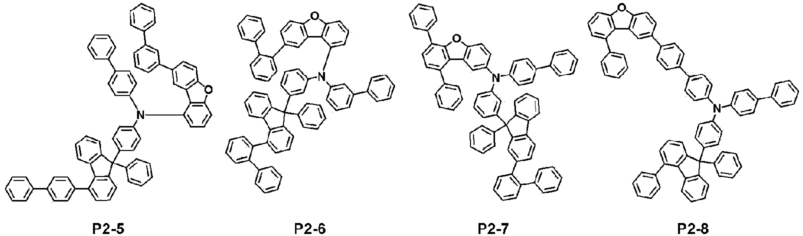

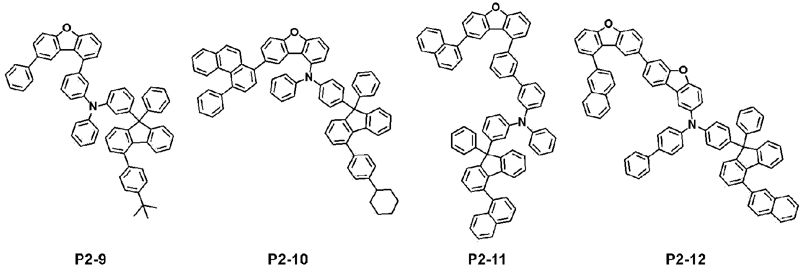

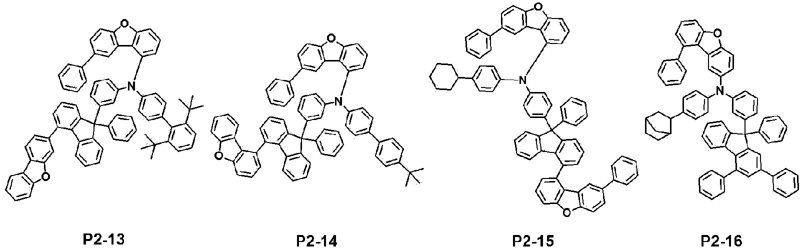

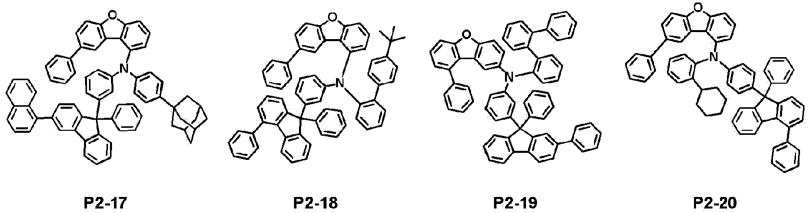

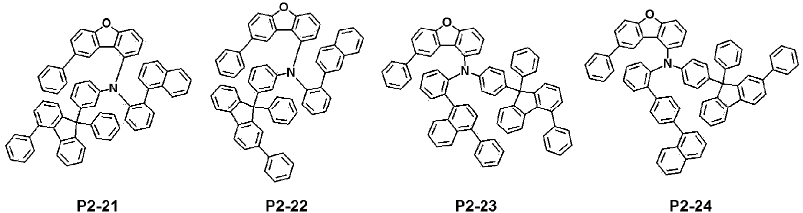

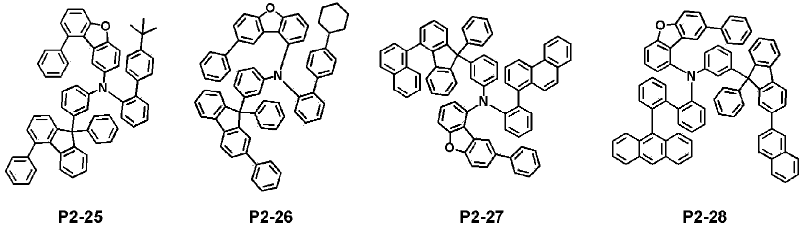

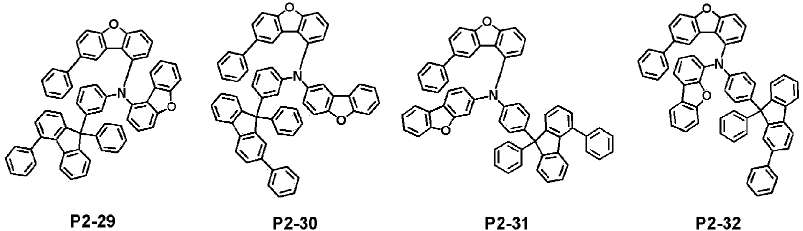

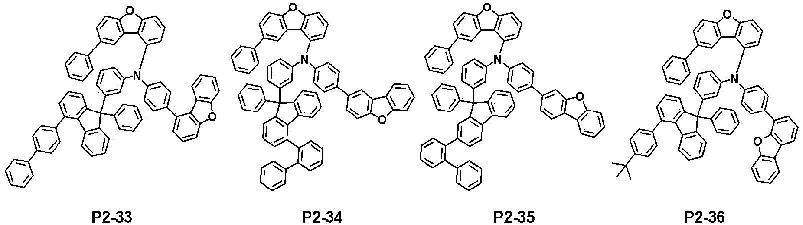

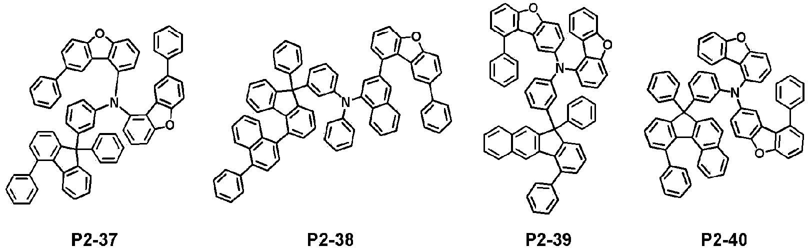

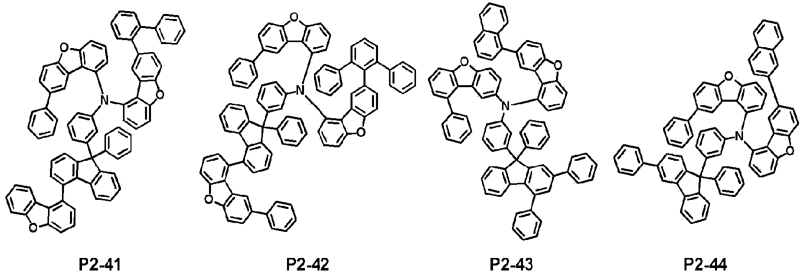

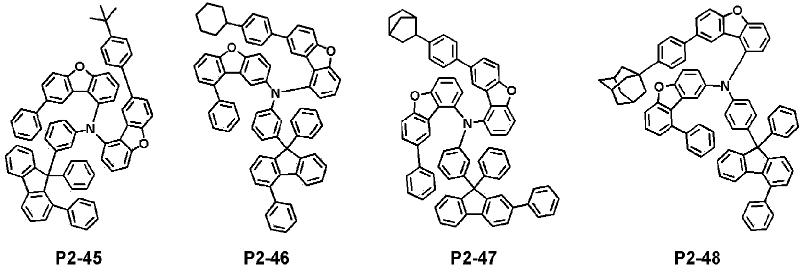

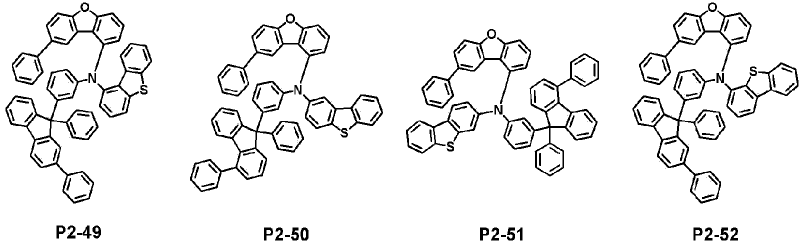

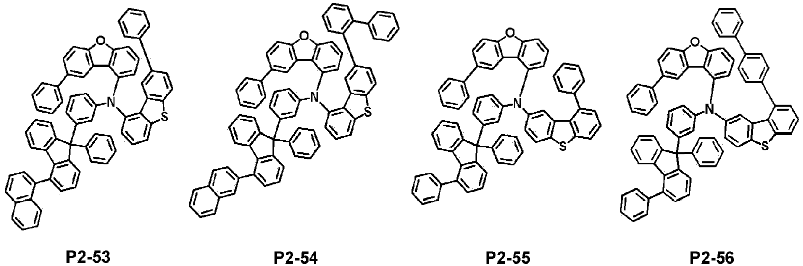

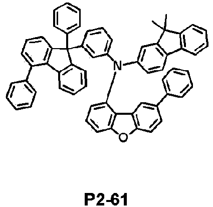

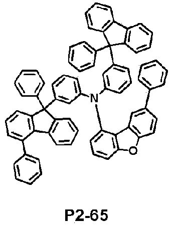

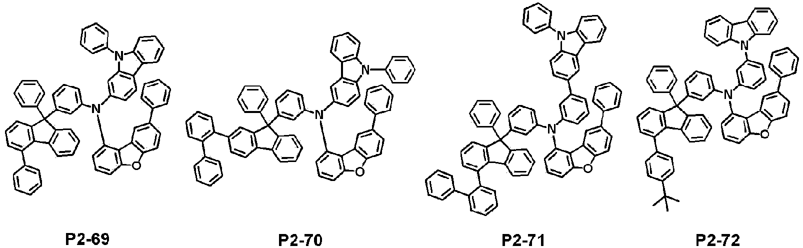

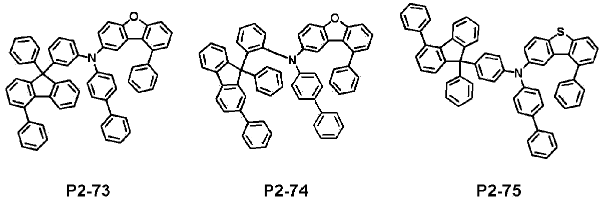

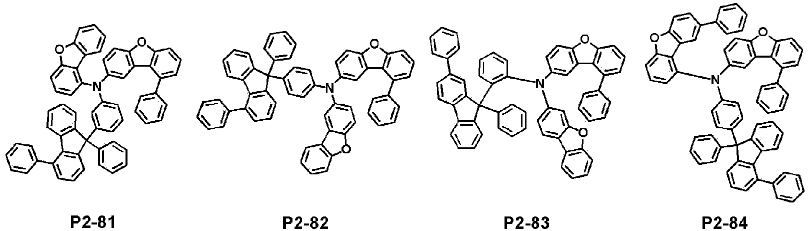

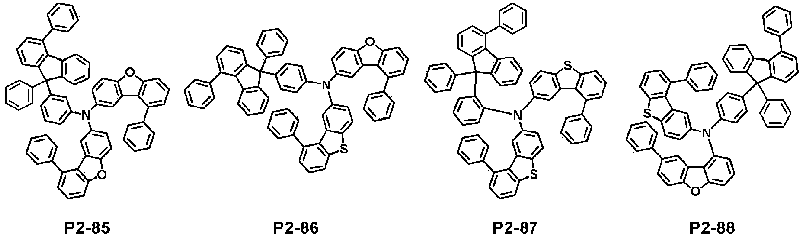

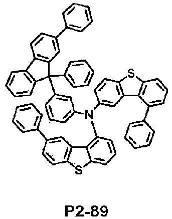

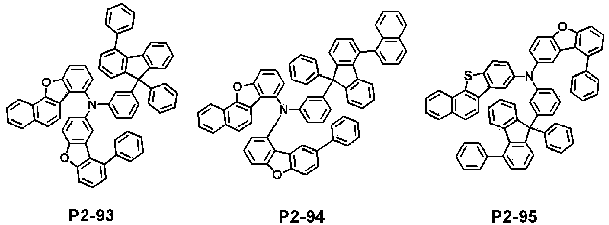

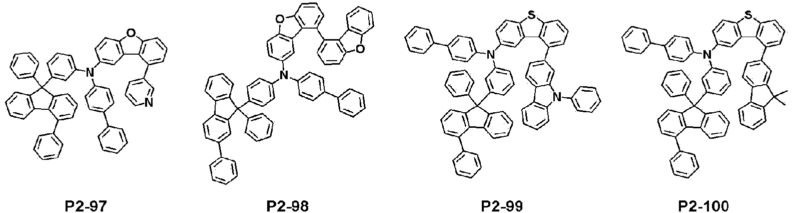

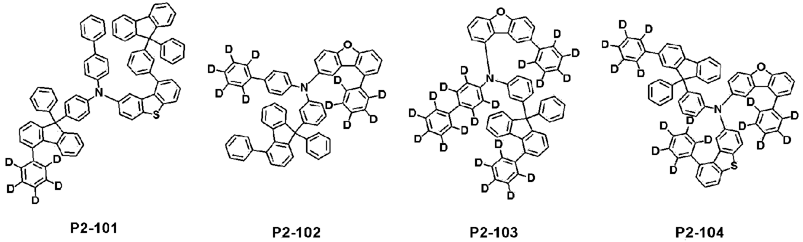

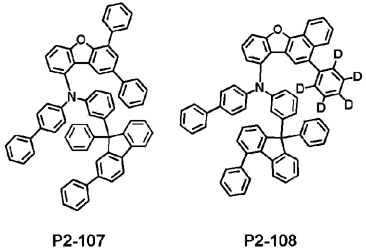

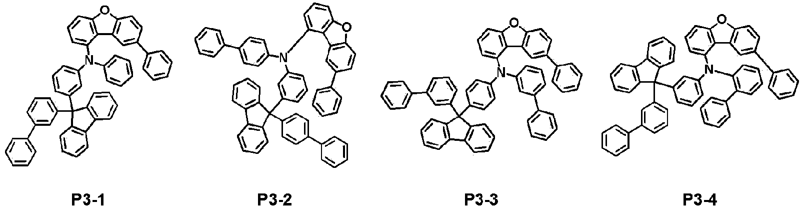

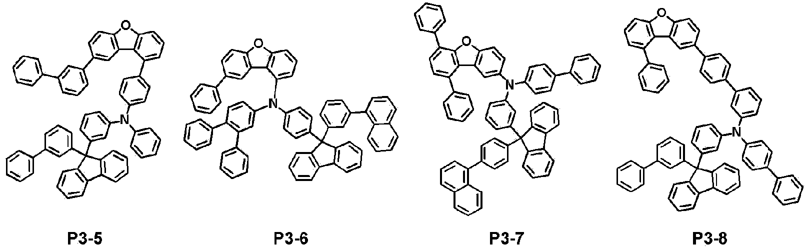

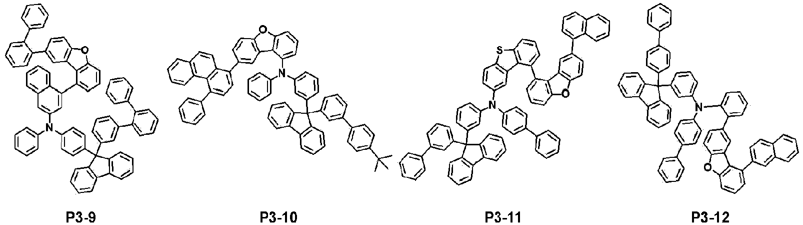

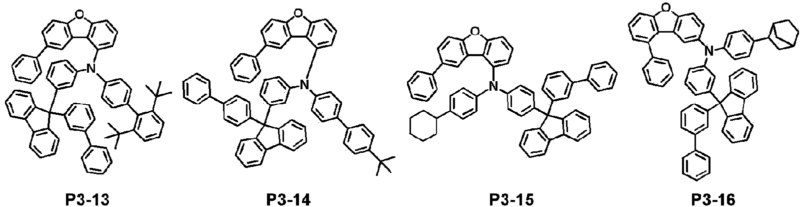

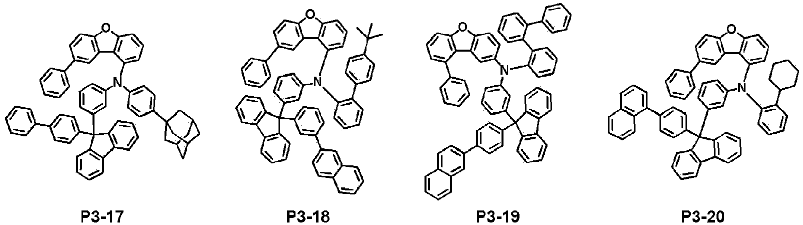

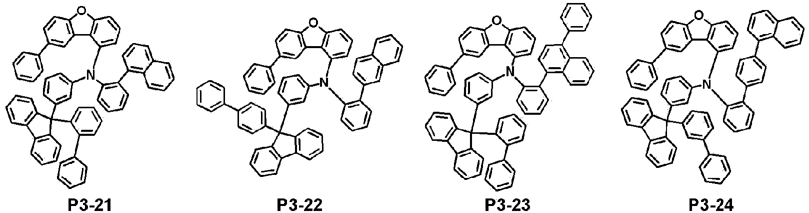

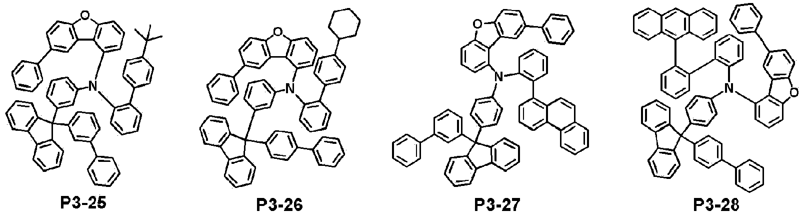

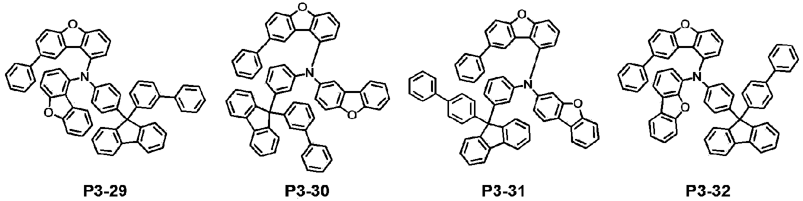

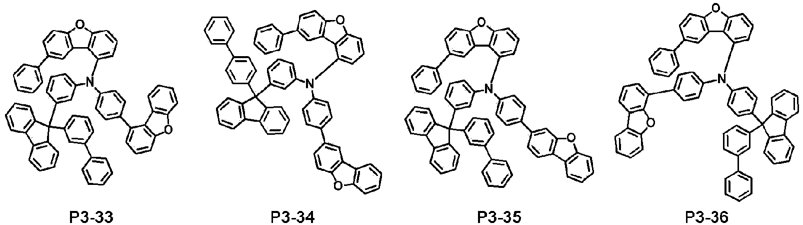

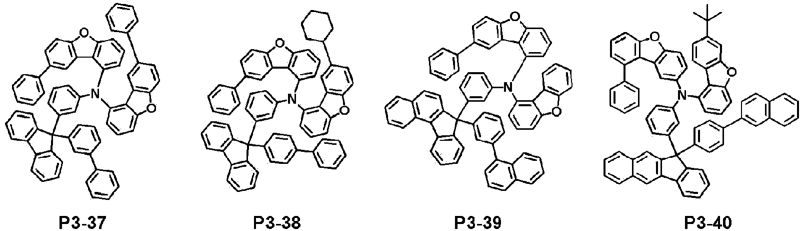

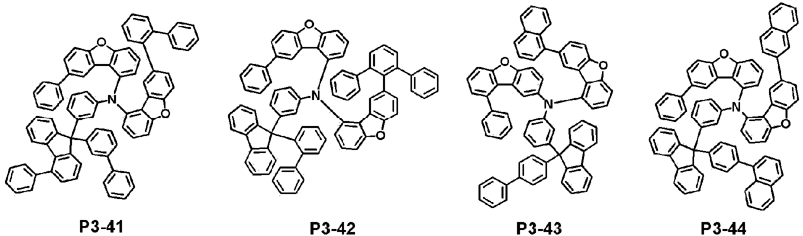

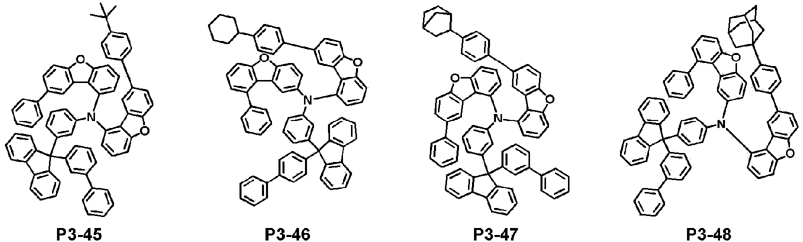

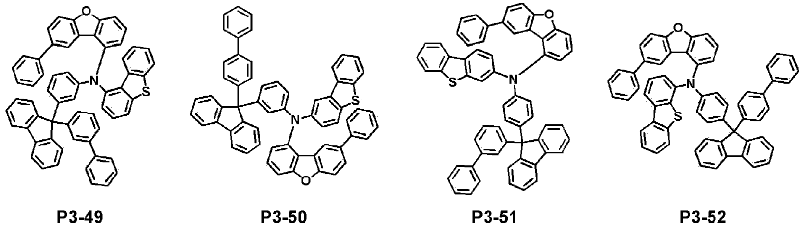

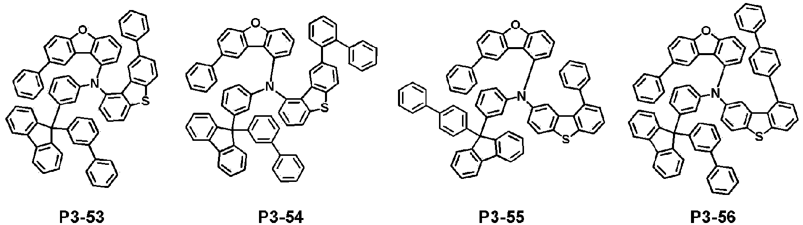

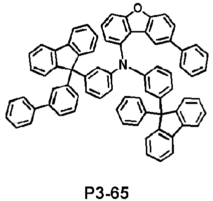

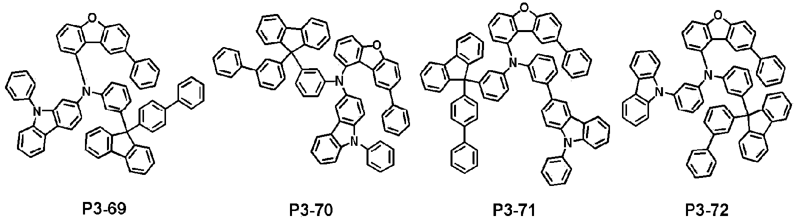

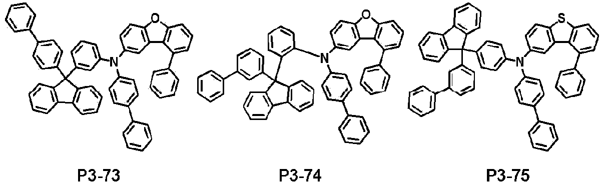

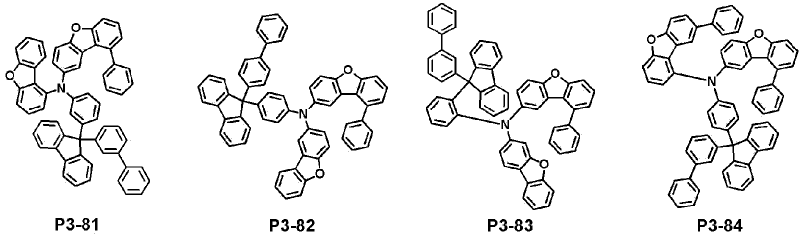

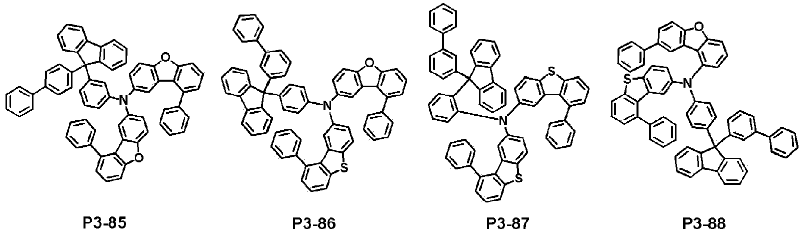

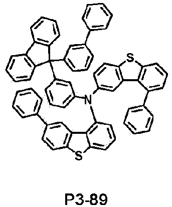

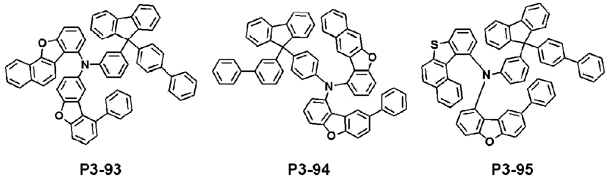

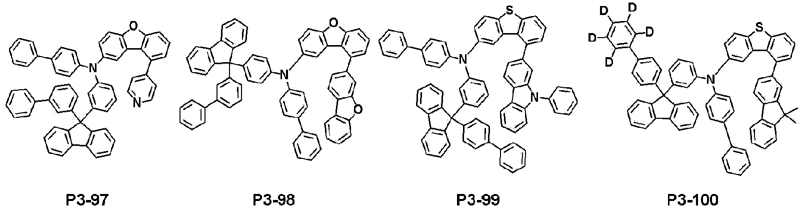

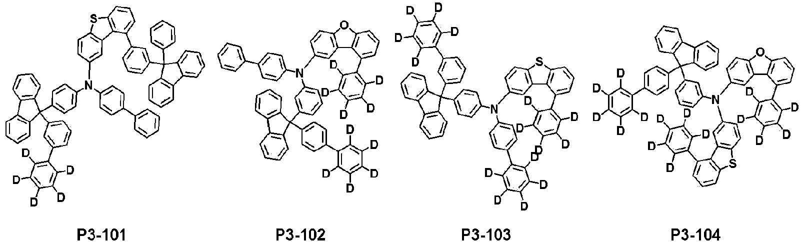

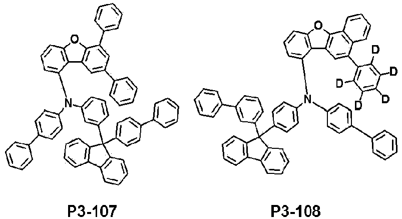

11. Elément électrique organique comprenant une première électrode, une deuxième électrode, et une couche de matériau organique entre la première électrode et la deuxième électrode, où la couche de matériau organique comprend le composé de formule 1 selon la revendication 1.

12. Elément électrique organique selon la revendication 11, où la couche de matériau organique comprend en outre une couche électroluminescente entre la première électrode et la deuxième électrode, et une couche auxiliaire électroluminescente entre la couche électroluminescente et la première électrode, et la couche auxiliaire électroluminescente comprend le composé.

13. Elément électrique organique selon la revendication 11, où la couche de matériau organique comprend deux empilements ou plus, et les deux empilements ou plus comprennent chacun une couche de transport de trous, une couche électroluminescente et une couche de transport d'électrons formées séquentiellement sur la première électrode.

14. Elément électrique organique selon la revendication 13, où la couche de matériau organique comprend en outre une couche de génération de charge entre les deux empilements ou plus.

15. Elément électrique organique selon la revendication 11, où l'élément électrique organique comprend en outre une couche pour améliorer l'efficacité lumineuse, et la couche pour améliorer l'efficacité lumineuse est formée sur un côté des deux côtés de la première électrode ou de la deuxième électrode, et sur le seul côté ne faisant pas face à la couche de matériau organique.

16. Dispositif électronique comprenant un dispositif d'affichage et une unité de commande pour commander le dispositif d'affichage, où le dispositif d'affichage comprend l'élément électrique organique selon la revendication 11.

17. Dispositif électronique selon la revendication 16, où l'élément électrique organique est choisi dans le groupe constitué par un élément électroluminescent organique, une cellule solaire organique, un photoconducteur organique, un transistor organique, un élément pour un éclairage monochromatique et un affichage à points quantiques.
